# EUROPÄISCHE PATENTSCHRIFT

(11) **EP 3 615 542 B1**
(45) Veröffentlichungstag und Bekanntmachung des Hinweises auf die Patenterteilung: **23.08.2023**
(21) Anmeldenummer: 18721011.7
(22) Anmeldetag: 24.04.2018
(51) Int. Cl.: C07D 498/22

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 25.04.2017 EP 17168075
(43) Veröffentlichungstag der Anmeldung: 04.03.2020
(73) Patentinhaber: Merck Patent GmbH, 64293 Darmstadt (DE)
(72) Erfinder: PARHAM, Amir, 60486 Frankfurt Am Main (DE); KROEBER, Jonas, 60311 Frankfurt Am Main (DE); JOOSTEN, Dominik, 64372 Ober-Ramstadt (DE); LUDEMANN, Aurélie, 60322 Frankfurt Am Main (DE); GROSSMANN, Tobias, 64297 Darmstadt (DE)
(86) Internationale Anmeldenummer: PCT/EP2018/060405
(87) Internationale Veröffentlichungsnummer: WO 2018/197447

(56) Entgegenhaltungen:
- EP-A1- 3 133 661
- EP-A1- 3 133 661
- EP-A1- 3 184 522
- EP-A1- 3 184 522
- WO-A2-2011/107186
- WO-A2-2011/107186
- US-A1- 2016 099 416
- US-A1- 2016 099 416
- US-A1- 2016 293 853
- US-A1- 2016 293 853
- US-A1- 2016 293 853

## Beschreibung

Die vorliegende Anmeldung betrifft verbrückte Triarylamine gemäß einer weiter unten definierten Formel (I), die mit einem Carbazolderivat substituiert sind. Die Verbindungen eignen sich zur Verwendung in elektronischen Vorrichtungen. Weiterhin betrifft die vorliegende Anmeldung Verfahren zur Herstellung der genannten Verbindungen, sowie elektronische Vorrichtungen enthaltend die genannten Verbindungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Emissionsschichten enthaltend phosphoreszierende Emitter und Schichten mit elektronentransportierender Funktion. Zur Verwendung in diesen Schichten werden weiterhin neue Materialien, insbesondere Materialien mit lochtransportierenden und elektronentransportierenden Eigenschaften, gesucht.

Im Stand der Technik sind zur Verwendung als Matrixmaterialien in Emissionsschichten, die phosphoreszierende Emitter enthalten, unter anderem Lactam-Derivate, Triazin-Derivate und Triarylamin-Derivate bekannt. Als Materialien für elektronentransportierende Schichten sind insbesondere Verbindungen bekannt, die eine oder mehrere elektronenarme Heteroarylgruppen tragen, insbesondere eine oder mehrere Gruppen gewählt aus Triazingruppen, Pyrimidingruppen und Chinolingruppen tragen.

Es besteht jedoch weiterhin Bedarf an alternativen Verbindungen, die zur Verwendung in elektronischen Vorrichtungen geeignet sind.

Es besteht zudem auch Verbesserungsbedarf bezüglich der Leistungsdaten der Verbindungen bei der Verwendung in elektronischen Vorrichtungen, insbesondere bezüglich Lebensdauer, Betriebsspannung und Effizienz.

Es wurde gefunden, dass sich bestimmte verbrückte Triarylamin-Verbindungen, die ein Carbazolderivat als Substituenten tragen, hervorragend zur Verwendung in elektronischen Vorrichtungen eignen, insbesondere zur Verwendung in OLEDs, nochmals insbesondere darin zur Verwendung als Matrixmaterialien für phosphoreszierende Emitter oder als Materialien einer elektronentransportierenden Schicht.

Die gefundenen Verbindungen zeigen einen oder mehrere, bevorzugt mehrere der folgenden vorteilhaften technischen Effekte:
- Bei der Verwendung in OLEDs führen sie zu einer hohen Effizienz der OLEDs
- Bei der Verwendung in OLEDs führen sie zu einer langen Lebensdauer der OLEDs
- Bei der Verwendung in OLEDs führen sie zu einer geringen Betriebsspannung der OLEDs
- Sie lassen sich sehr gut prozessieren.

Gegenstand der vorliegenden Anmeldung sind damit Verbindungen gemäß Formel (I) 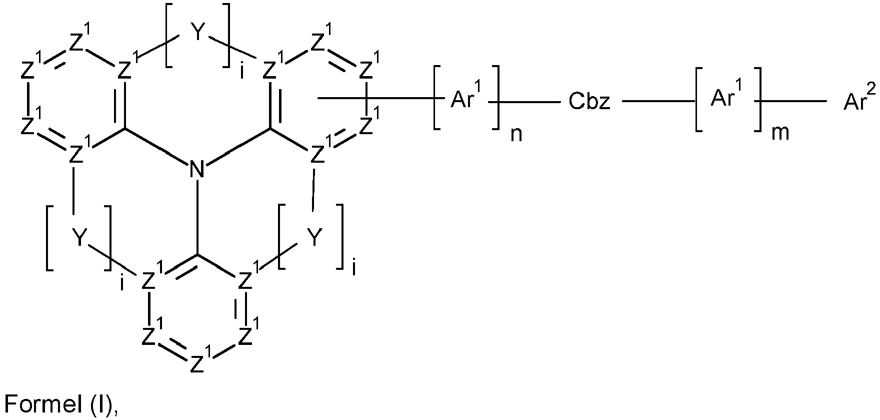 wobei für die auftretenden Variablen gilt:
- Y: ist bei jedem Auftreten gleich oder verschieden gewählt aus Einfachbindung, O und S, wobei mindestens eine Gruppe Y vorhanden ist, die gewählt ist aus O und S;
- Z¹: ist bei jedem Auftreten gleich oder verschieden CR¹, N oder C, wobei eine Gruppe Z¹ genau dann gleich C ist, wenn eine Gruppe Y daran gebunden ist;
- Ar¹: ist bei jedem Auftreten gleich oder verschieden ein aromatisches Ringsystem mit 6 bis 24 aromatischen Ringatomen, das mit einem oder mehreren Resten R² substituiert sein kann, oder ein heteroaromatisches Ringsystem mit 5 bis 24 aromatischen Ringatomen, das mit einem oder mehreren Resten R² substituiert sein kann;
- Cbz: ist eine divalente Gruppe, die an ihren freien Positionen jeweils mit einem Rest R³ substituiert ist, und die ein oder mehrere Strukturelemente der Formel (Cbz) enthält 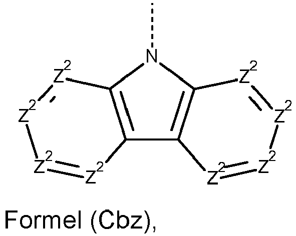
wobei eine der zwei Bindungen der divalenten Gruppe an den Rest der Verbindung die gestrichelte Bindung am Stickstoffatom der Formel (Cbz) ist,
wobei die zweite dieser zwei Bindungen an einer beliebigen freien Position der Gruppe angeordnet sein kann, und wobei
Z² bei jedem Auftreten gleich oder verschieden gewählt ist aus C und N;
- Ar²: ist gewählt aus Pyridin, Pyrimidin, Pyridazin, Pyrazin, Triazin, Benzimidazol, Chinolin, Chinazolin, Benzo[h]chinazolin, Phenanthrolin, Phenanthridin, Diazaphenanthren, und Acridin, die jeweils mit einem oder mehreren Resten R⁴ substituiert sein können;
- R¹, R², R³, R⁴: sind bei jedem Auftreten gleich oder verschieden gewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxy-gruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² bzw. R³ bzw. R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch - R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
- R⁵: ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
- R⁶: ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁶ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
- i: ist bei jedem Auftreten gleich oder verschieden 0 oder 1, wobei zwei der drei Indices i in Formel (I) gleich 1 sind, und einer der drei Indices i gleich 0 ist, und wobei die betreffende Gruppe Y nicht vorhanden ist, wenn der dazugehörige Index i = 0 ist;
- n: ist gleich 0, 1, 2, 3 oder 4;
- m: ist gleich 0, 1, 2, 3 oder 4.

Unter der Ausführungsform, bei der n = 2 ist, wird dabei verstanden, dass zwei Gruppen Ar¹ hintereinander gebunden sind, so dass eine Struktur 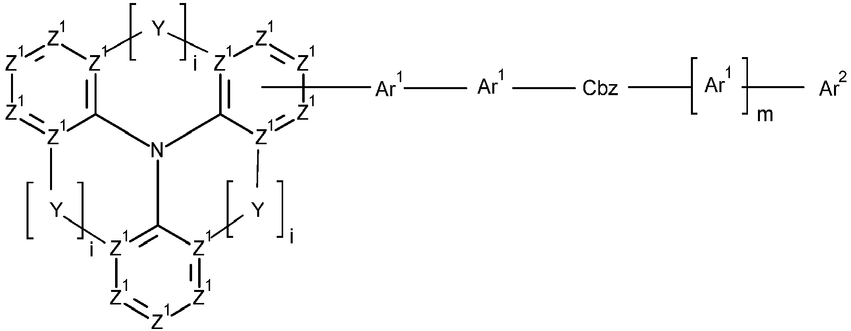 vorliegt.

Entsprechendes gilt für Ausführungsformen mit n=3 bzw. n=4, so dass dann drei bzw. vier Gruppen Ar¹ hintereinander gebunden sind.

Entsprechendes gilt für Ausführungsformen mit m=2 bzw. m=3 bzw. m=4.

Für n=0 bzw. m=0 gilt, dass die betreffende Gruppe Ar¹ nicht vorhanden ist, so dass die an diese Gruppe Ar¹ bindenden Gruppen direkt miteinander verbunden sind.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Unter einer elektronenarmen Heteroarylgruppe als Gruppe Ar², die mit einem oder mehreren Resten R⁴ substituiert sein kann, wird insbesondere eine Heteroarylgruppe mit 5 bis 30 aromatischen Ringatomen verstanden, die mindestens einen heteroaromatischen Fünfring mit zwei oder mehr Heteroatomen ausgewählt aus N, O und S enthält oder mindestens einen heteroaromatischen Sechsring mit einem oder mehr Heteroatomen ausgewählt aus N, O und S enthält.

Mögliche Ausführungsformen der elektronenarmen Heteroarylgruppe Ar² entsprechen einer der folgenden Formeln:

| | |
|---|---|
| 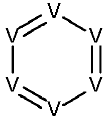 | 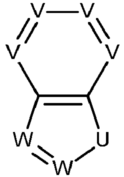 |
| (Ar²-A) | (Ar²-B) |
| 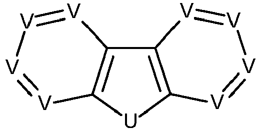 | 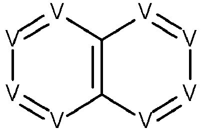 |
| (Ar²-C) | (Ar²-D) |
| 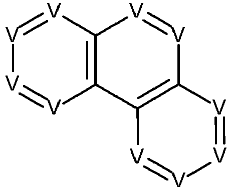 | |
| (Ar²-E) | |

wobei die auftretenden Variablen wie folgt definiert sind:
   - V: ist bei jedem Auftreten gleich oder verschieden N oder CR⁴, wobei in Formel (Ar²-A), (Ar²-C), (Ar²-D) und (Ar²-E) mindestens eine Gruppe V je Formel gleich N ist;
   - W: ist bei jedem Auftreten gleich oder verschieden N oder CR⁴;
   - U: ist gleich NR⁴;
wobei in Formel (Ar²-B) mindestens eine Gruppe gewählt aus Gruppen W und V gleich N ist; und
wobei eine Gruppe R⁴ je Formel durch die Bindung an die Gruppe Ar¹ bzw. Cbz ersetzt ist. Insofern der Umfang der Formeln (Ar²-A) bis (Ar²-E) breiter ist als der der anspruchsgemäßen Definition von Ar², gilt er als nicht anspruchsgemäß.

Ar² ist bevorzugt gewählt aus Pyridin, Pyrimidin, Pyridazin, Pyrazin, Triazin, Benzimidazol, Chinolin, Chinazolin, Benzo[h]chinazolin, Phenanthrolin, Phenanthridin, Diazaphenanthren, und Acridin, die jeweils mit einem oder mehreren Resten R⁴ substituiert sein können. Besonders bevorzugt sind Triazin und Chinazolin, die jeweils mit einem oder mehreren Resten R⁴ substituiert sein können, ganz besonders bevorzugt Triazin und 2-Chinazolin, die jeweils mit einem oder mehreren Resten R⁴ substituiert sein können.

Ganz besonders bevorzugte Gruppen Ar² sind gewählt aus Gruppen der folgenden Formeln

| | |
|---|---|
| 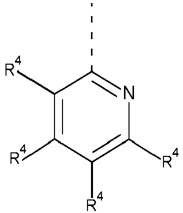 | 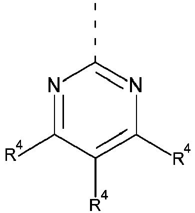 |
| Formel (Ar²-1) | Formel (Ar²-2) |
| 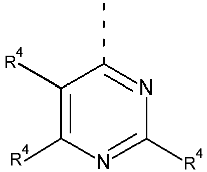 | 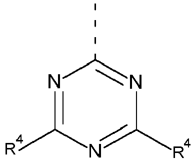 |
| Formel (Ar²-3) | Formel (Ar²-4) |
| 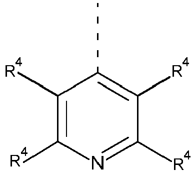 | 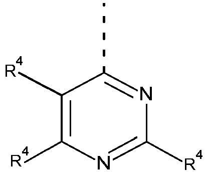 |
| Formel (Ar²-5) | Formel (Ar²-6) |
| 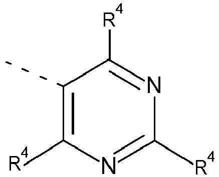 | 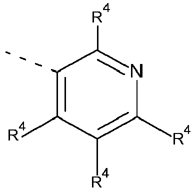 |
| Formel (Ar²-7) | Formel (Ar²-8) |
| 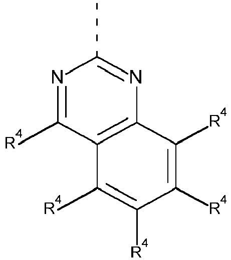 | 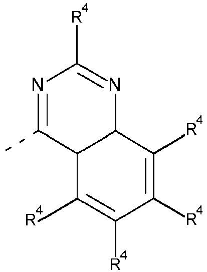 |
| Formel (Ar²-9) | Formel (Ar²-10) |
| 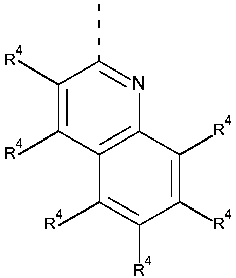 | 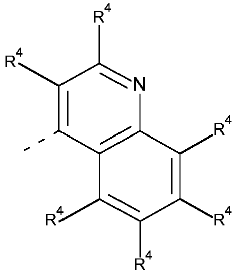 |
| Formel (Ar²-11) | Formel (Ar²-12) |
| 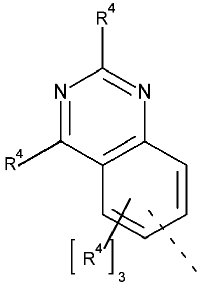 | 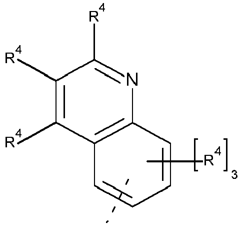 |
| Formel (Ar²-13) | Formel (Ar²-14) |
| 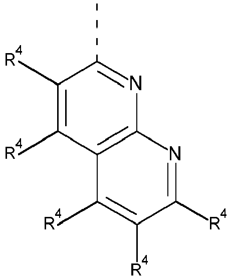 | 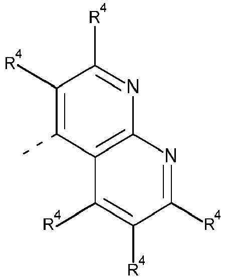 |
| #Formel (Ar²-15) | #Formel (Ar²-16) |
| 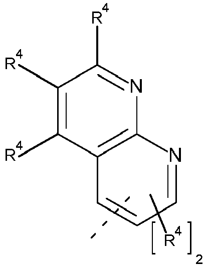 | 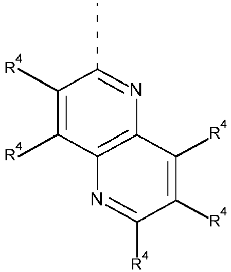 |
| #Formel (Ar²-17) | #Formel (Ar²-18) |
| 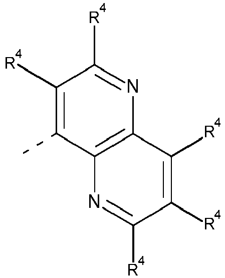 | 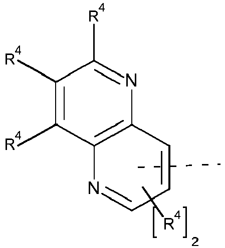 |
| #Formel (Ar²-19) | #Formel (Ar²-20) |
| 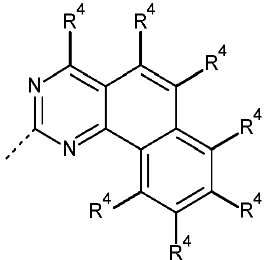 | 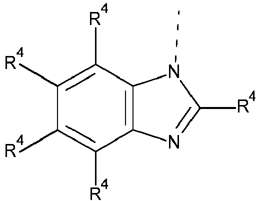 |
| Formel (Ar²-21) | Formel (Ar²-22) |
| 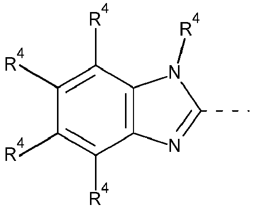 | |
| Formel (Ar²-23) | |

wobei die gestrichelte Bindung die Bindung an die Gruppe Ar¹ bzw. Cbz darstellt.

Die obenstehenden und im Folgenden mit # markierten Gruppen sind keine anspruchsgemäßen Ausführungsformen der Gruppe Ar².

Reste R⁴ in den oben genannten Formeln sind bevorzugt gewählt aus H und aromatischen Ringsystemen mit 6 bis 12 aromatischen Ringatomen, die jeweils mit einer oder mehreren Gruppen R⁵ substituiert sein können.

Besonders bevorzugt unter den oben genannten Formeln sind die Formeln (Ar²-1), (Ar²-2), (Ar²-4), (Ar²-6), (Ar²-7), (Ar²-9) und (Ar²-10).

Unter einer divalenten Gruppe, die ein oder mehrere Strukturelemente der Formel (Cbz) enthält, wird eine Gruppe verstanden, die i) neben dem Strukturelement der Formel (Cbz) weitere Strukturelemente enthält, die mit dem Strukturelement der Formel (Cbz) verbunden sind, und/oder die ii) das Strukturelement der Formel (Cbz) als einen Teil einer größeren zusammenhängenden Gruppe enthält.

Beispiele für den Fall ii) sind divalente Gruppen der untenstehenden Formeln:

| | |
|---|---|
| 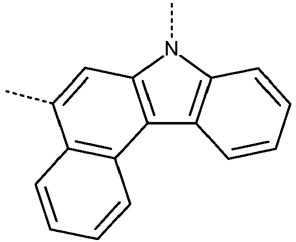 | 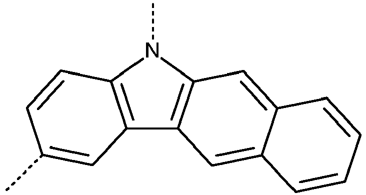 |
| (Cbz-Bsp-1) | (Cbz-Bsp-2) |
| 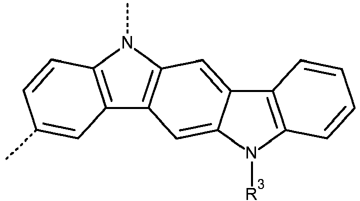 | 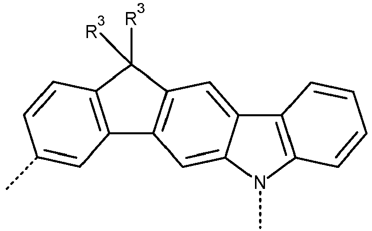 |
| (Cbz-Bsp-3) | (Cbz-Bsp-4). |

In Formel (Cbz) ist es bevorzugt, dass Z² gleich C ist.

Bevorzugte Gruppen Cbz sind gewählt aus Carbazol, Azacarbazol, Benzocarbazol, Dibenzocarbazol, Indenocarbazol, Indolocarbazol, Carbazol mit ankondensiertem Benzofuran und Carbazol mit ankondensiertem Benzothiophen, wobei die genannten Gruppen jeweils mit einem oder mehreren Resten R³ substituiert sein können.

Bevorzugte Gruppen Cbz entsprechen den folgenden Formeln:

| | |
|---|---|
| 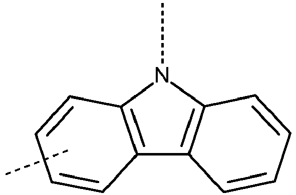 | 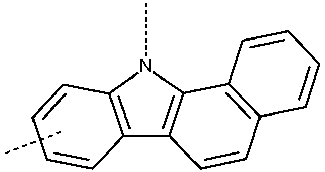 |
| Formel (Cbz-1) | Formel (Cbz-2) |
| 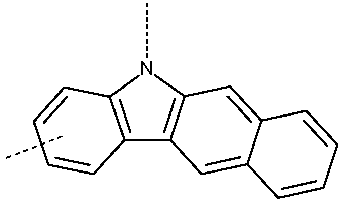 | 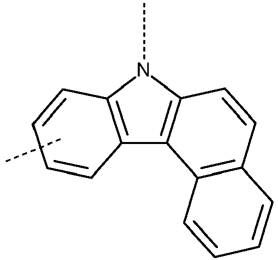 |
| Formel (Cbz-3) | Formel (Cbz-4) |
| 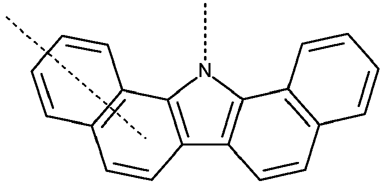 | 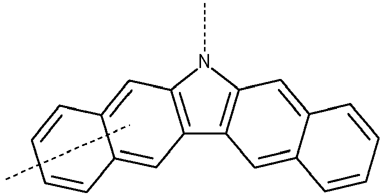 |
| Formel (Cbz-5) | Formel (Cbz-6) |
| 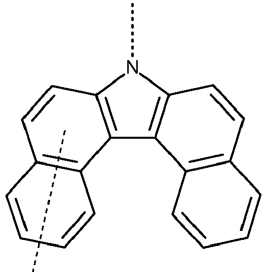 | 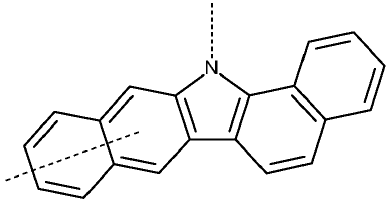 |
| Formel (Cbz-7) | Formel (Cbz-8) |
| 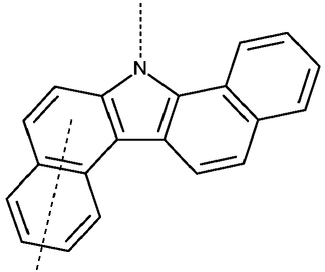 | 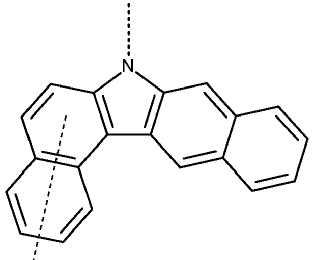 |
| Formel (Cbz-9) | Formel (Cbz-10) |
| 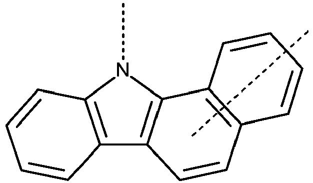 | 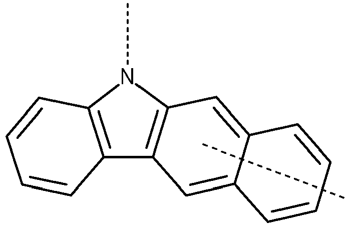 |
| Formel (Cbz-11) | Formel (Cbz-12) |
| 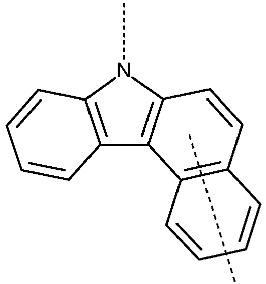 | 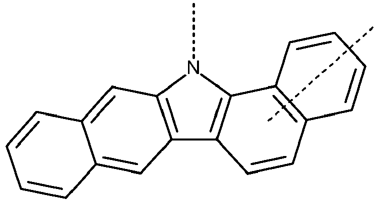 |
| Formel (Cbz-13) | Formel (Cbz-14) |
| 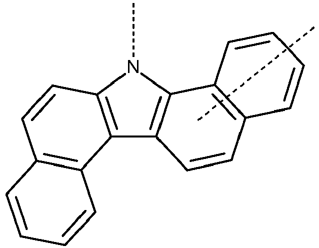 | 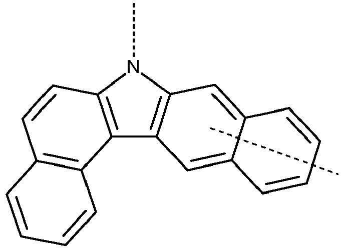 |
| Formel (Cbz-15) | Formel (Cbz-16) |
| 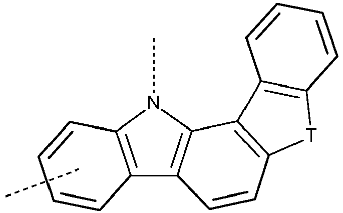 | 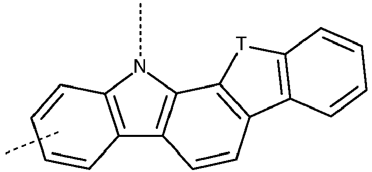 |
| Formel (Cbz-17) | Formel (Cbz-18) |
| 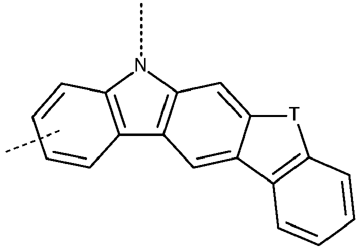 | 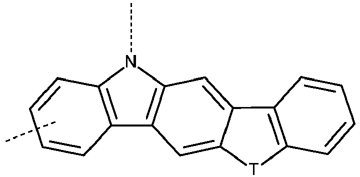 |
| Formel (Cbz-19) | Formel (Cbz-20) |
| 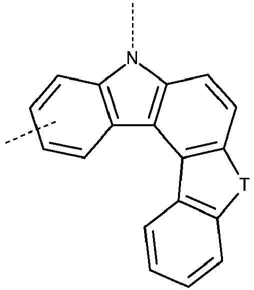 | 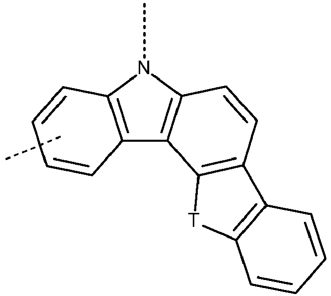 |
| Formel (Cbz-21) | Formel (Cbz-22) |
| 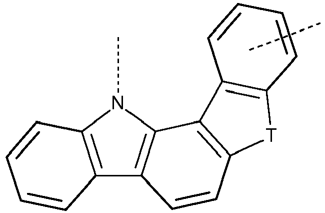 | 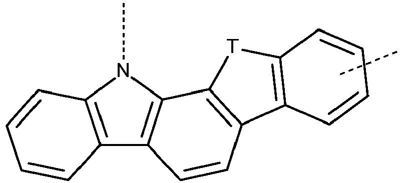 |
| Formel (Cbz-23) | Formel (Cbz-24) |
| 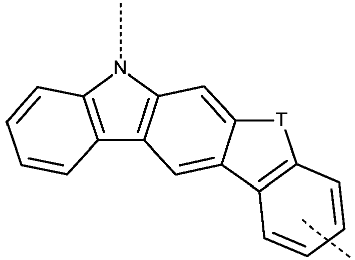 | 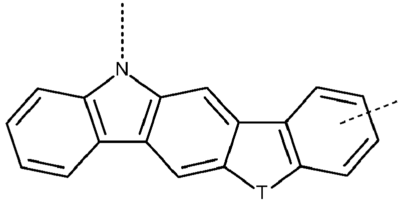 |
| Formel (Cbz-25) | Formel (Cbz-26) |
| 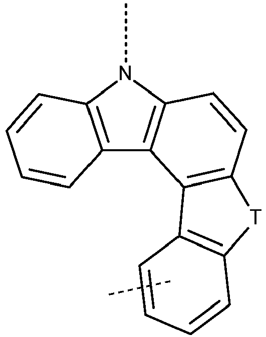 | 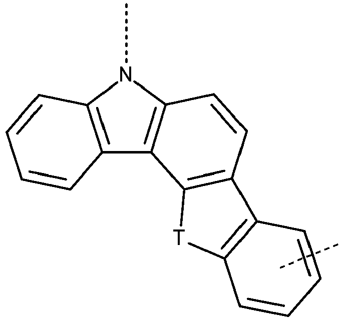 |
| Formel (Cbz-27) | Formel (Cbz-28) |
| 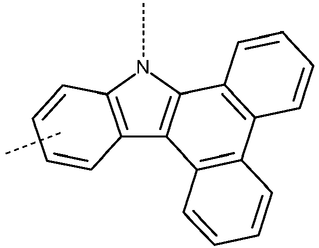 | |
| Formel (Cbz-29) | |

wobei gilt:
- T: ist gleich C(R³)₂, O, S oder NR³;

wobei die gestrichelten Bindungen die Bindungen an den Rest der Verbindung darstellen, und
wobei die Gruppen der oben genannten Formeln an allen unsubstituiert gezeichneten Positionen jeweils mit einem Rest R³ substituiert sein können.

Unter den oben genannten Formeln sind die Formeln (Cbz-1), (Cbz-2), (Cbz-4), (Cbz-18), (Cbz-19), (Cbz-20), (Cbz-22) und (Cbz-29) besonders bevorzugt. Ganz besonders bevorzugt ist die Formel (Cbz-1).

In den Formeln (Cbz-1) bis (Cbz-4) und (Cbz-17) bis (Cbz-22) ist es bevorzugt, dass diejenige Bindung an den Rest der Verbindung, die über den Sechsring erfolgt, in der Position para zum Stickstoffatom erfolgt.

Bevorzugte Ausführungsformen von Gruppen der Formel (Cbz-1) sind die im Folgenden gezeigten Gruppen der Formeln (Cbz-1-1) bis (Cbz-1-3), besonders bevorzugt ist (Cbz-1-2),

| | |
|---|---|
| 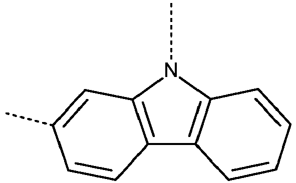 | 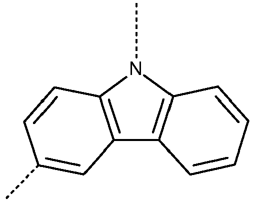 |
| Formel (Cbz-1-1) | Formel (Cbz-1-2) |
| 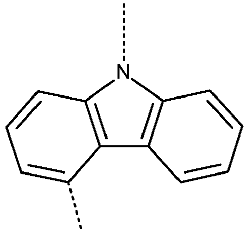 | |
| Formel (Cbz-1-3) | |

wobei die gestrichelten Bindungen die Bindungen an den Rest der Verbindung darstellen, und
wobei die Gruppen der oben genannten Formeln an allen unsubstituiert gezeichneten Positionen jeweils mit einem Rest R³ substituiert sein können.

Weiterhin ist es bevorzugt, dass die Gruppen der oben genannten Formeln an den unsubstituiert gezeichneten Positionen keinen Substituenten tragen.

Bevorzugt ist Y bei jedem Auftreten gleich gewählt. Weiterhin ist es bevorzugt, dass Y bei jedem Auftreten gleich oder verschieden gewählt ist aus O und S. Besonders bevorzugt ist Y gleich O.

Es ist bevorzugt, dass nicht mehr als 2 Gruppen Z¹ pro Formel (I) gleich N sind. Besonders bevorzugt ist Z¹ gleich CR¹ oder C, wobei eine Gruppe Z¹ genau dann gleich C ist, wenn eine Gruppe Y daran gebunden ist.

Bevorzugt ist Ar¹ bei jedem Auftreten gleich oder verschieden eine divalente Gruppe abgeleitet von den Grundkörpern Benzol, Biphenyl, Terphenyl, Naphthalin, Dibenzofuran, Dibenzothiophen, Carbazol, und Fluoren, wobei die divalente Gruppe mit einem oder mehreren Resten R² substituiert sein kann. Besonders bevorzugt ist Ar¹ 1,2-Phenylen, 1,3-Phenylen oder 1,4-Phenylen, die jeweils mit einem oder mehreren Resten R² substituiert sein können.

Bevorzugt sind R¹, R², R³ und R⁴ bei jedem Auftreten gleich oder verschieden gewählt aus H, D, F, CN, Si(R⁵)₃, N(R⁵)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁵ substituiert sein können; und wobei in den genannten Alkyl- oder Alkoxygruppen eine oder mehrere CH₂-Gruppen durch -C≡C-, -R⁵C=CR⁵-, Si(R⁵)₂, C=O, C=NR⁵, - NR⁵-, -O-, -S-, -C(=O)O- oder -C(=O)NR⁵- ersetzt sein können. Besonders bevorzugt sind R¹, R², R³ und R⁴ bei jedem Auftreten gleich oder verschieden gewählt aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁵ substituiert sein können.

Ganz besonders bevorzugt ist R¹ gleich H. Weiterhin ganz besonders bevorzugt ist R² gleich H. Weiterhin ganz besonders bevorzugt ist R³ gleich H. Weiterhin ganz besonders bevorzugt ist R⁴ bei jedem Auftreten gleich oder verschieden gewählt aus H, aromatischen Ringsystemen mit 6 bis 40 aromatischen Ringatomen, und heteroaromatischen Ringsystemen mit 5 bis 40 aromatischen Ringatomen.

Bevorzugt ist R⁵ bei jedem Auftreten gleich oder verschieden gewählt aus H, D, F, CN, Si(R⁶)₃, N(R⁶)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁶ substituiert sein können; und wobei in den genannten Alkyl- oder Alkoxygruppen eine oder mehrere CH₂-Gruppen durch -C=C-, - R⁶C=CR⁶-, Si(R⁶)₂, C=O, C=NR⁶, -NR⁶-, -O-, -S-, -C(=O)O- oder - C(=O)NR⁶- ersetzt sein können. Besonders bevorzugt ist R⁵ gleich H.

Bevorzugt ist R⁶ bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen. Besonders bevorzugt ist R⁶ gleich H.

Zwei der drei Indices i in Formel (I) sind gleich 1, und einer der drei Indices i ist gleich 0.

Bevorzugt ist m gleich 0 oder 1, besonders bevorzugt gleich 0.

Bevorzugt ist n gleich 0 oder 1, besonders bevorzugt gleich 0.

Bevorzugte Ausführungsformen der Formel (I) entsprechen den folgenden Formeln (I-1) bis (I-24)

| |
|---|
| 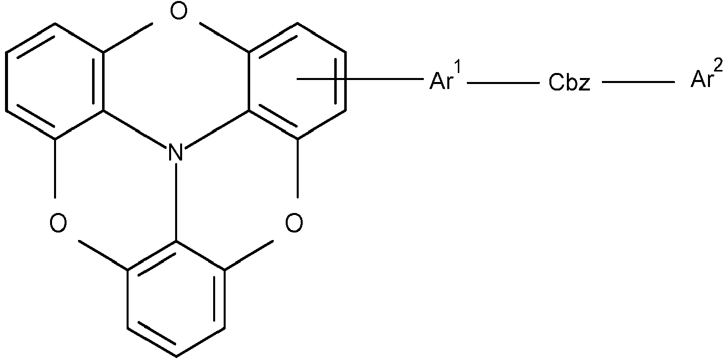 |
| #Formel (I-1) |
| 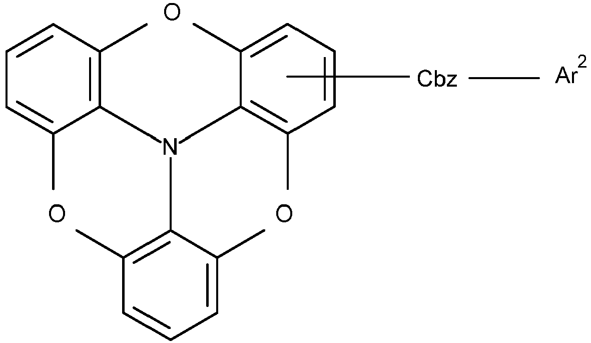 |
| #Formel (I-2) |
| 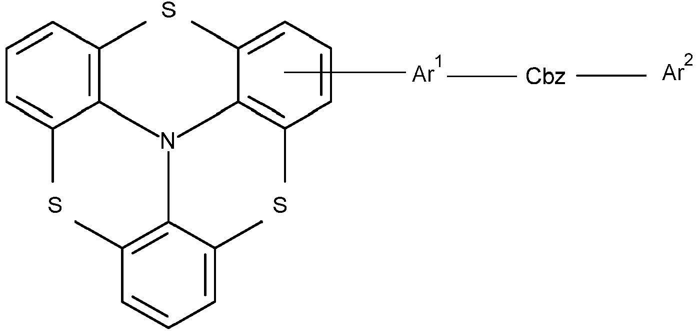 |
| #Formel (I-3) |
| 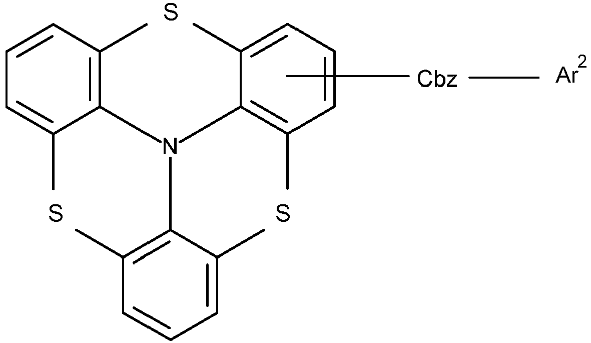 |
| #Formel (I-4) |
| 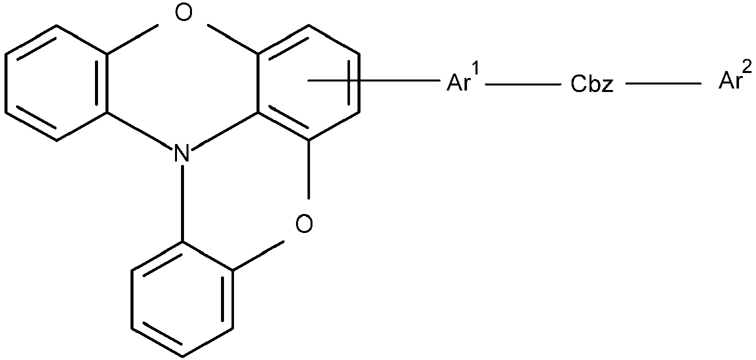 |
| Formel (I-5) |
| 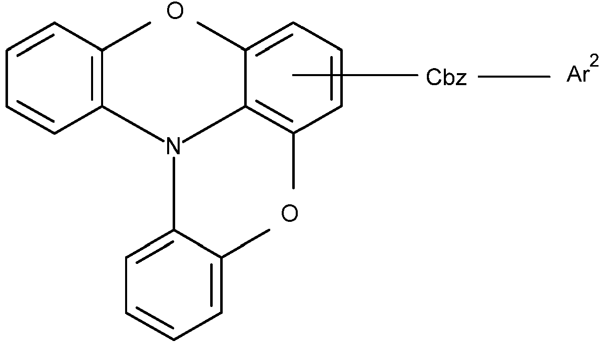 |
| Formel (I-6) |
| 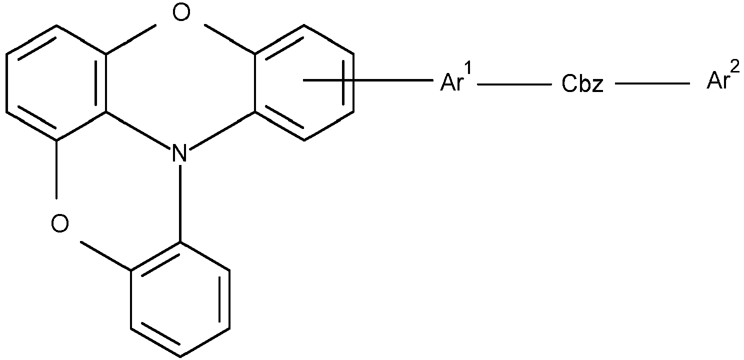 |
| Formel (I-7) |
| 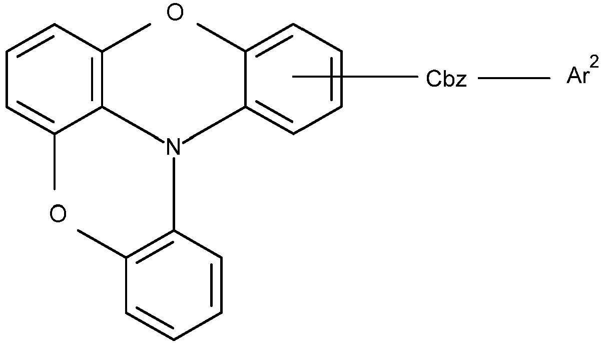 |
| Formel (I-8) |
| 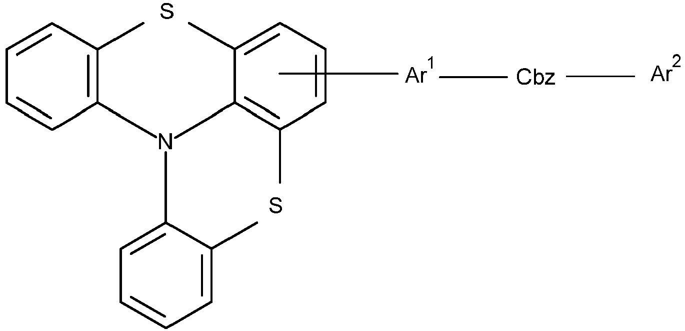 |
| Formel (I-9) |
| 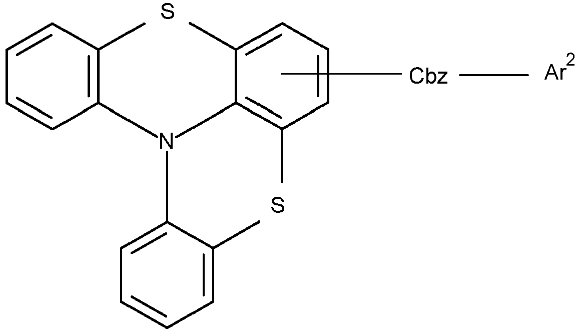 |
| Formel (I-10) |
| 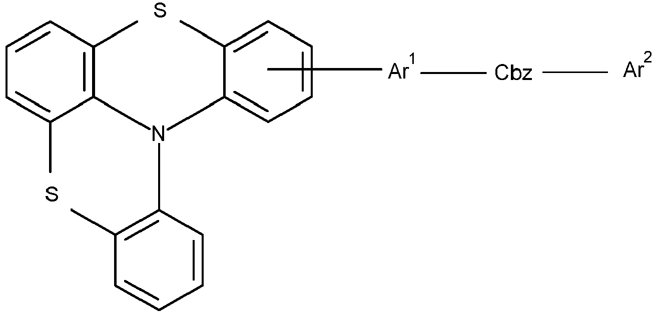 |
| Formel (I-11) |
| 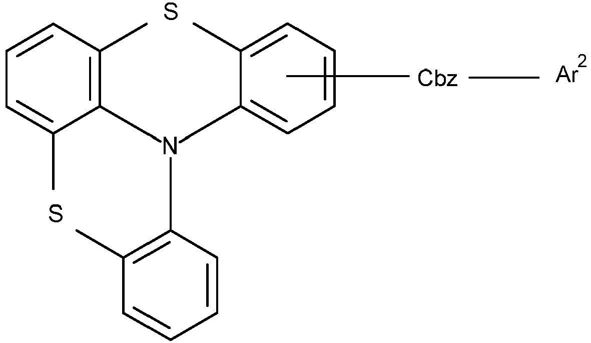 |
| Formel (I-12) |
| 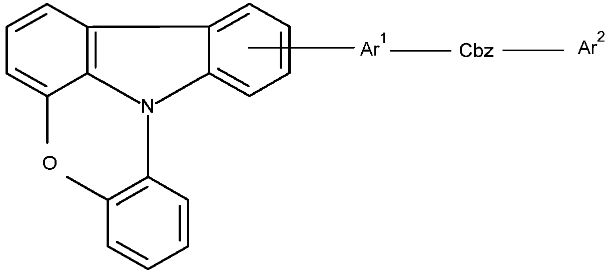 |
| Formel (I-13) |
| 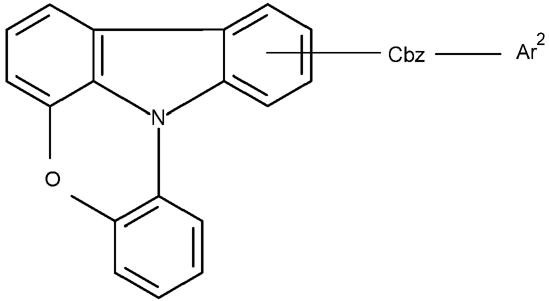 |
| Formel (I-14) |
| 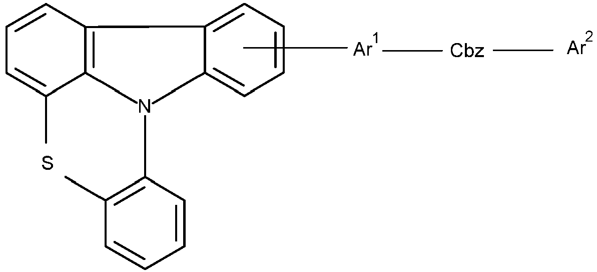 |
| Formel (I-15) |
| 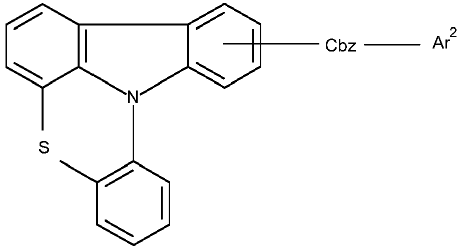 |
| Formel (I-16) |
| 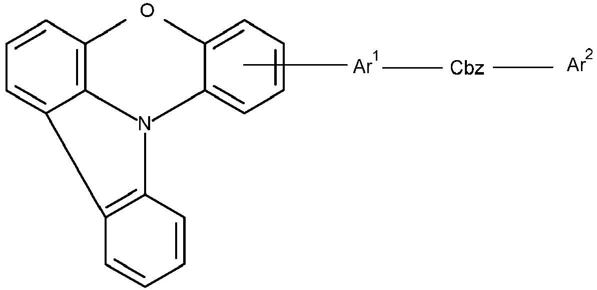 |
| Formel (I-17) |
| 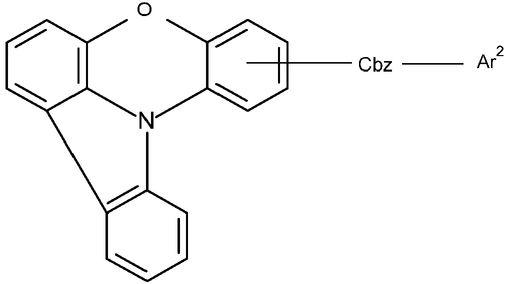 |
| Formel (I-18) |
| 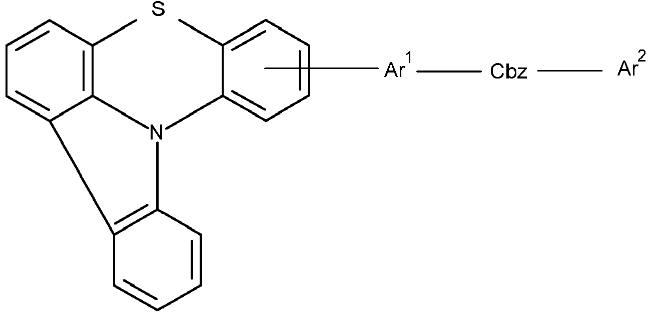 |
| Formel (I-19) |
| 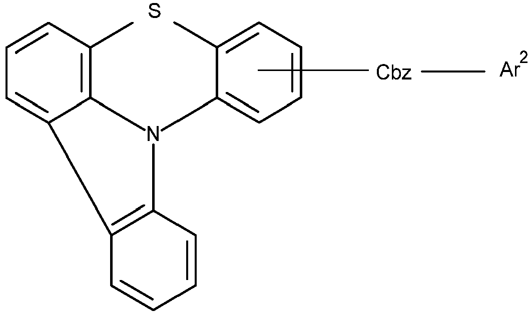 |
| Formel (I-20) |
| 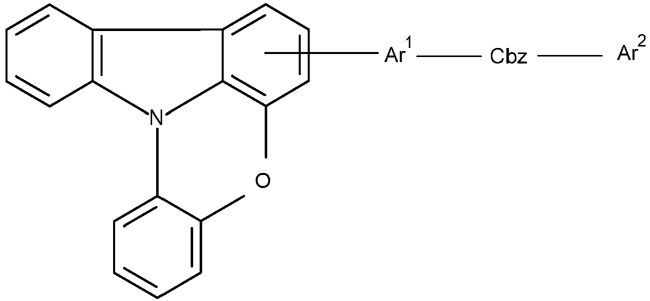 |
| Formel (I-21) |
| 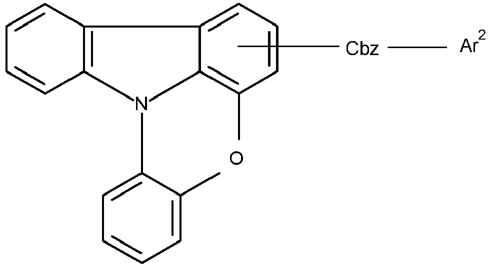 |
| Formel (I-22) |
| 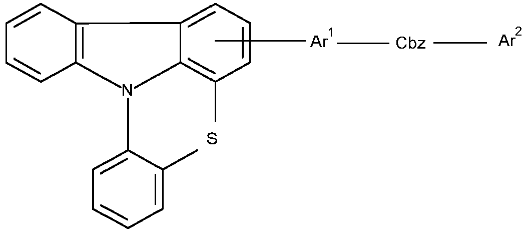 |
| Formel (I-23) |
| 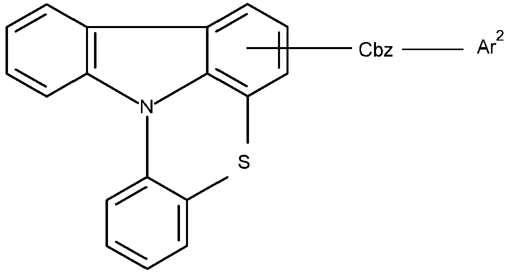 |
| Formel (I-24) |

wobei die auftretenden Variablen wie oben definiert sind, und die Formeln an den unsubstituiert gezeichneten Positionen an den aromatischen Sechsringen mit je einer Gruppe R¹ substituiert sein können.

Die obenstehenden und im Folgenden mit # markierten Verbindungen und Formeln sind nicht anspruchsgemäß.

Bevorzugt sind die Verbindungen der Formeln (I-1) bis (I-24) an allen unsubstituiert gezeichneten Positionen an den Sechsringen unsubstituiert.

Weiterhin ist es bevorzugt, dass die Verbindungsteile -Cbz-Ar² und -Ar¹-Cbz-Ar² in den Formeln (I-1) bis (I-24) jeweils in der Position para zum Stickstoffatom an den aromatischen Sechsring gebunden sind, wie am folgenden Beispiel für #Formel (I-1) gezeigt ist:

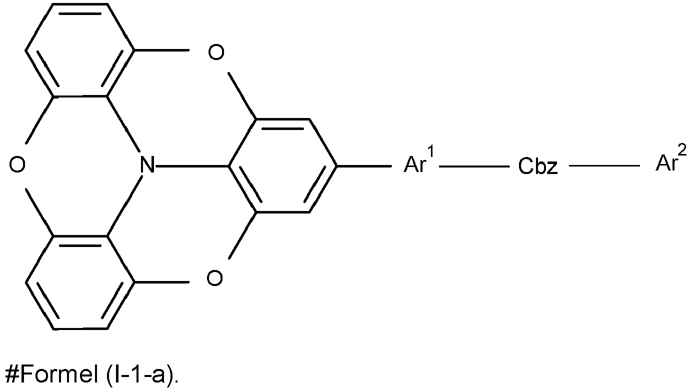

Für die Formeln (I-5) bis (I-24) gelten weiterhin die obenstehenden allgemein bevorzugten Ausführungsformen der Variablen.

Insbesondere bevorzugt sind die folgenden Ausführungsformen der Formeln (I-1) bis (I-24), in denen die Gruppe Cbz einer der bevorzugten Formeln (Cbz-1) bis (Cbz-29) entspricht:

| **Formel** | **Grundstruktur** | **Gruppe Cbz** |
|---|---|---|
| #Formel (I-1-1) | #Formel (I-1) | Formel (Cbz-1) |
| #Formel (I-1-2) | #Formel (I-1) | Formel (Cbz-2) |
| #Formel (I-1-3) | #Formel (I-1) | Formel (Cbz-3) |
| #Formel (I-1-4) | #Formel (I-1) | Formel (Cbz-4) |
| #Formel (I-1-5) | #Formel (I-1) | Formel (Cbz-5) |
| #Formel (I-1-6) | #Formel (I-1) | Formel (Cbz-6) |
| #Formel (I-1-7) | #Formel (I-1) | Formel (Cbz-7) |
| #Formel (I-1-8) | #Formel (I-1) | Formel (Cbz-8) |
| #Formel (I-1-9) | #Formel (I-1) | Formel (Cbz-9) |
| #Formel (I-1-10) | #Formel (I-1) | Formel (Cbz-10) |
| #Formel (I-1-11) | #Formel (I-1) | Formel (Cbz-11) |
| #Formel (I-1-12) | #Formel (I-1) | Formel (Cbz-12) |
| #Formel (I-1-13) | #Formel (I-1) | Formel (Cbz-13) |
| #Formel (I-1-14) | #Formel (I-1) | Formel (Cbz-14) |
| #Formel (I-1-15) | #Formel (I-1) | Formel (Cbz-15) |
| #Formel (I-1-16) | #Formel (I-1) | Formel (Cbz-16) |
| #Formel (I-1-17) | #Formel (I-1) | Formel (Cbz-17) |
| #Formel (I-1-18) | #Formel (I-1) | Formel (Cbz-18) |
| #Formel (I-1-19) | #Formel (I-1) | Formel (Cbz-19) |
| #Formel (I-1-20) | #Formel (I-1) | Formel (Cbz-20) |
| #Formel (I-1-21) | #Formel (I-1) | Formel (Cbz-21) |
| #Formel (I-1-22) | #Formel (I-1) | Formel (Cbz-22) |
| #Formel (I-1-23) | #Formel (I-1) | Formel (Cbz-23) |
| #Formel (I-1-24) | #Formel (I-1) | Formel (Cbz-24) |
| #Formel (I-1-25) | #Formel (I-1) | Formel (Cbz-25) |
| #Formel (I-1-26) | #Formel (I-1) | Formel (Cbz-26) |
| #Formel (I-1-27) | #Formel (I-1) | Formel (Cbz-27) |
| #Formel (I-1-28) | #Formel (I-1) | Formel (Cbz-28) |
| #Formel (I-1-29) | #Formel (I-1) | Formel (Cbz-29) |
| #Formel (I-2-1) | #Formel (I-2) | Formel (Cbz-1) |
| #Formel (I-2-2) | #Formel (I-2) | Formel (Cbz-2) |
| #Formel (I-2-3) | #Formel (I-2) | Formel (Cbz-3) |
| #Formel (I-2-4) | #Formel (I-2) | Formel (Cbz-4) |
| #Formel (I-2-5) | #Formel (I-2) | Formel (Cbz-5) |
| #Formel (I-2-6) | #Formel (I-2) | Formel (Cbz-6) |
| #Formel (I-2-7) | #Formel (I-2) | Formel (Cbz-7) |
| #Formel (I-2-8) | #Formel (I-2) | Formel (Cbz-8) |
| #Formel (I-2-9) | #Formel (I-2) | Formel (Cbz-9) |
| #Formel (I-2-10) | #Formel (I-2) | Formel (Cbz-10) |
| #Formel (I-2-11) | #Formel (I-2) | Formel (Cbz-11) |
| #Formel (I-2-12) | #Formel (I-2) | Formel (Cbz-12) |
| #Formel (I-2-13) | #Formel (I-2) | Formel (Cbz-13) |
| #Formel (I-2-14) | #Formel (I-2) | Formel (Cbz-14) |
| #Formel (I-2-15) | #Formel (I-2) | Formel (Cbz-15) |
| #Formel (I-2-16) | #Formel (I-2) | Formel (Cbz-16) |
| #Formel (I-2-17) | #Formel (I-2) | Formel (Cbz-17) |
| #Formel (I-2-18) | #Formel (I-2) | Formel (Cbz-18) |
| #Formel (I-2-19) | #Formel (I-2) | Formel (Cbz-19) |
| #Formel (I-2-20) | #Formel (I-2) | Formel (Cbz-20) |
| #Formel (I-2-21) | #Formel (I-2) | Formel (Cbz-21) |
| #Formel (I-2-22) | #Formel (I-2) | Formel (Cbz-22) |
| #Formel (I-2-23) | #Formel (I-2) | Formel (Cbz-23) |
| #Formel (I-2-24) | #Formel (I-2) | Formel (Cbz-24) |
| #Formel (I-2-25) | #Formel (I-2) | Formel (Cbz-25) |
| #Formel (I-2-26) | #Formel (I-2) | Formel (Cbz-26) |
| #Formel (I-2-27) | #Formel (I-2) | Formel (Cbz-27) |
| #Formel (I-2-28) | #Formel (I-2) | Formel (Cbz-28) |
| #Formel (I-2-29) | #Formel (I-2) | Formel (Cbz-29) |
| #Formel (I-3-1) | #Formel (I-3) | Formel (Cbz-1) |
| #Formel (I-3-2) | #Formel (I-3) | Formel (Cbz-2) |
| #Formel (I-3-3) | #Formel (I-3) | Formel (Cbz-3) |
| #Formel (I-3-4) | #Formel (I-3) | Formel (Cbz-4) |
| #Formel (I-3-5) | #Formel (I-3) | Formel (Cbz-5) |
| #Formel (I-3-6) | #Formel (I-3) | Formel (Cbz-6) |
| #Formel (I-3-7) | #Formel (I-3) | Formel (Cbz-7) |
| #Formel (I-3-8) | #Formel (I-3) | Formel (Cbz-8) |
| #Formel (I-3-9) | #Formel (I-3) | Formel (Cbz-9) |
| #Formel (I-3-10) | #Formel (I-3) | Formel (Cbz-10) |
| #Formel (I-3-11) | #Formel (I-3) | Formel (Cbz-11) |
| #Formel (I-3-12) | #Formel (I-3) | Formel (Cbz-12) |
| #Formel (I-3-13) | #Formel (I-3) | Formel (Cbz-13) |
| #Formel (I-3-14) | #Formel (I-3) | Formel (Cbz-14) |
| #Formel (I-3-15) | #Formel (I-3) | Formel (Cbz-15) |
| #Formel (I-3-16) | #Formel (I-3) | Formel (Cbz-16) |
| #Formel (I-3-17) | #Formel (I-3) | Formel (Cbz-17) |
| #Formel (I-3-18) | #Formel (I-3) | Formel (Cbz-18) |
| #Formel (I-3-19) | #Formel (I-3) | Formel (Cbz-19) |
| #Formel (I-3-20) | #Formel (I-3) | Formel (Cbz-20) |
| #Formel (I-3-21) | #Formel (I-3) | Formel (Cbz-21) |
| #Formel (I-3-22) | #Formel (I-3) | Formel (Cbz-22) |
| #Formel (I-3-23) | #Formel (I-3) | Formel (Cbz-23) |
| #Formel (I-3-24) | #Formel (I-3) | Formel (Cbz-24) |
| #Formel (I-3-25) | #Formel (I-3) | Formel (Cbz-25) |
| #Formel (I-3-26) | #Formel (I-3) | Formel (Cbz-26) |
| #Formel (I-3-27) | #Formel (I-3) | Formel (Cbz-27) |
| #Formel (I-3-28) | #Formel (I-3) | Formel (Cbz-28) |
| #Formel (I-3-29) | #Formel (I-3) | Formel (Cbz-29) |
| #Formel (I-4-1) | #Formel (I-4) | Formel (Cbz-1) |
| #Formel (I-4-2) | #Formel (I-4) | Formel (Cbz-2) |
| #Formel (I-4-3) | #Formel (I-4) | Formel (Cbz-3) |
| #Formel (I-4-4) | #Formel (I-4) | Formel (Cbz-4) |
| #Formel (I-4-5) | #Formel (I-4) | Formel (Cbz-5) |
| #Formel (I-4-6) | #Formel (I-4) | Formel (Cbz-6) |
| #Formel (I-4-7) | #Formel (I-4) | Formel (Cbz-7) |
| #Formel (I-4-8) | #Formel (I-4) | Formel (Cbz-8) |
| #Formel (I-4-9) | #Formel (I-4) | Formel (Cbz-9) |
| #Formel (I-4-10) | #Formel (I-4) | Formel (Cbz-10) |
| #Formel (I-4-11) | #Formel (I-4) | Formel (Cbz-11) |
| #Formel (I-4-12) | #Formel (I-4) | Formel (Cbz-12) |
| #Formel (I-4-13) | #Formel (I-4) | Formel (Cbz-13) |
| #Formel (I-4-14) | #Formel (I-4) | Formel (Cbz-14) |
| #Formel (I-4-15) | #Formel (I-4) | Formel (Cbz-15) |
| #Formel (I-4-16) | #Formel (I-4) | Formel (Cbz-16) |
| #Formel (I-4-17) | #Formel (I-4) | Formel (Cbz-17) |
| #Formel (I-4-18) | #Formel (I-4) | Formel (Cbz-18) |
| #Formel (I-4-19) | #Formel (I-4) | Formel (Cbz-19) |
| #Formel (I-4-20) | #Formel (I-4) | Formel (Cbz-20) |
| #Formel (I-4-21) | #Formel (I-4) | Formel (Cbz-21) |
| #Formel (I-4-22) | #Formel (I-4) | Formel (Cbz-22) |
| #Formel (I-4-23) | #Formel (I-4) | Formel (Cbz-23) |
| #Formel (I-4-24) | #Formel (I-4) | Formel (Cbz-24) |
| #Formel (I-4-25) | #Formel (I-4) | Formel (Cbz-25) |
| #Formel (I-4-26) | #Formel (I-4) | Formel (Cbz-26) |
| #Formel (I-4-27) | #Formel (I-4) | Formel (Cbz-27) |
| #Formel (I-4-28) | #Formel (I-4) | Formel (Cbz-28) |
| #Formel (I-4-29) | #Formel (I-4) | Formel (Cbz-29) |
| Formel (I-5-1) | Formel (I-5) | Formel (Cbz-1) |
| Formel (I-5-2) | Formel (I-5) | Formel (Cbz-2) |
| Formel (I-5-3) | Formel (I-5) | Formel (Cbz-3) |
| Formel (I-5-4) | Formel (I-5) | Formel (Cbz-4) |
| Formel (I-5-5) | Formel (I-5) | Formel (Cbz-5) |
| Formel (I-5-6) | Formel (I-5) | Formel (Cbz-6) |
| Formel (I-5-7) | Formel (I-5) | Formel (Cbz-7) |
| Formel (I-5-8) | Formel (I-5) | Formel (Cbz-8) |
| Formel (I-5-9) | Formel (I-5) | Formel (Cbz-9) |
| Formel (I-5-10) | Formel (I-5) | Formel (Cbz-10) |
| Formel (I-5-11) | Formel (I-5) | Formel (Cbz-11) |
| Formel (I-5-12) | Formel (I-5) | Formel (Cbz-12) |
| Formel (I-5-13) | Formel (I-5) | Formel (Cbz-13) |
| Formel (I-5-14) | Formel (I-5) | Formel (Cbz-14) |
| Formel (I-5-15) | Formel (I-5) | Formel (Cbz-15) |
| Formel (I-5-16) | Formel (I-5) | Formel (Cbz-16) |
| Formel (I-5-17) | Formel (I-5) | Formel (Cbz-17) |
| Formel (I-5-18) | Formel (I-5) | Formel (Cbz-18) |
| Formel (I-5-19) | Formel (I-5) | Formel (Cbz-19) |
| Formel (I-5-20) | Formel (I-5) | Formel (Cbz-20) |
| Formel (I-5-21) | Formel (I-5) | Formel (Cbz-21) |
| Formel (I-5-22) | Formel (I-5) | Formel (Cbz-22) |
| Formel (I-5-23) | Formel (I-5) | Formel (Cbz-23) |
| Formel (I-5-24) | Formel (I-5) | Formel (Cbz-24) |
| Formel (I-5-25) | Formel (I-5) | Formel (Cbz-25) |
| Formel (I-5-26) | Formel (I-5) | Formel (Cbz-26) |
| Formel (I-5-27) | Formel (I-5) | Formel (Cbz-27) |
| Formel (I-5-28) | Formel (I-5) | Formel (Cbz-28) |
| Formel (I-5-29) | Formel (I-5) | Formel (Cbz-29) |
| Formel (I-6-1) | Formel (I-6) | Formel (Cbz-1) |
| Formel (I-6-2) | Formel (I-6) | Formel (Cbz-2) |
| Formel (I-6-3) | Formel (I-6) | Formel (Cbz-3) |
| Formel (I-6-4) | Formel (I-6) | Formel (Cbz-4) |
| Formel (I-6-5) | Formel (I-6) | Formel (Cbz-5) |
| Formel (I-6-6) | Formel (I-6) | Formel (Cbz-6) |
| Formel (I-6-7) | Formel (I-6) | Formel (Cbz-7) |
| Formel (I-6-8) | Formel (I-6) | Formel (Cbz-8) |
| Formel (I-6-9) | Formel (I-6) | Formel (Cbz-9) |
| Formel (I-6-10) | Formel (I-6) | Formel (Cbz-10) |
| Formel (I-6-11) | Formel (I-6) | Formel (Cbz-11) |
| Formel (I-6-12) | Formel (I-6) | Formel (Cbz-12) |
| Formel (I-6-13) | Formel (I-6) | Formel (Cbz-13) |
| Formel (I-6-14) | Formel (I-6) | Formel (Cbz-14) |
| Formel (I-6-15) | Formel (I-6) | Formel (Cbz-15) |
| Formel (I-6-16) | Formel (I-6) | Formel (Cbz-16) |
| Formel (I-6-17) | Formel (I-6) | Formel (Cbz-17) |
| Formel (I-6-18) | Formel (I-6) | Formel (Cbz-18) |
| Formel (I-6-19) | Formel (I-6) | Formel (Cbz-19) |
| Formel (I-6-20) | Formel (I-6) | Formel (Cbz-20) |
| Formel (I-6-21) | Formel (I-6) | Formel (Cbz-21) |
| Formel (I-6-22) | Formel (I-6) | Formel (Cbz-22) |
| Formel (I-6-23) | Formel (I-6) | Formel (Cbz-23) |
| Formel (I-6-24) | Formel (I-6) | Formel (Cbz-24) |
| Formel (I-6-25) | Formel (I-6) | Formel (Cbz-25) |
| Formel (I-6-26) | Formel (I-6) | Formel (Cbz-26) |
| Formel (I-6-27) | Formel (I-6) | Formel (Cbz-27) |
| Formel (I-6-28) | Formel (I-6) | Formel (Cbz-28) |
| Formel (I-6-29) | Formel (I-6) | Formel (Cbz-29) |
| Formel (I-7-1) | Formel (I-7) | Formel (Cbz-1) |
| Formel (I-7-2) | Formel (I-7) | Formel (Cbz-2) |
| Formel (I-7-3) | Formel (I-7) | Formel (Cbz-3) |
| Formel (I-7-4) | Formel (I-7) | Formel (Cbz-4) |
| Formel (I-7-5) | Formel (I-7) | Formel (Cbz-5) |
| Formel (I-7-6) | Formel (I-7) | Formel (Cbz-6) |
| Formel (I-7-7) | Formel (I-7) | Formel (Cbz-7) |
| Formel (I-7-8) | Formel (I-7) | Formel (Cbz-8) |
| Formel (I-7-9) | Formel (I-7) | Formel (Cbz-9) |
| Formel (I-7-10) | Formel (I-7) | Formel (Cbz-10) |
| Formel (I-7-11) | Formel (I-7) | Formel (Cbz-11) |
| Formel (I-7-12) | Formel (I-7) | Formel (Cbz-12) |
| Formel (I-7-13) | Formel (I-7) | Formel (Cbz-13) |
| Formel (I-7-14) | Formel (I-7) | Formel (Cbz-14) |
| Formel (I-7-15) | Formel (I-7) | Formel (Cbz-15) |
| Formel (I-7-16) | Formel (I-7) | Formel (Cbz-16) |
| Formel (I-7-17) | Formel (I-7) | Formel (Cbz-17) |
| Formel (I-7-18) | Formel (I-7) | Formel (Cbz-18) |
| Formel (I-7-19) | Formel (I-7) | Formel (Cbz-19) |
| Formel (I-7-20) | Formel (I-7) | Formel (Cbz-20) |
| Formel (I-7-21) | Formel (I-7) | Formel (Cbz-21) |
| Formel (I-7-22) | Formel (I-7) | Formel (Cbz-22) |
| Formel (I-7-23) | Formel (I-7) | Formel (Cbz-23) |
| Formel (I-7-24) | Formel (I-7) | Formel (Cbz-24) |
| Formel (I-7-25) | Formel (I-7) | Formel (Cbz-25) |
| Formel (I-7-26) | Formel (I-7) | Formel (Cbz-26) |
| Formel (I-7-27) | Formel (I-7) | Formel (Cbz-27) |
| Formel (I-7-28) | Formel (I-7) | Formel (Cbz-28) |
| Formel (I-7-29) | Formel (I-7) | Formel (Cbz-29) |
| Formel (I-8-1) | Formel (I-8) | Formel (Cbz-1) |
| Formel (I-8-2) | Formel (I-8) | Formel (Cbz-2) |
| Formel (I-8-3) | Formel (I-8) | Formel (Cbz-3) |
| Formel (I-8-4) | Formel (I-8) | Formel (Cbz-4) |
| Formel (I-8-5) | Formel (I-8) | Formel (Cbz-5) |
| Formel (I-8-6) | Formel (I-8) | Formel (Cbz-6) |
| Formel (I-8-7) | Formel (I-8) | Formel (Cbz-7) |
| Formel (I-8-8) | Formel (I-8) | Formel (Cbz-8) |
| Formel (I-8-9) | Formel (I-8) | Formel (Cbz-9) |
| Formel (I-8-10) | Formel (I-8) | Formel (Cbz-10) |
| Formel (I-8-11) | Formel (I-8) | Formel (Cbz-11) |
| Formel (I-8-12) | Formel (I-8) | Formel (Cbz-12) |
| Formel (I-8-13) | Formel (I-8) | Formel (Cbz-13) |
| Formel (I-8-14) | Formel (I-8) | Formel (Cbz-14) |
| Formel (I-8-15) | Formel (I-8) | Formel (Cbz-15) |
| Formel (I-8-16) | Formel (I-8) | Formel (Cbz-16) |
| Formel (I-8-17) | Formel (I-8) | Formel (Cbz-17) |
| Formel (I-8-18) | Formel (I-8) | Formel (Cbz-18) |
| Formel (I-8-19) | Formel (I-8) | Formel (Cbz-19) |
| Formel (I-8-20) | Formel (I-8) | Formel (Cbz-20) |
| Formel (I-8-21) | Formel (I-8) | Formel (Cbz-21) |
| Formel (I-8-22) | Formel (I-8) | Formel (Cbz-22) |
| Formel (I-8-23) | Formel (I-8) | Formel (Cbz-23) |
| Formel (I-8-24) | Formel (I-8) | Formel (Cbz-24) |
| Formel (I-8-25) | Formel (I-8) | Formel (Cbz-25) |
| Formel (I-8-26) | Formel (I-8) | Formel (Cbz-26) |
| Formel (I-8-27) | Formel (I-8) | Formel (Cbz-27) |
| Formel (I-8-28) | Formel (I-8) | Formel (Cbz-28) |
| Formel (I-8-29) | Formel (I-8) | Formel (Cbz-29) |
| Formel (I-9-1) | Formel (I-9) | Formel (Cbz-1) |
| Formel (I-9-2) | Formel (I-9) | Formel (Cbz-2) |
| Formel (I-9-3) | Formel (I-9) | Formel (Cbz-3) |
| Formel (I-9-4) | Formel (I-9) | Formel (Cbz-4) |
| Formel (I-9-5) | Formel (I-9) | Formel (Cbz-5) |
| Formel (I-9-6) | Formel (I-9) | Formel (Cbz-6) |
| Formel (I-9-7) | Formel (I-9) | Formel (Cbz-7) |
| Formel (I-9-8) | Formel (I-9) | Formel (Cbz-8) |
| Formel (I-9-9) | Formel (I-9) | Formel (Cbz-9) |
| Formel (I-9-10) | Formel (I-9) | Formel (Cbz-10) |
| Formel (I-9-11) | Formel (I-9) | Formel (Cbz-11) |
| Formel (I-9-12) | Formel (I-9) | Formel (Cbz-12) |
| Formel (I-9-13) | Formel (I-9) | Formel (Cbz-13) |
| Formel (I-9-14) | Formel (I-9) | Formel (Cbz-14) |
| Formel (I-9-15) | Formel (I-9) | Formel (Cbz-15) |
| Formel (I-9-16) | Formel (I-9) | Formel (Cbz-16) |
| Formel (I-9-17) | Formel (I-9) | Formel (Cbz-17) |
| Formel (I-9-18) | Formel (I-9) | Formel (Cbz-18) |
| Formel (I-9-19) | Formel (I-9) | Formel (Cbz-19) |
| Formel (I-9-20) | Formel (I-9) | Formel (Cbz-20) |
| Formel (I-9-21) | Formel (I-9) | Formel (Cbz-21) |
| Formel (I-9-22) | Formel (I-9) | Formel (Cbz-22) |
| Formel (I-9-23) | Formel (I-9) | Formel (Cbz-23) |
| Formel (I-9-24) | Formel (I-9) | Formel (Cbz-24) |
| Formel (I-9-25) | Formel (I-9) | Formel (Cbz-25) |
| Formel (I-9-26) | Formel (I-9) | Formel (Cbz-26) |
| Formel (I-9-27) | Formel (I-9) | Formel (Cbz-27) |
| Formel (I-9-28) | Formel (I-9) | Formel (Cbz-28) |
| Formel (I-9-29) | Formel (I-9) | Formel (Cbz-29) |
| Formel (I-10-1) | Formel (I-10) | Formel (Cbz-1) |
| Formel (I-10-2) | Formel (I-10) | Formel (Cbz-2) |
| Formel (I-10-3) | Formel (I-10) | Formel (Cbz-3) |
| Formel (I-10-4) | Formel (I-10) | Formel (Cbz-4) |
| Formel (I-10-5) | Formel (I-10) | Formel (Cbz-5) |
| Formel (I-10-6) | Formel (I-10) | Formel (Cbz-6) |
| Formel (I-10-7) | Formel (I-10) | Formel (Cbz-7) |
| Formel (I-10-8) | Formel (I-10) | Formel (Cbz-8) |
| Formel (I-10-9) | Formel (I-10) | Formel (Cbz-9) |
| Formel (I-10-10) | Formel (I-10) | Formel (Cbz-10) |
| Formel (I-10-11) | Formel (I-10) | Formel (Cbz-11) |
| Formel (I-10-12) | Formel (I-10) | Formel (Cbz-12) |
| Formel (I-10-13) | Formel (I-10) | Formel (Cbz-13) |
| Formel (I-10-14) | Formel (I-10) | Formel (Cbz-14) |
| Formel (I-10-15) | Formel (I-10) | Formel (Cbz-15) |
| Formel (I-10-16) | Formel (I-10) | Formel (Cbz-16) |
| Formel (I-10-17) | Formel (I-10) | Formel (Cbz-17) |
| Formel (I-10-18) | Formel (I-10) | Formel (Cbz-18) |
| Formel (I-10-19) | Formel (I-10) | Formel (Cbz-19) |
| Formel (I-10-20) | Formel (I-10) | Formel (Cbz-20) |
| Formel (I-10-21) | Formel (I-10) | Formel (Cbz-21) |
| Formel (I-10-22) | Formel (I-10) | Formel (Cbz-22) |
| Formel (I-10-23) | Formel (I-10) | Formel (Cbz-23) |
| Formel (I-10-24) | Formel (I-10) | Formel (Cbz-24) |
| Formel (I-10-25) | Formel (I-10) | Formel (Cbz-25) |
| Formel (I-10-26) | Formel (I-10) | Formel (Cbz-26) |
| Formel (I-10-27) | Formel (I-10) | Formel (Cbz-27) |
| Formel (I-10-28) | Formel (I-10) | Formel (Cbz-28) |
| Formel (I-10-29) | Formel (I-10) | Formel (Cbz-29) |
| Formel (I-11-1) | Formel (I-11) | Formel (Cbz-1) |
| Formel (I-11-2) | Formel (I-11) | Formel (Cbz-2) |
| Formel (I-11-3) | Formel (I-11) | Formel (Cbz-3) |
| Formel (I-11-4) | Formel (I-11) | Formel (Cbz-4) |
| Formel (I-11-5) | Formel (I-11) | Formel (Cbz-5) |
| Formel (I-11-6) | Formel (I-11) | Formel (Cbz-6) |
| Formel (I-11-7) | Formel (I-11) | Formel (Cbz-7) |
| Formel (I-11-8) | Formel (I-11) | Formel (Cbz-8) |
| Formel (I-11-9) | Formel (I-11) | Formel (Cbz-9) |
| Formel (I-11-10) | Formel (I-11) | Formel (Cbz-10) |
| Formel (I-11-11) | Formel (I-11) | Formel (Cbz-11) |
| Formel (I-11-12) | Formel (I-11) | Formel (Cbz-12) |
| Formel (I-11-13) | Formel (I-11) | Formel (Cbz-13) |
| Formel (I-11-14) | Formel (I-11) | Formel (Cbz-14) |
| Formel (I-11-15) | Formel (I-11) | Formel (Cbz-15) |
| Formel (I-11-16) | Formel (I-11) | Formel (Cbz-16) |
| Formel (I-11-17) | Formel (I-11) | Formel (Cbz-17) |
| Formel (I-11-18) | Formel (I-11) | Formel (Cbz-18) |
| Formel (I-11-19) | Formel (I-11) | Formel (Cbz-19) |
| Formel (I-11-20) | Formel (I-11) | Formel (Cbz-20) |
| Formel (I-11-21) | Formel (I-11) | Formel (Cbz-21) |
| Formel (I-11-22) | Formel (I-11) | Formel (Cbz-22) |
| Formel (I-11-23) | Formel (I-11) | Formel (Cbz-23) |
| Formel (I-11-24) | Formel (I-11) | Formel (Cbz-24) |
| Formel (I-11-25) | Formel (I-11) | Formel (Cbz-25) |
| Formel (I-11-26) | Formel (I-11) | Formel (Cbz-26) |
| Formel (I-11-27) | Formel (I-11) | Formel (Cbz-27) |
| Formel (I-11-28) | Formel (I-11) | Formel (Cbz-28) |
| Formel (I-11-29) | Formel (I-11) | Formel (Cbz-29) |
| Formel (I-12-1) | Formel (I-12) | Formel (Cbz-1) |
| Formel (I-12-2) | Formel (I-12) | Formel (Cbz-2) |
| Formel (I-12-3) | Formel (I-12) | Formel (Cbz-3) |
| Formel (I-12-4) | Formel (I-12) | Formel (Cbz-4) |
| Formel (I-12-5) | Formel (I-12) | Formel (Cbz-5) |
| Formel (I-12-6) | Formel (I-12) | Formel (Cbz-6) |
| Formel (I-12-7) | Formel (I-12) | Formel (Cbz-7) |
| Formel (I-12-8) | Formel (I-12) | Formel (Cbz-8) |
| Formel (I-12-9) | Formel (I-12) | Formel (Cbz-9) |
| Formel (I-12-10) | Formel (I-12) | Formel (Cbz-10) |
| Formel (I-12-11) | Formel (I-12) | Formel (Cbz-11) |
| Formel (I-12-12) | Formel (I-12) | Formel (Cbz-12) |
| Formel (I-12-13) | Formel (I-12) | Formel (Cbz-13) |
| Formel (I-12-14) | Formel (I-12) | Formel (Cbz-14) |
| Formel (I-12-15) | Formel (I-12) | Formel (Cbz-15) |
| Formel (I-12-16) | Formel (I-12) | Formel (Cbz-16) |
| Formel (I-12-17) | Formel (I-12) | Formel (Cbz-17) |
| Formel (I-12-18) | Formel (I-12) | Formel (Cbz-18) |
| Formel (I-12-19) | Formel (I-12) | Formel (Cbz-19) |
| Formel (I-12-20) | Formel (I-12) | Formel (Cbz-20) |
| Formel (I-12-21) | Formel (I-12) | Formel (Cbz-21) |
| Formel (I-12-22) | Formel (I-12) | Formel (Cbz-22) |
| Formel (I-12-23) | Formel (I-12) | Formel (Cbz-23) |
| Formel (I-12-24) | Formel (I-12) | Formel (Cbz-24) |
| Formel (I-12-25) | Formel (I-12) | Formel (Cbz-25) |
| Formel (I-12-26) | Formel (I-12) | Formel (Cbz-26) |
| Formel (I-12-27) | Formel (I-12) | Formel (Cbz-27) |
| Formel (I-12-28) | Formel (I-12) | Formel (Cbz-28) |
| Formel (I-12-29) | Formel (I-12) | Formel (Cbz-29) |
| Formel (I-13-1) | Formel (I-13) | Formel (Cbz-1) |
| Formel (I-13-2) | Formel (I-13) | Formel (Cbz-2) |
| Formel (I-13-3) | Formel (I-13) | Formel (Cbz-3) |
| Formel (I-13-4) | Formel (I-13) | Formel (Cbz-4) |
| Formel (I-13-5) | Formel (I-13) | Formel (Cbz-5) |
| Formel (I-13-6) | Formel (I-13) | Formel (Cbz-6) |
| Formel (I-13-7) | Formel (I-13) | Formel (Cbz-7) |
| Formel (I-13-8) | Formel (I-13) | Formel (Cbz-8) |
| Formel (I-13-9) | Formel (I-13) | Formel (Cbz-9) |
| Formel (I-13-10) | Formel (I-13) | Formel (Cbz-10) |
| Formel (I-13-11) | Formel (I-13) | Formel (Cbz-11) |
| Formel (I-13-12) | Formel (I-13) | Formel (Cbz-12) |
| Formel (I-13-13) | Formel (I-13) | Formel (Cbz-13) |
| Formel (I-13-14) | Formel (I-13) | Formel (Cbz-14) |
| Formel (I-13-15) | Formel (I-13) | Formel (Cbz-15) |
| Formel (I-13-16) | Formel (I-13) | Formel (Cbz-16) |
| Formel (I-13-17) | Formel (I-13) | Formel (Cbz-17) |
| Formel (I-13-18) | Formel (I-13) | Formel (Cbz-18) |
| Formel (I-13-19) | Formel (I-13) | Formel (Cbz-19) |
| Formel (I-13-20) | Formel (I-13) | Formel (Cbz-20) |
| Formel (I-13-21) | Formel (I-13) | Formel (Cbz-21) |
| Formel (I-13-22) | Formel (I-13) | Formel (Cbz-22) |
| Formel (I-13-23) | Formel (I-13) | Formel (Cbz-23) |
| Formel (I-13-24) | Formel (I-13) | Formel (Cbz-24) |
| Formel (I-13-25) | Formel (I-13) | Formel (Cbz-25) |
| Formel (I-13-26) | Formel (I-13) | Formel (Cbz-26) |
| Formel (I-13-27) | Formel (I-13) | Formel (Cbz-27) |
| Formel (I-13-28) | Formel (I-13) | Formel (Cbz-28) |
| Formel (I-13-29) | Formel (I-13) | Formel (Cbz-29) |
| Formel (I-14-1) | Formel (I-14) | Formel (Cbz-1) |
| Formel (I-14-2) | Formel (I-14) | Formel (Cbz-2) |
| Formel (I-14-3) | Formel (I-14) | Formel (Cbz-3) |
| Formel (I-14-4) | Formel (I-14) | Formel (Cbz-4) |
| Formel (I-14-5) | Formel (I-14) | Formel (Cbz-5) |
| Formel (I-14-6) | Formel (I-14) | Formel (Cbz-6) |
| Formel (I-14-7) | Formel (I-14) | Formel (Cbz-7) |
| Formel (I-14-8) | Formel (I-14) | Formel (Cbz-8) |
| Formel (I-14-9) | Formel (I-14) | Formel (Cbz-9) |
| Formel (I-14-10) | Formel (I-14) | Formel (Cbz-10) |
| Formel (I-14-11) | Formel (I-14) | Formel (Cbz-11) |
| Formel (I-14-12) | Formel (I-14) | Formel (Cbz-12) |
| Formel (I-14-13) | Formel (I-14) | Formel (Cbz-13) |
| Formel (I-14-14) | Formel (I-14) | Formel (Cbz-14) |
| Formel (I-14-15) | Formel (I-14) | Formel (Cbz-15) |
| Formel (I-14-16) | Formel (I-14) | Formel (Cbz-16) |
| Formel (I-14-17) | Formel (I-14) | Formel (Cbz-17) |
| Formel (I-14-18) | Formel (I-14) | Formel (Cbz-18) |
| Formel (I-14-19) | Formel (I-14) | Formel (Cbz-19) |
| Formel (I-14-20) | Formel (I-14) | Formel (Cbz-20) |
| Formel (I-14-21) | Formel (I-14) | Formel (Cbz-21) |
| Formel (I-14-22) | Formel (I-14) | Formel (Cbz-22) |
| Formel (I-14-23) | Formel (I-14) | Formel (Cbz-23) |
| Formel (I-14-24) | Formel (I-14) | Formel (Cbz-24) |
| Formel (I-14-25) | Formel (I-14) | Formel (Cbz-25) |
| Formel (I-14-26) | Formel (I-14) | Formel (Cbz-26) |
| Formel (I-14-27) | Formel (I-14) | Formel (Cbz-27) |
| Formel (I-14-28) | Formel (I-14) | Formel (Cbz-28) |
| Formel (I-14-29) | Formel (I-14) | Formel (Cbz-29) |
| Formel (I-15-1) | Formel (I-15) | Formel (Cbz-1) |
| Formel (I-15-2) | Formel (I-15) | Formel (Cbz-2) |
| Formel (I-15-3) | Formel (I-15) | Formel (Cbz-3) |
| Formel (I-15-4) | Formel (I-15) | Formel (Cbz-4) |
| Formel (I-15-5) | Formel (I-15) | Formel (Cbz-5) |
| Formel (I-15-6) | Formel (I-15) | Formel (Cbz-6) |
| Formel (I-15-7) | Formel (I-15) | Formel (Cbz-7) |
| Formel (I-15-8) | Formel (I-15) | Formel (Cbz-8) |
| Formel (I-15-9) | Formel (I-15) | Formel (Cbz-9) |
| Formel (I-15-10) | Formel (I-15) | Formel (Cbz-10) |
| Formel (I-15-11) | Formel (I-15) | Formel (Cbz-11) |
| Formel (I-15-12) | Formel (I-15) | Formel (Cbz-12) |
| Formel (I-15-13) | Formel (I-15) | Formel (Cbz-13) |
| Formel (I-15-14) | Formel (I-15) | Formel (Cbz-14) |
| Formel (I-15-15) | Formel (I-15) | Formel (Cbz-15) |
| Formel (I-15-16) | Formel (I-15) | Formel (Cbz-16) |
| Formel (I-15-17) | Formel (I-15) | Formel (Cbz-17) |
| Formel (I-15-18) | Formel (I-15) | Formel (Cbz-18) |
| Formel (I-15-19) | Formel (I-15) | Formel (Cbz-19) |
| Formel (I-15-20) | Formel (I-15) | Formel (Cbz-20) |
| Formel (I-15-21) | Formel (I-15) | Formel (Cbz-21) |
| Formel (I-15-22) | Formel (I-15) | Formel (Cbz-22) |
| Formel (I-15-23) | Formel (I-15) | Formel (Cbz-23) |
| Formel (I-15-24) | Formel (I-15) | Formel (Cbz-24) |
| Formel (I-15-25) | Formel (I-15) | Formel (Cbz-25) |
| Formel (I-15-26) | Formel (I-15) | Formel (Cbz-26) |
| Formel (I-15-27) | Formel (I-15) | Formel (Cbz-27) |
| Formel (I-15-28) | Formel (I-15) | Formel (Cbz-28) |
| Formel (I-15-29) | Formel (I-15) | Formel (Cbz-29) |
| Formel (I-16-1) | Formel (I-16) | Formel (Cbz-1) |
| Formel (I-16-2) | Formel (I-16) | Formel (Cbz-2) |
| Formel (I-16-3) | Formel (I-16) | Formel (Cbz-3) |
| Formel (I-16-4) | Formel (I-16) | Formel (Cbz-4) |
| Formel (I-16-5) | Formel (I-16) | Formel (Cbz-5) |
| Formel (I-16-6) | Formel (I-16) | Formel (Cbz-6) |
| Formel (I-16-7) | Formel (I-16) | Formel (Cbz-7) |
| Formel (I-16-8) | Formel (I-16) | Formel (Cbz-8) |
| Formel (I-16-9) | Formel (I-16) | Formel (Cbz-9) |
| Formel (I-16-10) | Formel (I-16) | Formel (Cbz-10) |
| Formel (I-16-11) | Formel (I-16) | Formel (Cbz-11) |
| Formel (I-16-12) | Formel (I-16) | Formel (Cbz-12) |
| Formel (I-16-13) | Formel (I-16) | Formel (Cbz-13) |
| Formel (I-16-14) | Formel (I-16) | Formel (Cbz-14) |
| Formel (I-16-15) | Formel (I-16) | Formel (Cbz-15) |
| Formel (I-16-16) | Formel (I-16) | Formel (Cbz-16) |
| Formel (I-16-17) | Formel (I-16) | Formel (Cbz-17) |
| Formel (I-16-18) | Formel (I-16) | Formel (Cbz-18) |
| Formel (I-16-19) | Formel (I-16) | Formel (Cbz-19) |
| Formel (I-16-20) | Formel (I-16) | Formel (Cbz-20) |
| Formel (I-16-21) | Formel (I-16) | Formel (Cbz-21) |
| Formel (I-16-22) | Formel (I-16) | Formel (Cbz-22) |
| Formel (I-16-23) | Formel (I-16) | Formel (Cbz-23) |
| Formel (I-16-24) | Formel (I-16) | Formel (Cbz-24) |
| Formel (I-16-25) | Formel (I-16) | Formel (Cbz-25) |
| Formel (I-16-26) | Formel (I-16) | Formel (Cbz-26) |
| Formel (I-16-27) | Formel (I-16) | Formel (Cbz-27) |
| Formel (I-16-28) | Formel (I-16) | Formel (Cbz-28) |
| Formel (I-16-29) | Formel (I-16) | Formel (Cbz-29) |
| Formel (I-17-1) | Formel (I-17) | Formel (Cbz-1) |
| Formel (I-17-2) | Formel (I-17) | Formel (Cbz-2) |
| Formel (I-17-3) | Formel (I-17) | Formel (Cbz-3) |
| Formel (I-17-4) | Formel (I-17) | Formel (Cbz-4) |
| Formel (I-17-5) | Formel (I-17) | Formel (Cbz-5) |
| Formel (I-17-6) | Formel (I-17) | Formel (Cbz-6) |
| Formel (I-17-7) | Formel (I-17) | Formel (Cbz-7) |
| Formel (I-17-8) | Formel (I-17) | Formel (Cbz-8) |
| Formel (I-17-9) | Formel (I-17) | Formel (Cbz-9) |
| Formel (I-17-10) | Formel (I-17) | Formel (Cbz-10) |
| Formel (I-17-11) | Formel (I-17) | Formel (Cbz-11) |
| Formel (I-17-12) | Formel (I-17) | Formel (Cbz-12) |
| Formel (I-17-13) | Formel (I-17) | Formel (Cbz-13) |
| Formel (I-17-14) | Formel (I-17) | Formel (Cbz-14) |
| Formel (I-17-15) | Formel (I-17) | Formel (Cbz-15) |
| Formel (I-17-16) | Formel (I-17) | Formel (Cbz-16) |
| Formel (I-17-17) | Formel (I-17) | Formel (Cbz-17) |
| Formel (I-17-18) | Formel (I-17) | Formel (Cbz-18) |
| Formel (I-17-19) | Formel (I-17) | Formel (Cbz-19) |
| Formel (I-17-20) | Formel (I-17) | Formel (Cbz-20) |
| Formel (I-17-21) | Formel (I-17) | Formel (Cbz-21) |
| Formel (I-17-22) | Formel (I-17) | Formel (Cbz-22) |
| Formel (I-17-23) | Formel (I-17) | Formel (Cbz-23) |
| Formel (I-17-24) | Formel (I-17) | Formel (Cbz-24) |
| Formel (I-17-25) | Formel (I-17) | Formel (Cbz-25) |
| Formel (I-17-26) | Formel (I-17) | Formel (Cbz-26) |
| Formel (I-17-27) | Formel (I-17) | Formel (Cbz-27) |
| Formel (I-17-28) | Formel (I-17) | Formel (Cbz-28) |
| Formel (I-17-29) | Formel (I-17) | Formel (Cbz-29) |
| Formel (I-18-1) | Formel (I-18) | Formel (Cbz-1) |
| Formel (I-18-2) | Formel (I-18) | Formel (Cbz-2) |
| Formel (I-18-3) | Formel (I-18) | Formel (Cbz-3) |
| Formel (I-18-4) | Formel (I-18) | Formel (Cbz-4) |
| Formel (I-18-5) | Formel (I-18) | Formel (Cbz-5) |
| Formel (I-18-6) | Formel (I-18) | Formel (Cbz-6) |
| Formel (I-18-7) | Formel (I-18) | Formel (Cbz-7) |
| Formel (I-18-8) | Formel (I-18) | Formel (Cbz-8) |
| Formel (I-18-9) | Formel (I-18) | Formel (Cbz-9) |
| Formel (I-18-10) | Formel (I-18) | Formel (Cbz-10) |
| Formel (I-18-11) | Formel (I-18) | Formel (Cbz-11) |
| Formel (I-18-12) | Formel (I-18) | Formel (Cbz-12) |
| Formel (I-18-13) | Formel (I-18) | Formel (Cbz-13) |
| Formel (I-18-14) | Formel (I-18) | Formel (Cbz-14) |
| Formel (I-18-15) | Formel (I-18) | Formel (Cbz-15) |
| Formel (I-18-16) | Formel (I-18) | Formel (Cbz-16) |
| Formel (I-18-17) | Formel (I-18) | Formel (Cbz-17) |
| Formel (I-18-18) | Formel (I-18) | Formel (Cbz-18) |
| Formel (I-18-19) | Formel (I-18) | Formel (Cbz-19) |
| Formel (I-18-20) | Formel (I-18) | Formel (Cbz-20) |
| Formel (I-18-21) | Formel (I-18) | Formel (Cbz-21) |
| Formel (I-18-22) | Formel (I-18) | Formel (Cbz-22) |
| Formel (I-18-23) | Formel (I-18) | Formel (Cbz-23) |
| Formel (I-18-24) | Formel (I-18) | Formel (Cbz-24) |
| Formel (I-18-25) | Formel (I-18) | Formel (Cbz-25) |
| Formel (I-18-26) | Formel (I-18) | Formel (Cbz-26) |
| Formel (I-18-27) | Formel (I-18) | Formel (Cbz-27) |
| Formel (I-18-28) | Formel (I-18) | Formel (Cbz-28) |
| Formel (I-18-29) | Formel (I-18) | Formel (Cbz-29) |
| Formel (I-19-1) | Formel (I-19) | Formel (Cbz-1) |
| Formel (I-19-2) | Formel (I-19) | Formel (Cbz-2) |
| Formel (I-19-3) | Formel (I-19) | Formel (Cbz-3) |
| Formel (I-19-4) | Formel (I-19) | Formel (Cbz-4) |
| Formel (I-19-5) | Formel (I-19) | Formel (Cbz-5) |
| Formel (I-19-6) | Formel (I-19) | Formel (Cbz-6) |
| Formel (I-19-7) | Formel (I-19) | Formel (Cbz-7) |
| Formel (I-19-8) | Formel (I-19) | Formel (Cbz-8) |
| Formel (I-19-9) | Formel (I-19) | Formel (Cbz-9) |
| Formel (I-19-10) | Formel (I-19) | Formel (Cbz-10) |
| Formel (I-19-11) | Formel (I-19) | Formel (Cbz-11) |
| Formel (I-19-12) | Formel (I-19) | Formel (Cbz-12) |
| Formel (I-19-13) | Formel (I-19) | Formel (Cbz-13) |
| Formel (I-19-14) | Formel (I-19) | Formel (Cbz-14) |
| Formel (I-19-15) | Formel (I-19) | Formel (Cbz-15) |
| Formel (I-19-16) | Formel (I-19) | Formel (Cbz-16) |
| Formel (I-19-17) | Formel (I-19) | Formel (Cbz-17) |
| Formel (I-19-18) | Formel (I-19) | Formel (Cbz-18) |
| Formel (I-19-19) | Formel (I-19) | Formel (Cbz-19) |
| Formel (I-19-20) | Formel (I-19) | Formel (Cbz-20) |
| Formel (I-19-21) | Formel (I-19) | Formel (Cbz-21) |
| Formel (I-19-22) | Formel (I-19) | Formel (Cbz-22) |
| Formel (I-19-23) | Formel (I-19) | Formel (Cbz-23) |
| Formel (I-19-24) | Formel (I-19) | Formel (Cbz-24) |
| Formel (I-19-25) | Formel (I-19) | Formel (Cbz-25) |
| Formel (I-19-26) | Formel (I-19) | Formel (Cbz-26) |
| Formel (I-19-27) | Formel (I-19) | Formel (Cbz-27) |
| Formel (I-19-28) | Formel (I-19) | Formel (Cbz-28) |
| Formel (I-19-29) | Formel (I-19) | Formel (Cbz-29) |
| Formel (I-20-1) | Formel (I-20) | Formel (Cbz-1) |
| Formel (I-20-2) | Formel (I-20) | Formel (Cbz-2) |
| Formel (I-20-3) | Formel (I-20) | Formel (Cbz-3) |
| Formel (I-20-4) | Formel (I-20) | Formel (Cbz-4) |
| Formel (I-20-5) | Formel (I-20) | Formel (Cbz-5) |
| Formel (I-20-6) | Formel (I-20) | Formel (Cbz-6) |
| Formel (I-20-7) | Formel (I-20) | Formel (Cbz-7) |
| Formel (I-20-8) | Formel (I-20) | Formel (Cbz-8) |
| Formel (I-20-9) | Formel (I-20) | Formel (Cbz-9) |
| Formel (I-20-10) | Formel (I-20) | Formel (Cbz-10) |
| Formel (I-20-11) | Formel (I-20) | Formel (Cbz-11) |
| Formel (I-20-12) | Formel (I-20) | Formel (Cbz-12) |
| Formel (I-20-13) | Formel (I-20) | Formel (Cbz-13) |
| Formel (I-20-14) | Formel (I-20) | Formel (Cbz-14) |
| Formel (I-20-15) | Formel (I-20) | Formel (Cbz-15) |
| Formel (I-20-16) | Formel (I-20) | Formel (Cbz-16) |
| Formel (I-20-17) | Formel (I-20) | Formel (Cbz-17) |
| Formel (I-20-18) | Formel (I-20) | Formel (Cbz-18) |
| Formel (I-20-19) | Formel (I-20) | Formel (Cbz-19) |
| Formel (I-20-20) | Formel (I-20) | Formel (Cbz-20) |
| Formel (I-20-21) | Formel (I-20) | Formel (Cbz-21) |
| Formel (I-20-22) | Formel (I-20) | Formel (Cbz-22) |
| Formel (I-20-23) | Formel (I-20) | Formel (Cbz-23) |
| Formel (I-20-24) | Formel (I-20) | Formel (Cbz-24) |
| Formel (I-20-25) | Formel (I-20) | Formel (Cbz-25) |
| Formel (I-20-26) | Formel (I-20) | Formel (Cbz-26) |
| Formel (I-20-27) | Formel (I-20) | Formel (Cbz-27) |
| Formel (I-20-28) | Formel (I-20) | Formel (Cbz-28) |
| Formel (I-20-29) | Formel (I-20) | Formel (Cbz-29) |
| Formel (I-21-1) | Formel (I-21) | Formel (Cbz-1) |
| Formel (I-21-2) | Formel (I-21) | Formel (Cbz-2) |
| Formel (I-21-3) | Formel (I-21) | Formel (Cbz-3) |
| Formel (I-21-4) | Formel (I-21) | Formel (Cbz-4) |
| Formel (I-21-5) | Formel (I-21) | Formel (Cbz-5) |
| Formel (I-21-6) | Formel (I-21) | Formel (Cbz-6) |
| Formel (I-21-7) | Formel (I-21) | Formel (Cbz-7) |
| Formel (I-21-8) | Formel (I-21) | Formel (Cbz-8) |
| Formel (I-21-9) | Formel (I-21) | Formel (Cbz-9) |
| Formel (I-21-10) | Formel (I-21) | Formel (Cbz-10) |
| Formel (I-21-11) | Formel (I-21) | Formel (Cbz-11) |
| Formel (I-21-12) | Formel (I-21) | Formel (Cbz-12) |
| Formel (I-21-13) | Formel (I-21) | Formel (Cbz-13) |
| Formel (I-21-14) | Formel (I-21) | Formel (Cbz-14) |
| Formel (I-21-15) | Formel (I-21) | Formel (Cbz-15) |
| Formel (I-21-16) | Formel (I-21) | Formel (Cbz-16) |
| Formel (I-21-17) | Formel (I-21) | Formel (Cbz-17) |
| Formel (I-21-18) | Formel (I-21) | Formel (Cbz-18) |
| Formel (I-21-19) | Formel (I-21) | Formel (Cbz-19) |
| Formel (I-21-20) | Formel (I-21) | Formel (Cbz-20) |
| Formel (I-21-21) | Formel (I-21) | Formel (Cbz-21) |
| Formel (I-21-22) | Formel (I-21) | Formel (Cbz-22) |
| Formel (I-21-23) | Formel (I-21) | Formel (Cbz-23) |
| Formel (I-21-24) | Formel (I-21) | Formel (Cbz-24) |
| Formel (I-21-25) | Formel (I-21) | Formel (Cbz-25) |
| Formel (I-21-26) | Formel (I-21) | Formel (Cbz-26) |
| Formel (I-21-27) | Formel (I-21) | Formel (Cbz-27) |
| Formel (I-21-28) | Formel (I-21) | Formel (Cbz-28) |
| Formel (I-21-29) | Formel (I-21) | Formel (Cbz-29) |
| Formel (I-22-1) | Formel (I-22) | Formel (Cbz-1) |
| Formel (I-22-2) | Formel (I-22) | Formel (Cbz-2) |
| Formel (I-22-3) | Formel (I-22) | Formel (Cbz-3) |
| Formel (I-22-4) | Formel (I-22) | Formel (Cbz-4) |
| Formel (I-22-5) | Formel (I-22) | Formel (Cbz-5) |
| Formel (I-22-6) | Formel (I-22) | Formel (Cbz-6) |
| Formel (I-22-7) | Formel (I-22) | Formel (Cbz-7) |
| Formel (I-22-8) | Formel (I-22) | Formel (Cbz-8) |
| Formel (I-22-9) | Formel (I-22) | Formel (Cbz-9) |
| Formel (I-22-10) | Formel (I-22) | Formel (Cbz-10) |
| Formel (I-22-11) | Formel (I-22) | Formel (Cbz-11) |
| Formel (I-22-12) | Formel (I-22) | Formel (Cbz-12) |
| Formel (I-22-13) | Formel (I-22) | Formel (Cbz-13) |
| Formel (I-22-14) | Formel (I-22) | Formel (Cbz-14) |
| Formel (I-22-15) | Formel (I-22) | Formel (Cbz-15) |
| Formel (I-22-16) | Formel (I-22) | Formel (Cbz-16) |
| Formel (I-22-17) | Formel (I-22) | Formel (Cbz-17) |
| Formel (I-22-18) | Formel (I-22) | Formel (Cbz-18) |
| Formel (I-22-19) | Formel (I-22) | Formel (Cbz-19) |
| Formel (I-22-20) | Formel (I-22) | Formel (Cbz-20) |
| Formel (I-22-21) | Formel (I-22) | Formel (Cbz-21) |
| Formel (I-22-22) | Formel (I-22) | Formel (Cbz-22) |
| Formel (I-22-23) | Formel (I-22) | Formel (Cbz-23) |
| Formel (I-22-24) | Formel (I-22) | Formel (Cbz-24) |
| Formel (I-22-25) | Formel (I-22) | Formel (Cbz-25) |
| Formel (I-22-26) | Formel (I-22) | Formel (Cbz-26) |
| Formel (I-22-27) | Formel (I-22) | Formel (Cbz-27) |
| Formel (I-22-28) | Formel (I-22) | Formel (Cbz-28) |
| Formel (I-22-29) | Formel (I-22) | Formel (Cbz-29) |
| Formel (I-23-1) | Formel (I-23) | Formel (Cbz-1) |
| Formel (I-23-2) | Formel (I-23) | Formel (Cbz-2) |
| Formel (I-23-3) | Formel (I-23) | Formel (Cbz-3) |
| Formel (I-23-4) | Formel (I-23) | Formel (Cbz-4) |
| Formel (I-23-5) | Formel (I-23) | Formel (Cbz-5) |
| Formel (I-23-6) | Formel (I-23) | Formel (Cbz-6) |
| Formel (I-23-7) | Formel (I-23) | Formel (Cbz-7) |
| Formel (I-23-8) | Formel (I-23) | Formel (Cbz-8) |
| Formel (I-23-9) | Formel (I-23) | Formel (Cbz-9) |
| Formel (I-23-10) | Formel (I-23) | Formel (Cbz-10) |
| Formel (I-23-11) | Formel (I-23) | Formel (Cbz-11) |
| Formel (I-23-12) | Formel (I-23) | Formel (Cbz-12) |
| Formel (I-23-13) | Formel (I-23) | Formel (Cbz-13) |
| Formel (I-23-14) | Formel (I-23) | Formel (Cbz-14) |
| Formel (I-23-15) | Formel (I-23) | Formel (Cbz-15) |
| Formel (I-23-16) | Formel (I-23) | Formel (Cbz-16) |
| Formel (I-23-17) | Formel (I-23) | Formel (Cbz-17) |
| Formel (I-23-18) | Formel (I-23) | Formel (Cbz-18) |
| Formel (I-23-19) | Formel (I-23) | Formel (Cbz-19) |
| Formel (I-23-20) | Formel (I-23) | Formel (Cbz-20) |
| Formel (I-23-21) | Formel (I-23) | Formel (Cbz-21) |
| Formel (I-23-22) | Formel (I-23) | Formel (Cbz-22) |
| Formel (I-23-23) | Formel (I-23) | Formel (Cbz-23) |
| Formel (I-23-24) | Formel (I-23) | Formel (Cbz-24) |
| Formel (I-23-25) | Formel (I-23) | Formel (Cbz-25) |
| Formel (I-23-26) | Formel (I-23) | Formel (Cbz-26) |
| Formel (I-23-27) | Formel (I-23) | Formel (Cbz-27) |
| Formel (I-23-28) | Formel (I-23) | Formel (Cbz-28) |
| Formel (I-23-29) | Formel (I-23) | Formel (Cbz-29) |
| Formel (I-24-1) | Formel (I-24) | Formel (Cbz-1) |
| Formel (I-24-2) | Formel (I-24) | Formel (Cbz-2) |
| Formel (I-24-3) | Formel (I-24) | Formel (Cbz-3) |
| Formel (I-24-4) | Formel (I-24) | Formel (Cbz-4) |
| Formel (I-24-5) | Formel (I-24) | Formel (Cbz-5) |
| Formel (I-24-6) | Formel (I-24) | Formel (Cbz-6) |
| Formel (I-24-7) | Formel (I-24) | Formel (Cbz-7) |
| Formel (I-24-8) | Formel (I-24) | Formel (Cbz-8) |
| Formel (I-24-9) | Formel (I-24) | Formel (Cbz-9) |
| Formel (I-24-10) | Formel (I-24) | Formel (Cbz-10) |
| Formel (I-24-11) | Formel (I-24) | Formel (Cbz-11) |
| Formel (I-24-12) | Formel (I-24) | Formel (Cbz-12) |
| Formel (I-24-13) | Formel (I-24) | Formel (Cbz-13) |
| Formel (I-24-14) | Formel (I-24) | Formel (Cbz-14) |
| Formel (I-24-15) | Formel (I-24) | Formel (Cbz-15) |
| Formel (I-24-16) | Formel (I-24) | Formel (Cbz-16) |
| Formel (I-24-17) | Formel (I-24) | Formel (Cbz-17) |
| Formel (I-24-18) | Formel (I-24) | Formel (Cbz-18) |
| Formel (I-24-19) | Formel (I-24) | Formel (Cbz-19) |
| Formel (I-24-20) | Formel (I-24) | Formel (Cbz-20) |
| Formel (I-24-21) | Formel (I-24) | Formel (Cbz-21) |
| Formel (I-24-22) | Formel (I-24) | Formel (Cbz-22) |
| Formel (I-24-23) | Formel (I-24) | Formel (Cbz-23) |
| Formel (I-24-24) | Formel (I-24) | Formel (Cbz-24) |
| Formel (I-24-25) | Formel (I-24) | Formel (Cbz-25) |
| Formel (I-24-26) | Formel (I-24) | Formel (Cbz-26) |
| Formel (I-24-27) | Formel (I-24) | Formel (Cbz-27) |
| Formel (I-24-28) | Formel (I-24) | Formel (Cbz-28) |
| Formel (I-24-29) | Formel (I-24) | Formel (Cbz-29) |

Für die oben genannten Formeln ist es bevorzugt, dass die Verbindungsteile -Cbz-Ar² und -Ar¹-Cbz-Ar² jeweils in der Position para zum Stickstoffatom an den aromatischen Sechsring gebunden sind, wie am folgenden Beispiel für #Formel (I-1) gezeigt ist:

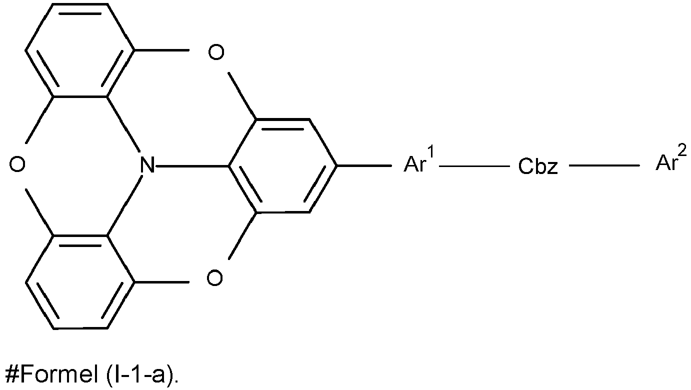

Für die oben genannten Formeln ist es bevorzugt, dass die Variablen den oben genannten bevorzugten Ausführungsformen entsprechen. Insbesondere ist es bevorzugt, dass dort die Gruppen Ar¹ und Ar² den oben genannten bevorzugten Ausführungsformen entsprechen.

Bevorzugte explizite Verbindungen der Formel (I) sind im Folgenden gezeigt:

| | | |
|---|---|---|
| 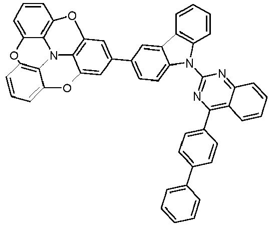 | 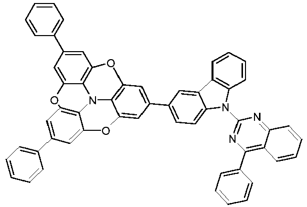 | 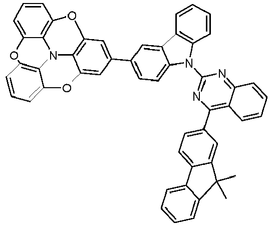 |
| #1 | #2 | #3 |
| 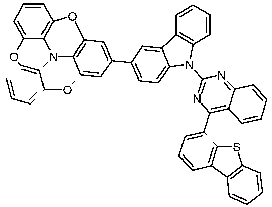 | 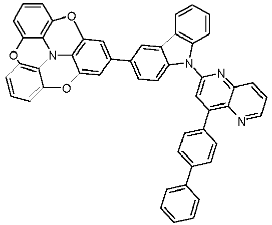 | 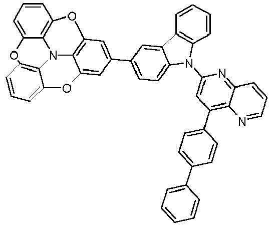 |
| #4 | #5 | #6 |
| 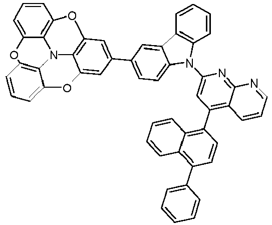 | 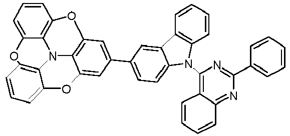 | 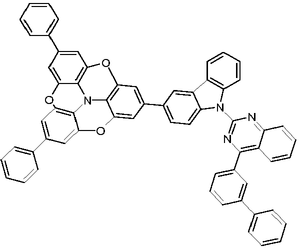 |
| #7 | #8 | #9 |
| 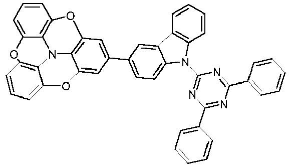 | 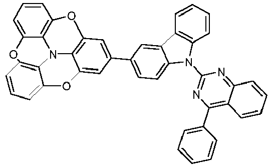 | 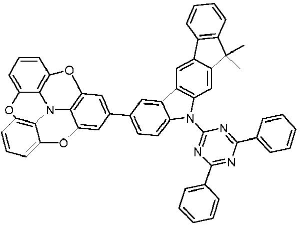 |
| #10 | #11 | #12 |
| 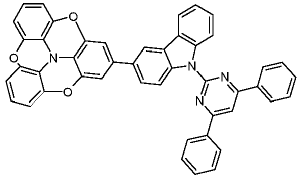 | 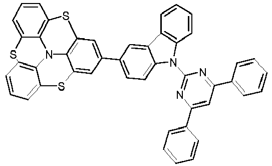 | 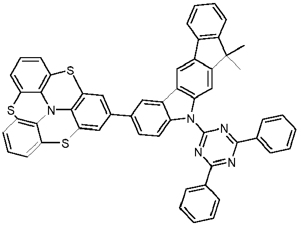 |
| #13 | #14 | #15 |
| 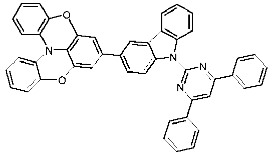 | 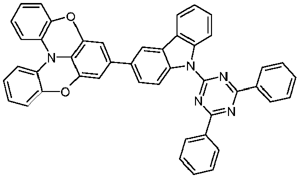 | 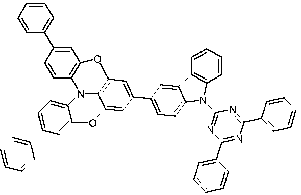 |
| 16 | 17 | 18 |
| 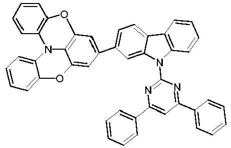 | 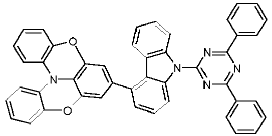 | 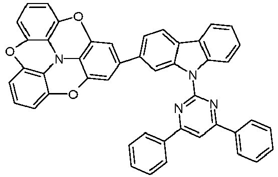 |
| 19 | 20 | #21 |
| 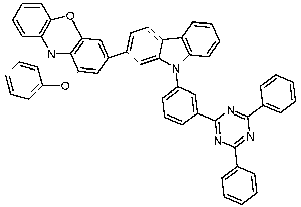 | 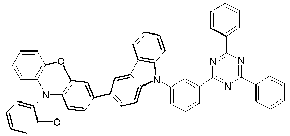 | 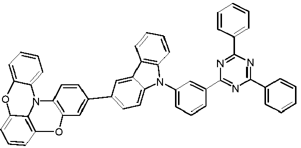 |
| 22 | 23 | 24 |
| 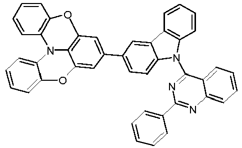 | 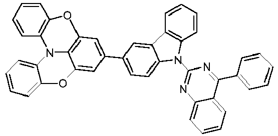 | 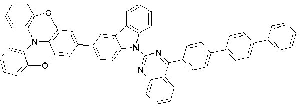 |
| 25 | 26 | 27 |
| 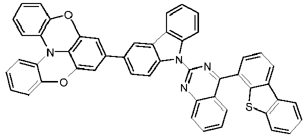 | 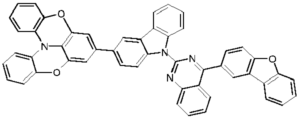 | 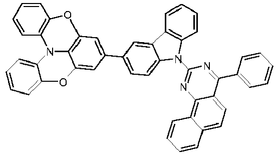 |
| 28 | 29 | 30 |
| 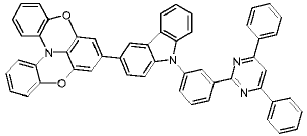 | 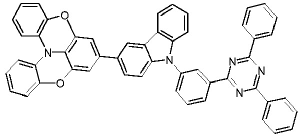 | 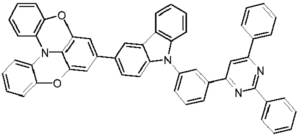 |
| 31 | 32 | 33 |
| 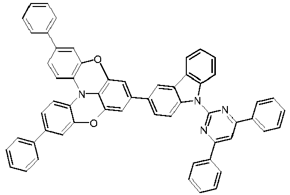 | 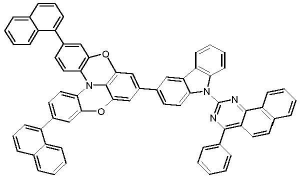 | 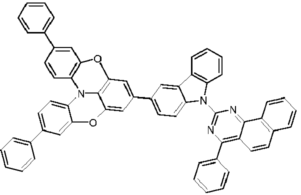 |
| 34 | 35 | 36 |
| 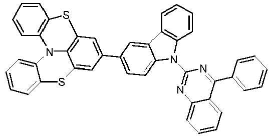 | 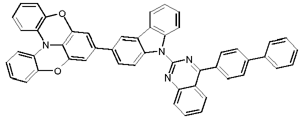 | 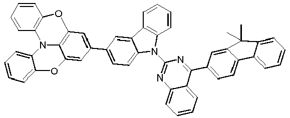 |
| 37 | 38 | 39 |
| 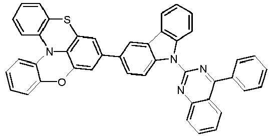 | 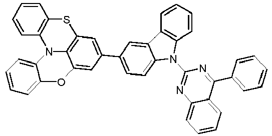 | 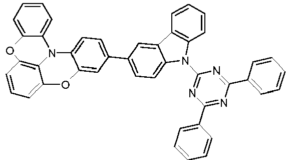 |
| 40 | 41 | 42 |
| 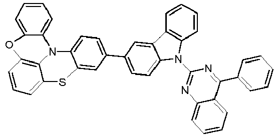 | 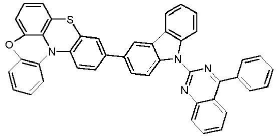 | 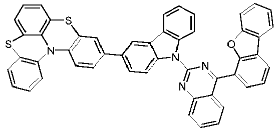 |
| 43 | 44 | 45 |
| 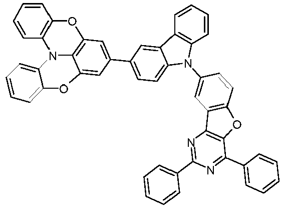 | 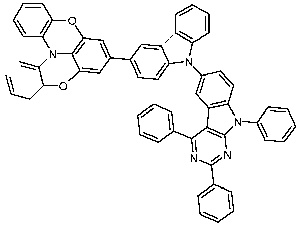 | |
| 46 | 47 | |
| 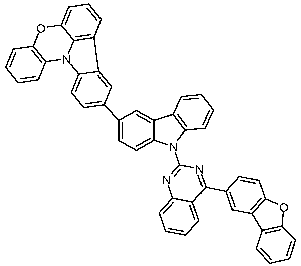 | 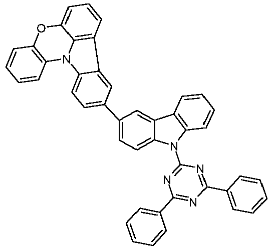 | 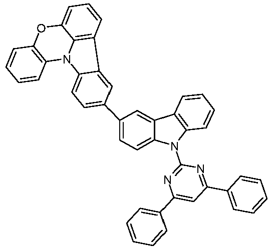 |
| 48 | 49 | 50 |
| 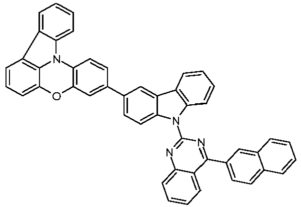 | 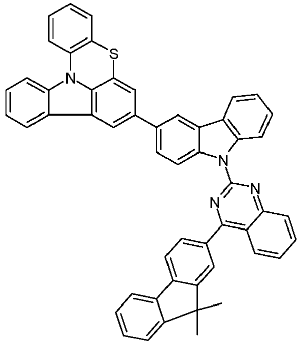 | 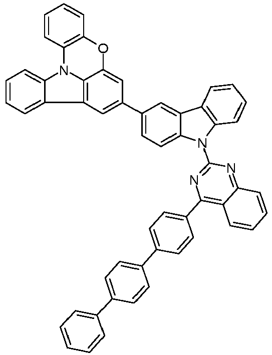 |
| 51 | 52 | 53 |
| 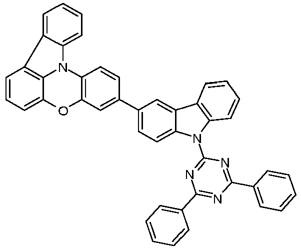 | 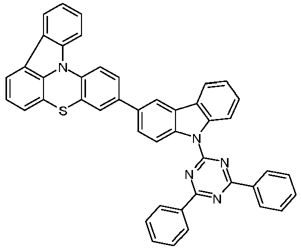 | 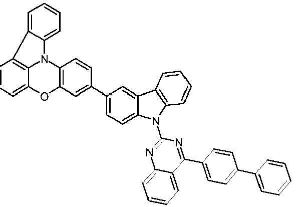 |
| 54 | 55 | 56 |
| 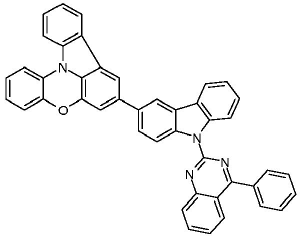 | 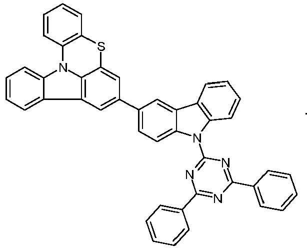 | 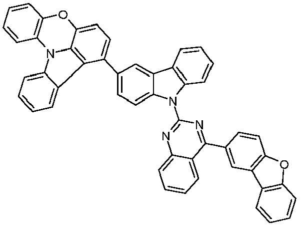 |
| 57 | 58 | 59 |
| 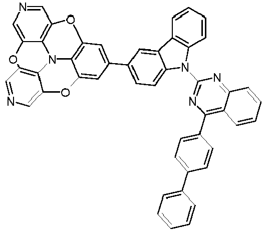 | 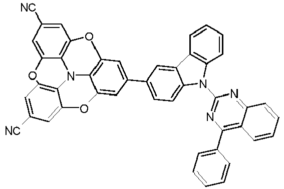 | 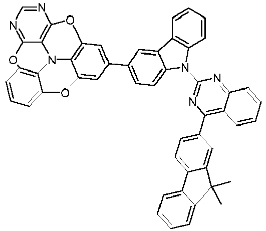 |
| #60 | #61 | #62 |
| 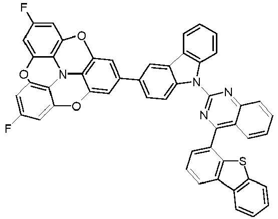 | 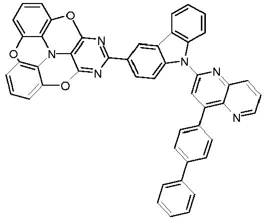 | 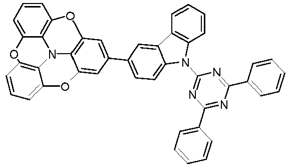 |
| #63 | #64 | #65 |
| 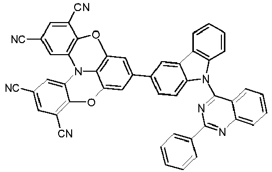 | 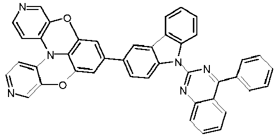 | 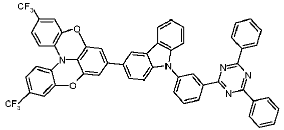 |
| 66 | 67 | 68 |
| 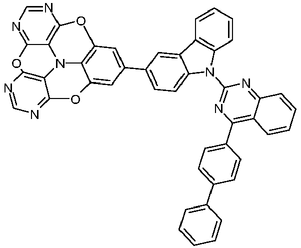 | 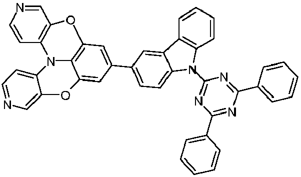 | 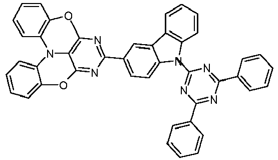 |
| #69 | 70 | 71 |
| 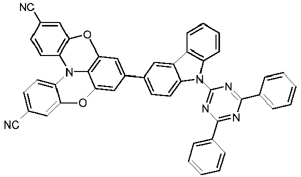 | 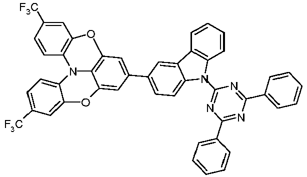 | 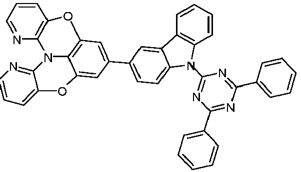 1 |
| 72 | 73 | 74 |
| 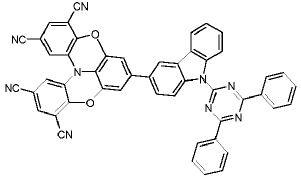 | 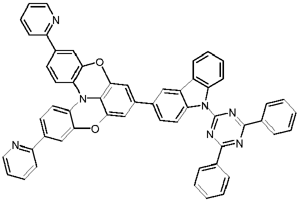 | 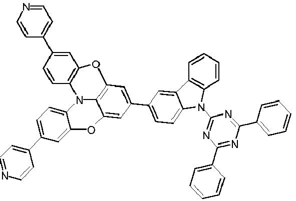 |
| 75 | 76 | 77 |

Die Verbindungen der Formel (I) können gemäß üblichen Verfahren der organischen Synthesechemie, die dem Fachmann bekannt sind, hergestellt werden. Bei der Herstellung der Verbindungen werden insbesondere übergangsmetallkatalysierte Kupplungsreaktionen, wie Halogenierungsreaktionen und Suzuki-Kupplungsreaktionen eingesetzt.

Bei der Herstellung der Verbindungen wird bevorzugt zunächst die verbrückte Triphenylamin-Gruppe hergestellt, die eine reaktive Gruppe trägt (Schemata 1-5). Alternativ können viele dieser Intermediate auch kommerziell erhalten werden.

Im folgenden Schema 1 ist beispielhaft gezeigt, wie eine mit drei Sauerstoff-Brücken verbrückte Triphenylamin-Gruppe, die ein Brom-Atom in para-Position zur zentralen Amingruppe trägt, hergestellt werden kann. Verbindungen mit derartigen dreifach verbrückten Triphenylamin-Gruppen als Grundstrukturen der Formel (I) sind nicht von den erteilten Ansprüchen umfasst.

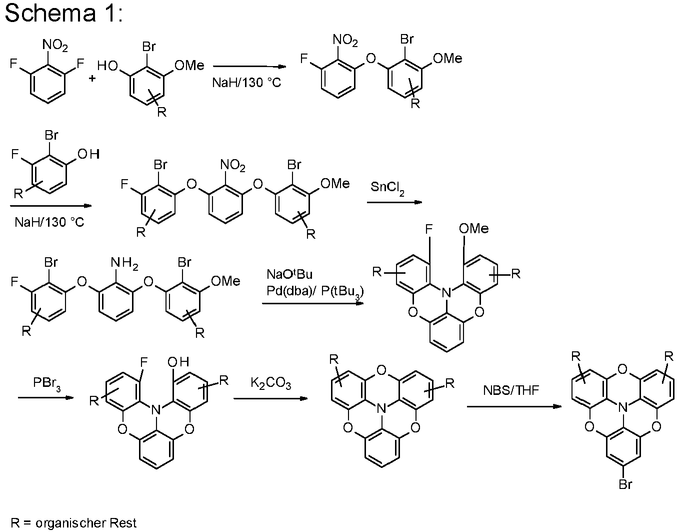

Im folgenden Schema 2 ist beispielhaft gezeigt, wie mit zwei Sauerstoff-Brücken (oben) bzw. mit zwei Schwefel-Brücken (unten) verbrückte Triphenylamin-Gruppen hergestellt werden können. Diese tragen jeweils ein Brom-Atom in para-Position zur zentralen Amingruppe.

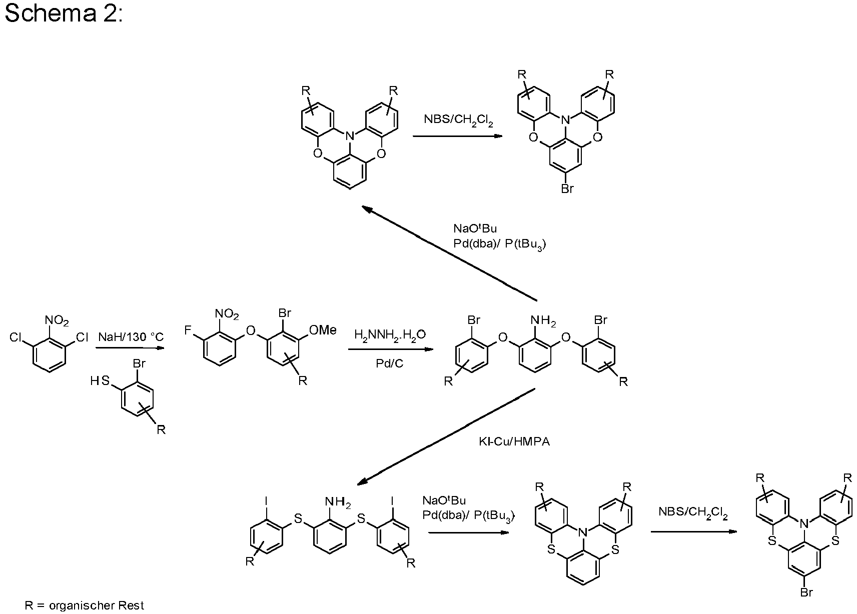

In Schema 3 sind zwei Wege zu den mit zwei Sauerstoff-Brücken verbrückten Triphenylamin-Gruppen gezeigt, die alternativ zu dem in Schema 2 oben gezeigten Weg verwendet werden können.

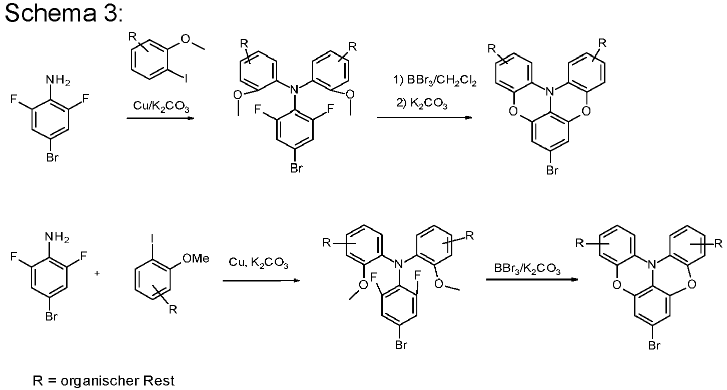

In Schema 4 ist ein Weg gezeigt, auf dem eine mit zwei Sauerstoff-Brücken verbrückte Triphenylamin-Gruppe, die asymmetrisch substituiert ist, und die ein Brom-Atom in para-Position zur zentralen Amingruppe trägt, hergestellt werden kann.

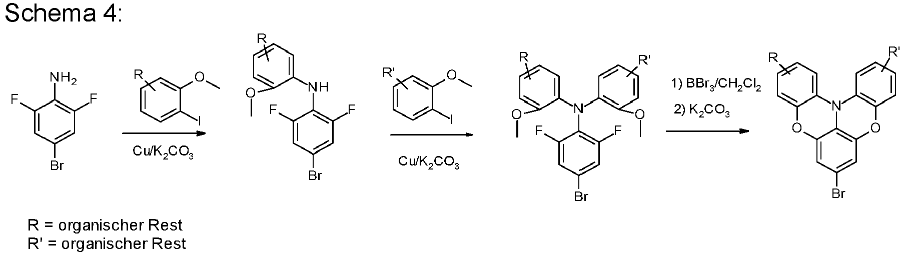

Schema 5 zeigt eine Synthese für mit zwei Sauerstoff-Brücken verbrückte Triphenylamin-Gruppen, die ein Brom-Atom an einer anderen Phenylgruppe tragen, als es in den Beispielen von Schema 2 bis 4 der Fall ist.

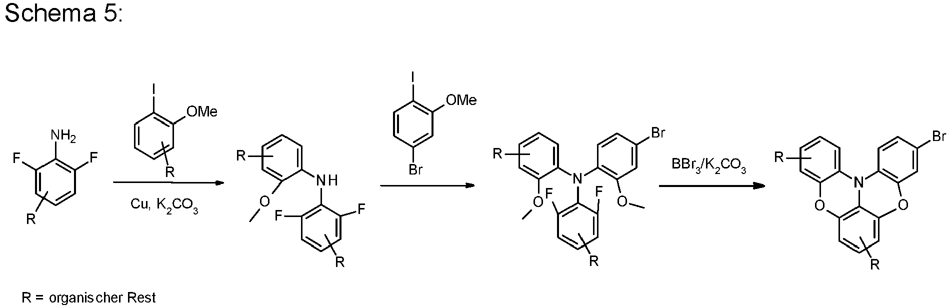

Im Anschluss an die Herstellung der verbrückten Triarylamin-Gruppe, die eine reaktive Gruppe trägt, bevorzugt ein Halogenatom, besonders bevorzugt Brom, wird in einer Suzuki-Kupplung ein Carbazol-Derivat an die verbrückte Triarylamingruppe angebunden (Schema 6).

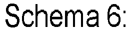

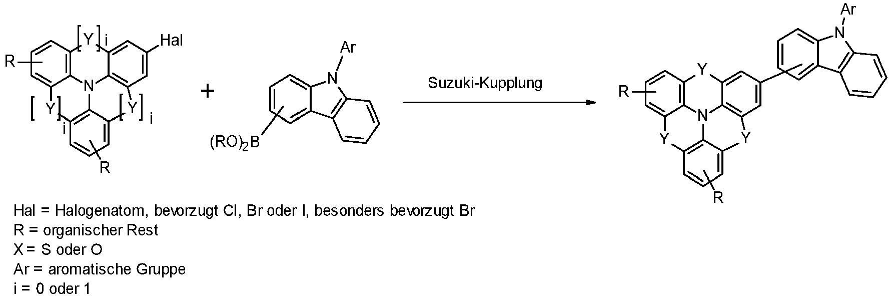

Gemäß einer alternativen Ausführungsform zu Schema 6 (Schema 7) wird an die verbrückte Triarylamin-Gruppe, die eine reaktive Gruppe trägt, bevorzugt ein Halogenatom, besonders bevorzugt Brom, in einer Suzuki Reaktion eine aromatische Gruppe angebunden, die eine reaktive Gruppe trägt, bevorzugt ein Halogenatom. In einem weiteren Schritt wird dann in einer weiteren Suzuki-Reaktion ein Carbazol-Derivat angebunden.

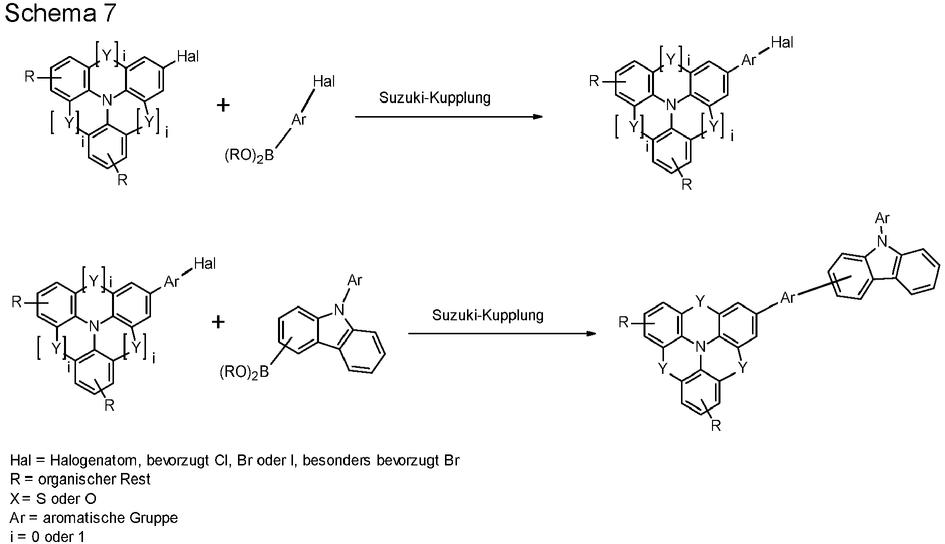

Die erhaltenen Verbindungen können gegebenfalls in folgenden Reaktionen weiter modifiziert und/oder substituiert werden.

Gegenstand der vorliegenden Erfindung ist somit ein Verfahren zur Herstellung einer Verbindung der Formel (I), wie oben definiert, dadurch gekennzeichnet, dass in einem ersten Schritt eine mit einer reaktiven Gruppe an einer der Phenylgruppen substituierte Triphenylaminverbindung hergestellt wird, wobei die Brücken zwischen den Phenylgruppen gewählt sind aus Einfachbindung, O und S, und wobei mindestens 2 Brücken vorliegen, und dass in einem weiteren Schritt über eine Übergangsmetallkatalysierte Kupplungsreaktion eine Carbazolgruppe in die Verbindung eingeführt wird.

Die reaktive Gruppe ist dabei bevorzugt gewählt aus Halogen, besonders bevorzugt Brom. Die Umsetzung des weiteren Schritts ist dabei bevorzugt eine Suzuki-Kupplungsreaktion. Die Carbazolgruppe weist dabei bevorzugt eine reaktive Gruppe auf, besonders bevorzugt eine Boronsäure oder ein Derivat der Boronsäure, beispielsweise einen Boronsäureester.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I), wobei die Bindung(en) zum Polymer, Oligomer oder Dendrimer an beliebigen, in Formel (I) mit R¹, R², R³, oder R⁴ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine Elektronentransportschicht, eine Lochblockierschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung enthaltend die Verbindung der Formel (I) ist bevorzugt die folgende: Anode-Lochinjektionsschicht-Lochtransportschicht-wahlweise weitere Lochtransportschicht(en)-wahlweise Elektronenblockierschichtemittierende Schicht-wahlweise Lochblockierschicht-Elektronentransportschicht-Elektroneninjektionsschicht-Kathode. Es können zusätzlich weitere Schichten in der OLED vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen (für den prinzipiellen Aufbau siehe z. B. WO 2005/011013). Die erfindungsgemäßen Verbindungen sind dabei bevorzugt in der emittierenden Schicht oder der Lochtransportschicht vorhanden.

Es ist erfindungsgemäß bevorzugt, wenn die Verbindung gemäß Formel (I) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Elektronentransportschicht oder in einer emittierenden Schicht, enthalten sein. Besonders bevorzugt ist sie in einer emittierenden Schicht in Kombination mit einer phosphoreszierenden emittierenden Verbindung enthalten.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen. Weitere Beispiele sind in einer folgenden Tabelle aufgeführt.

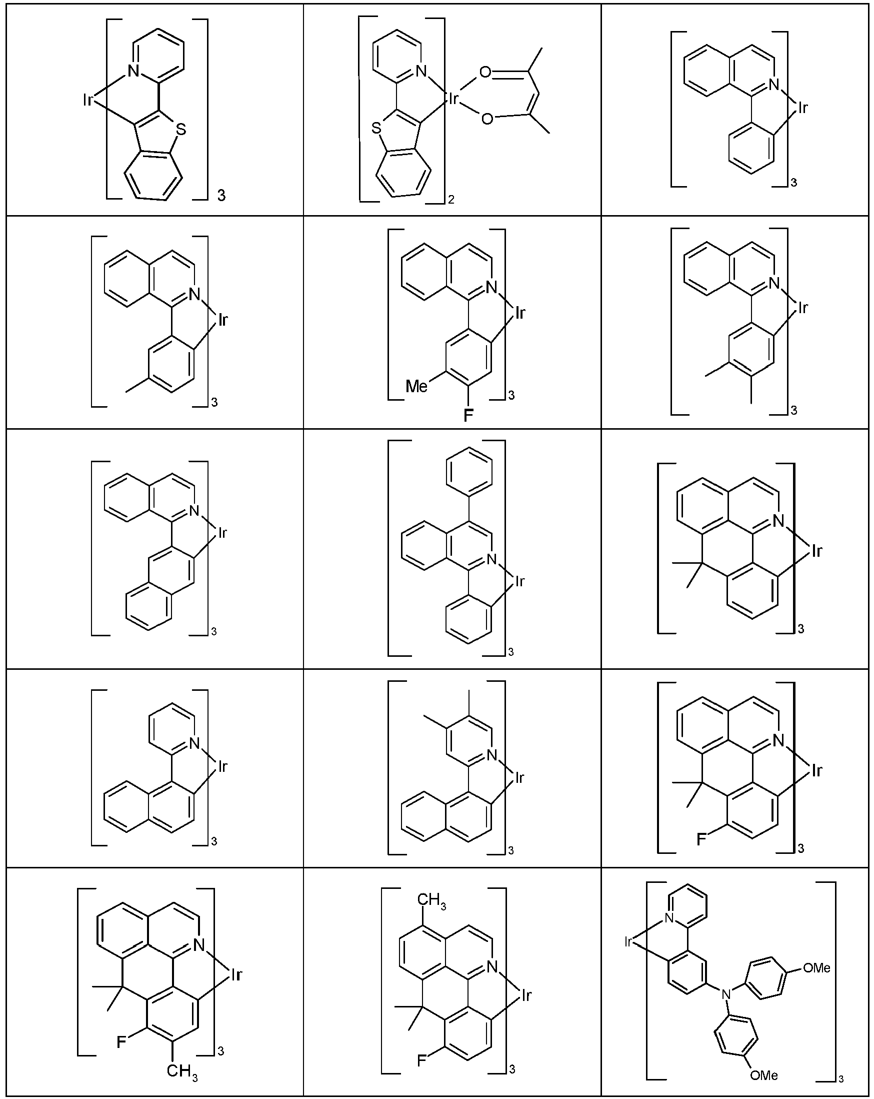

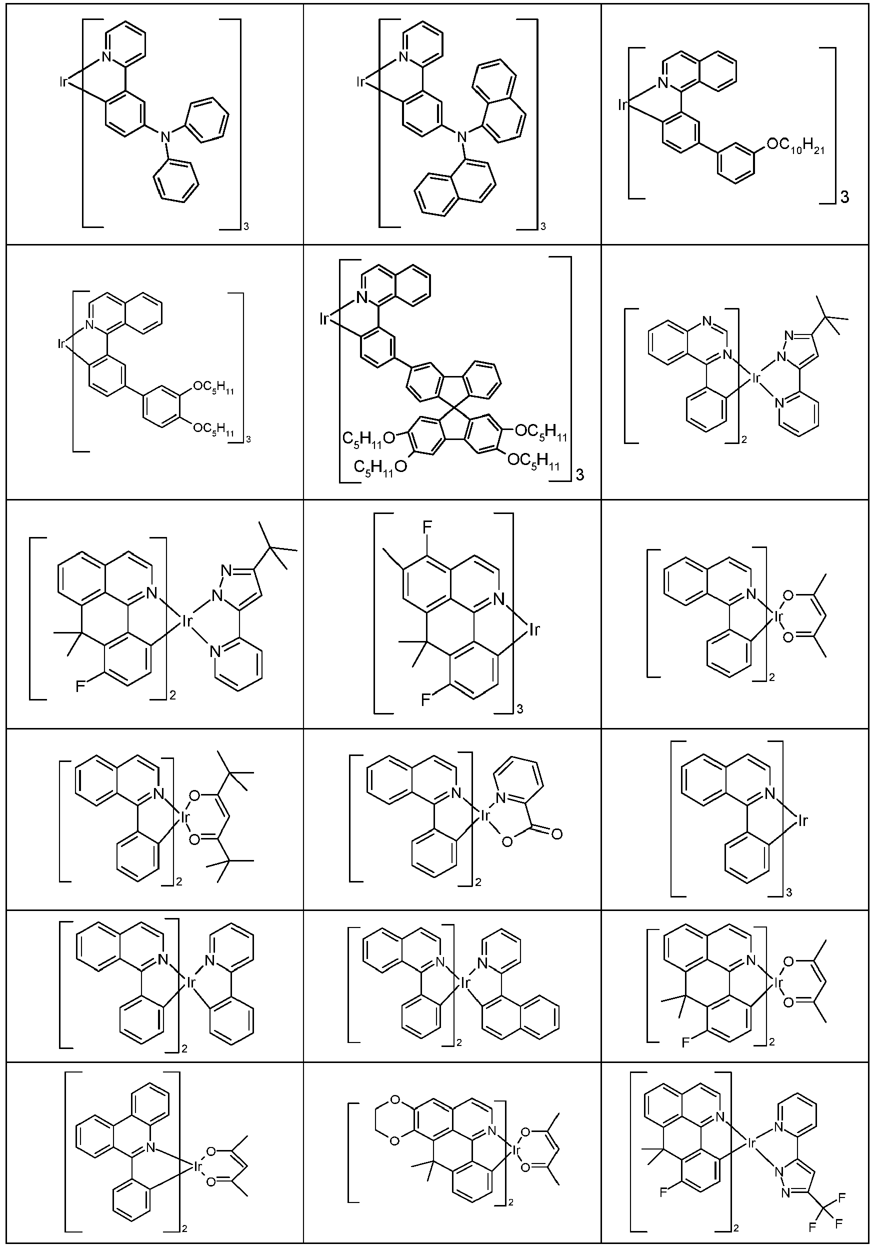

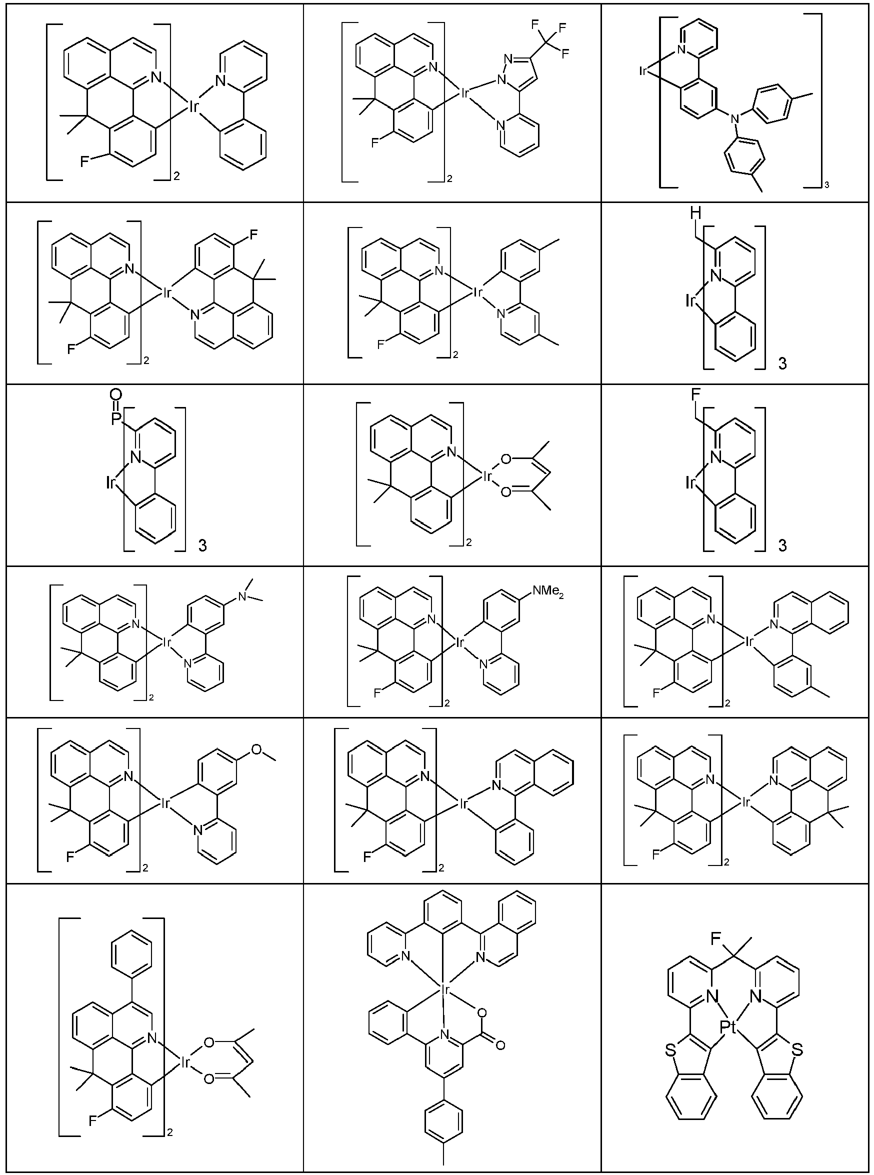

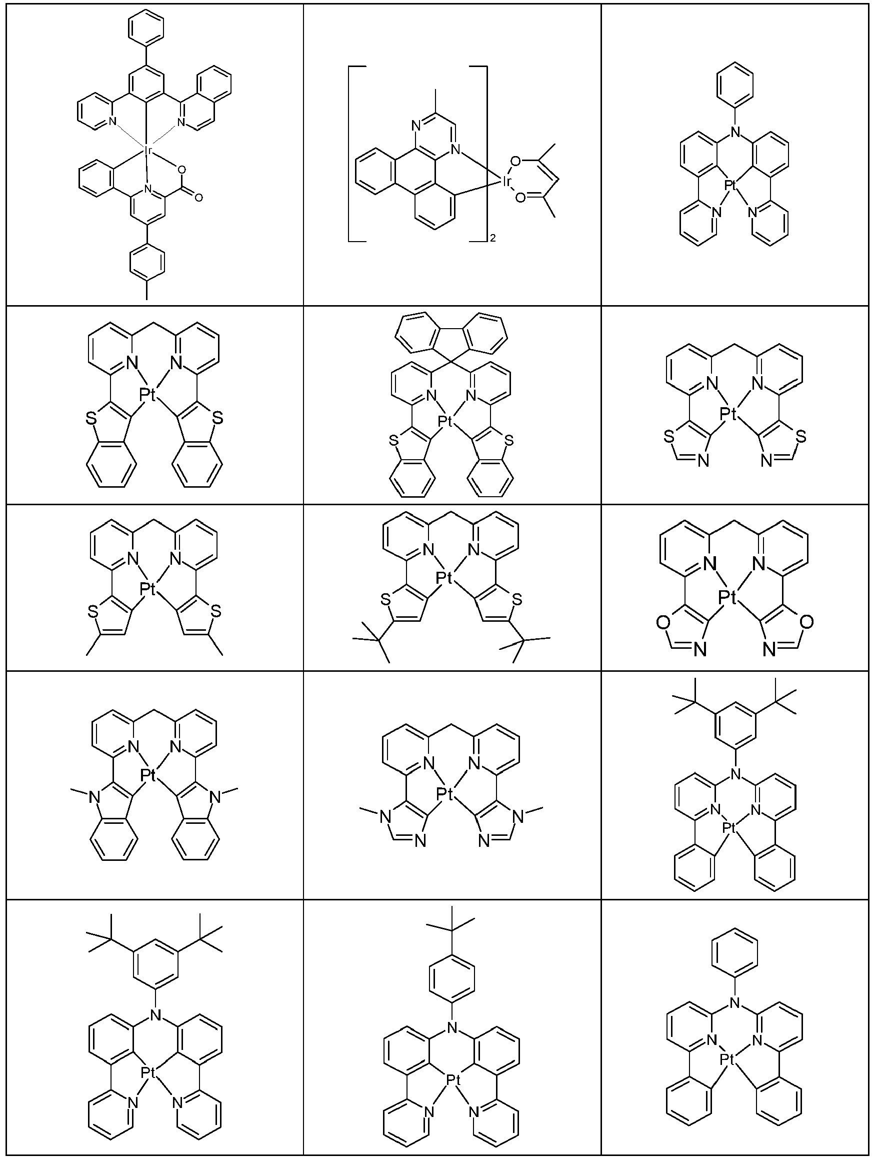

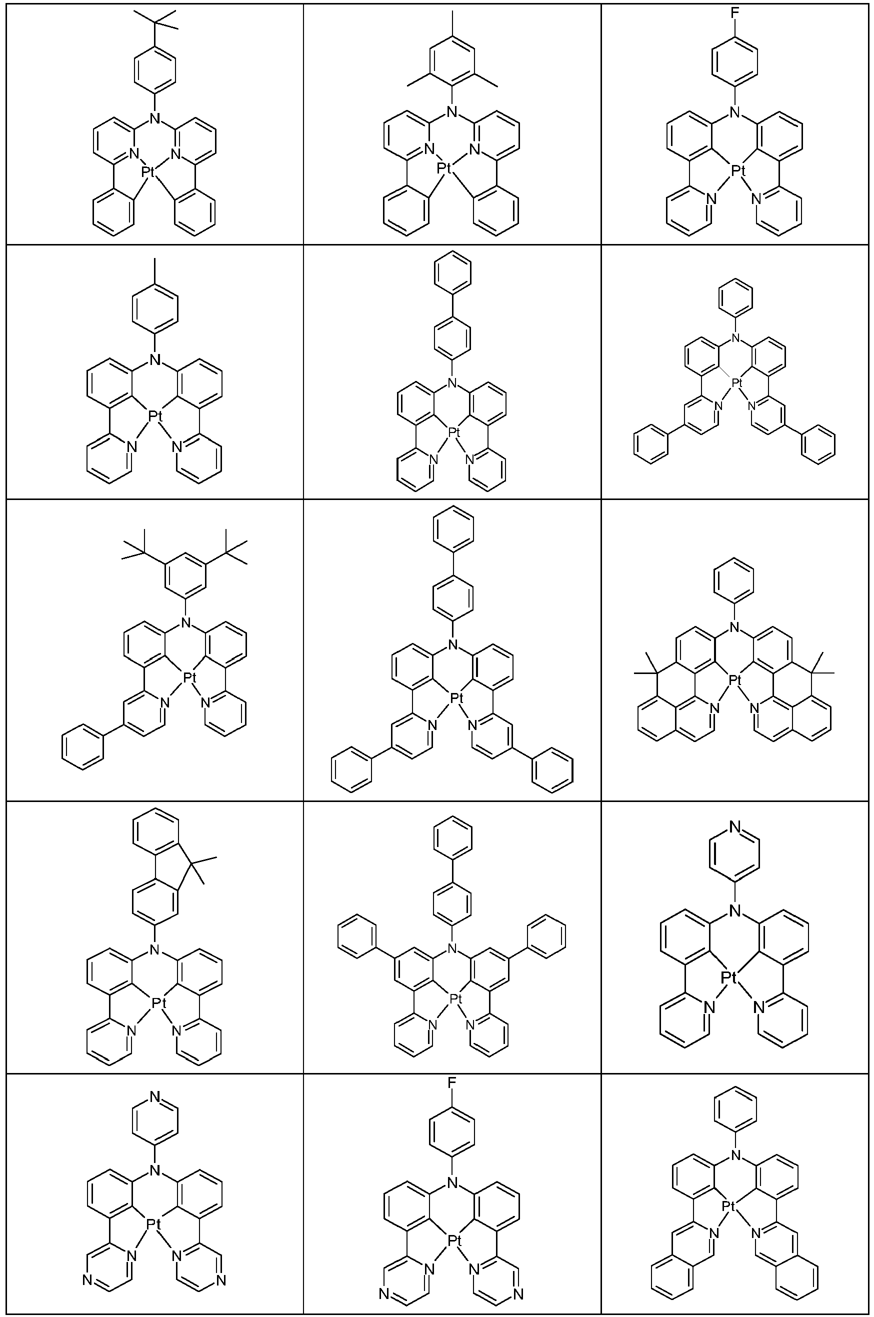

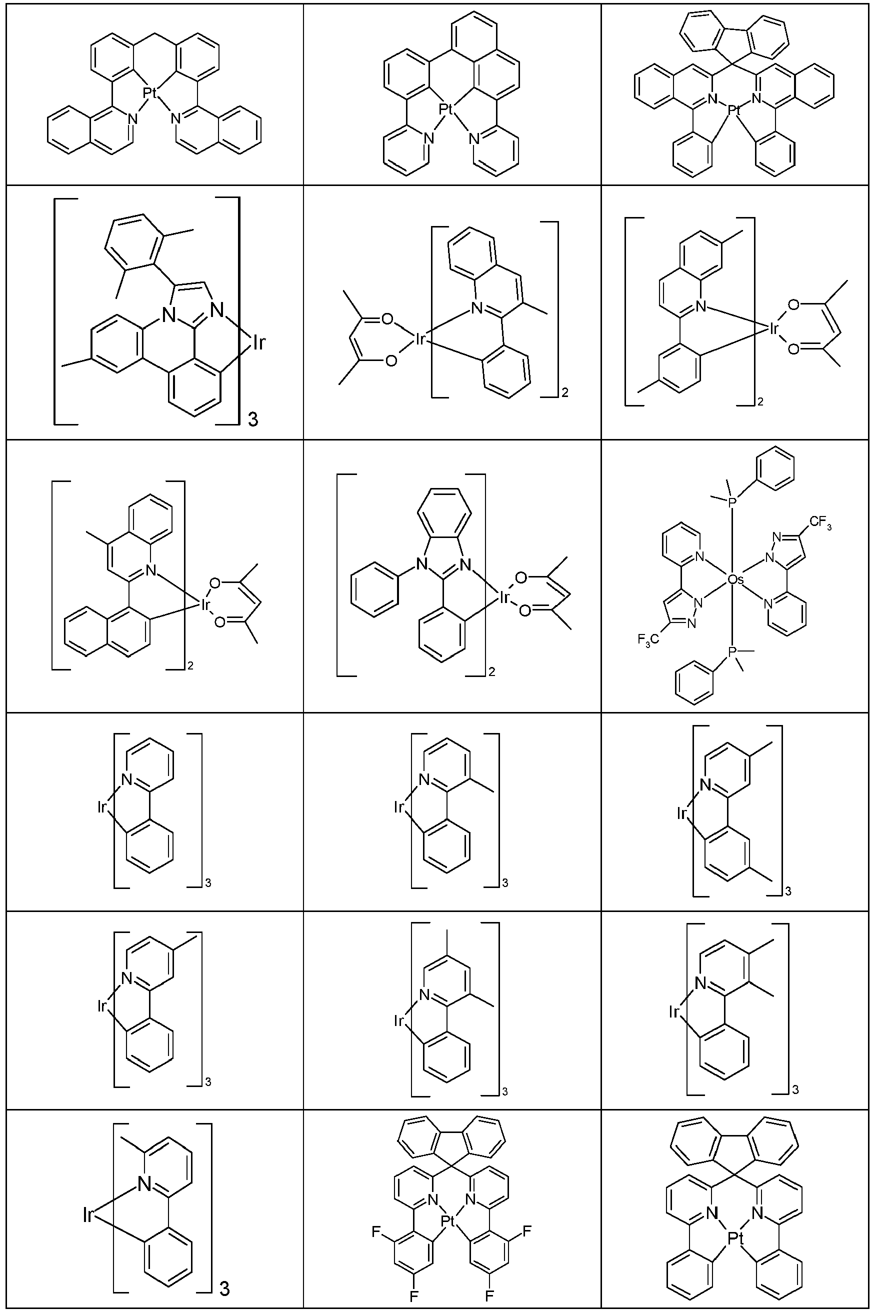

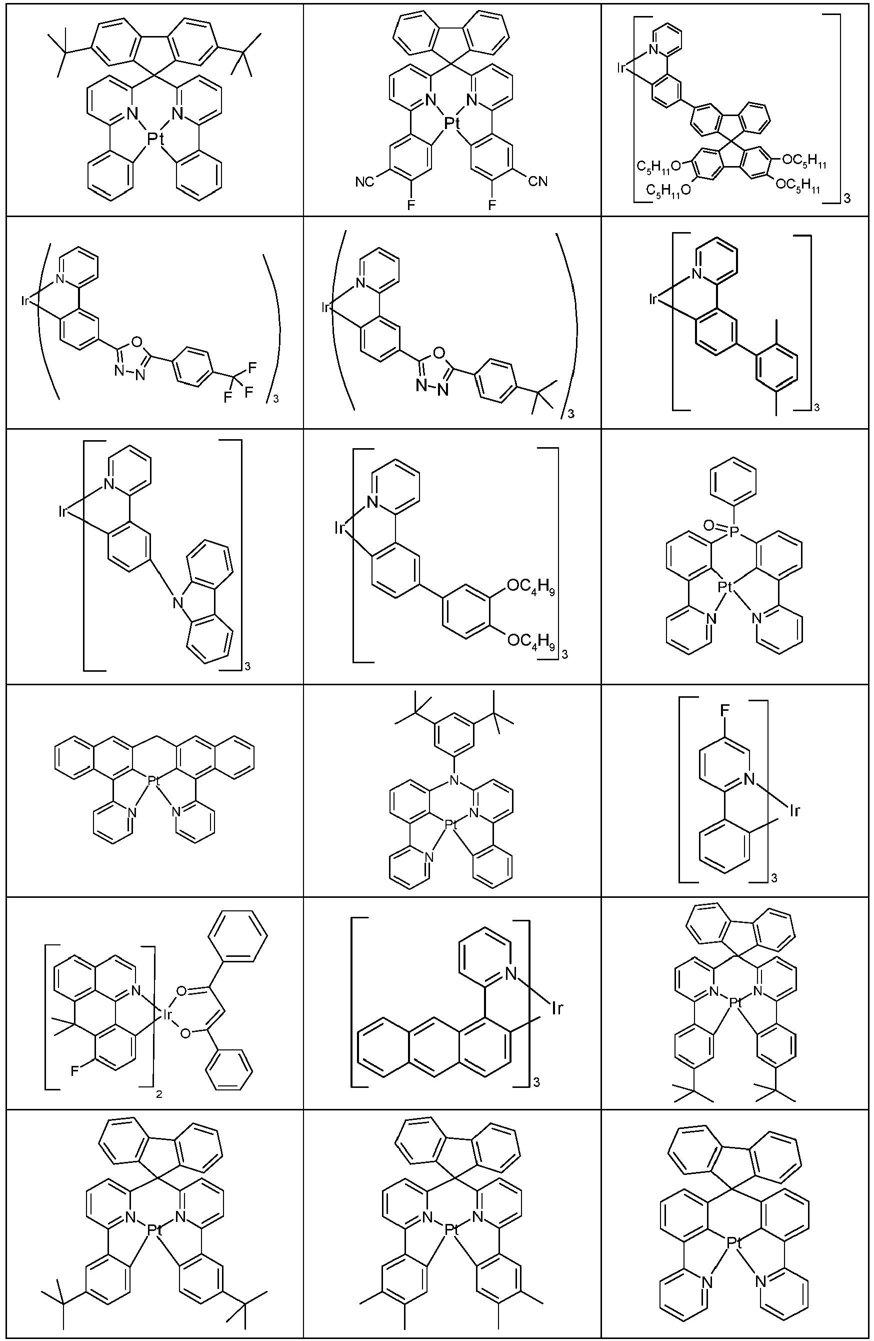

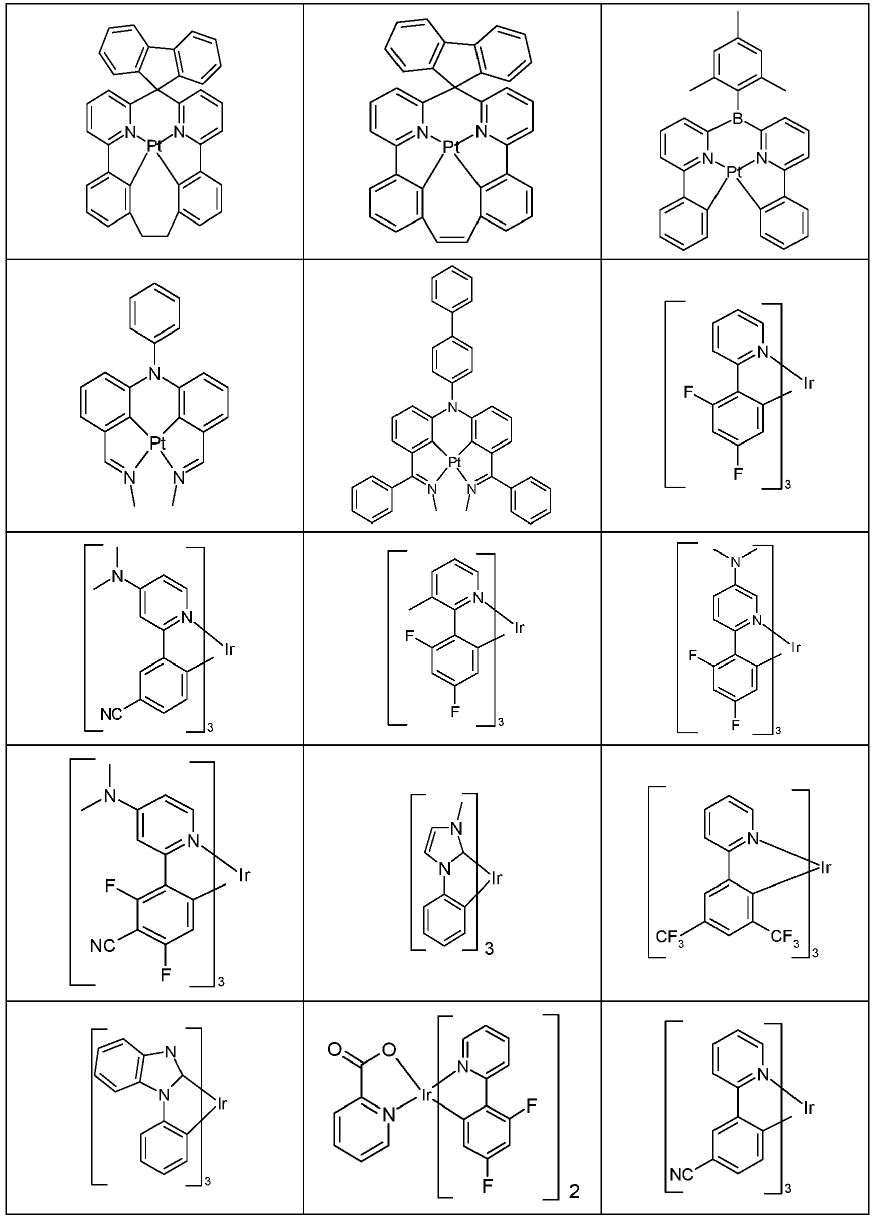

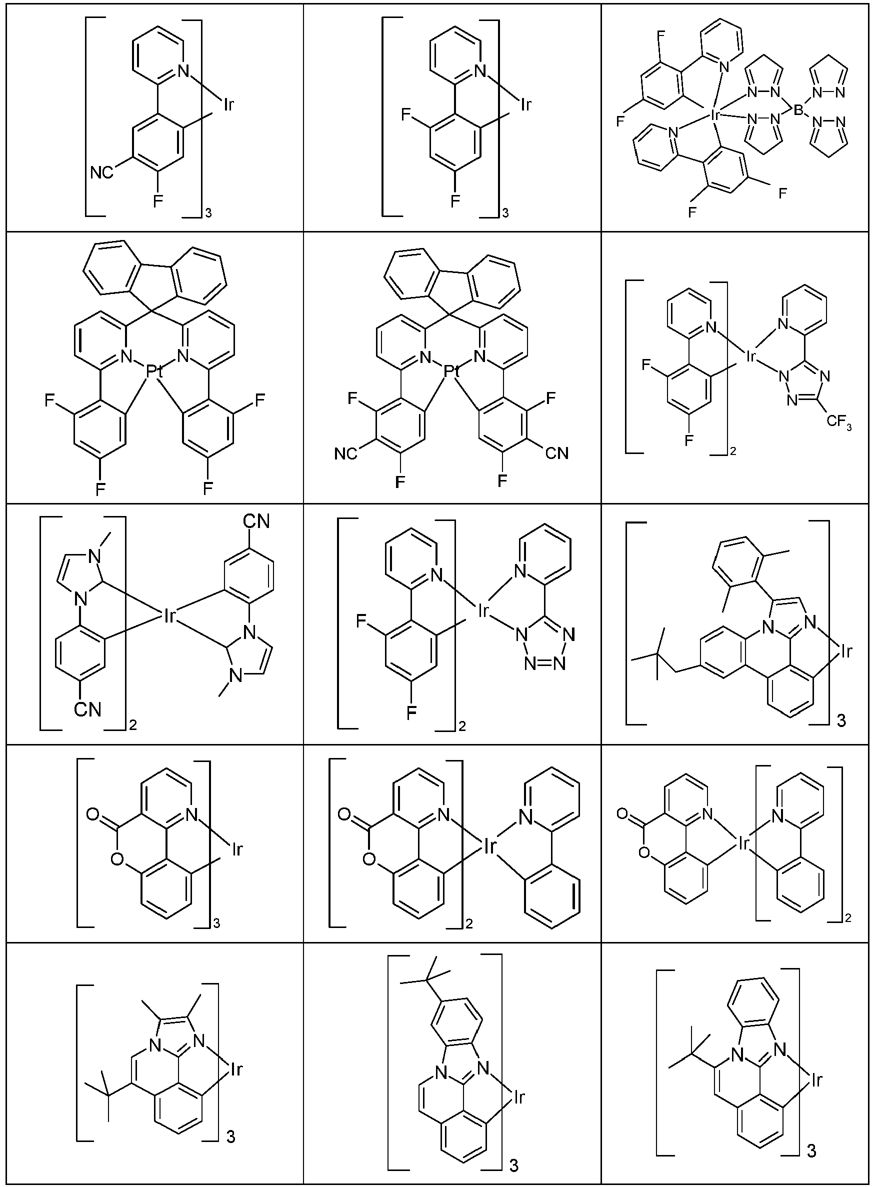

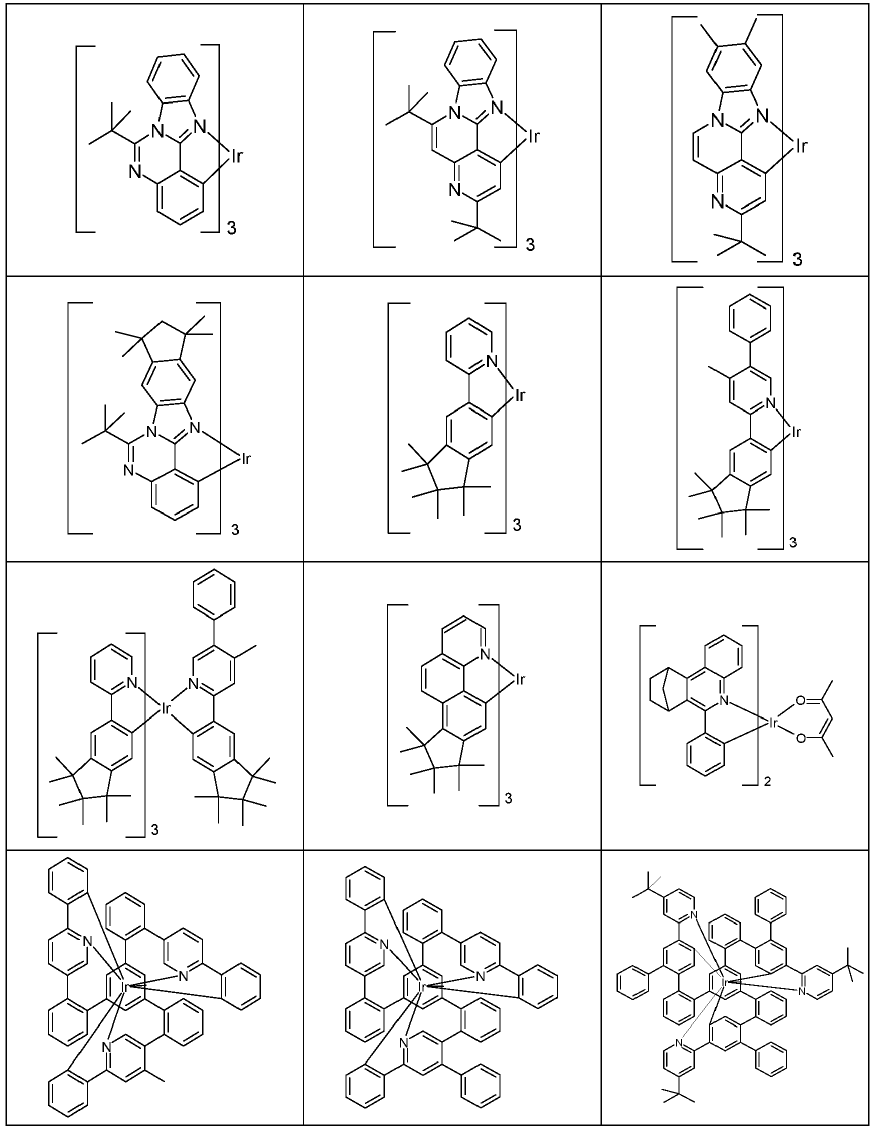

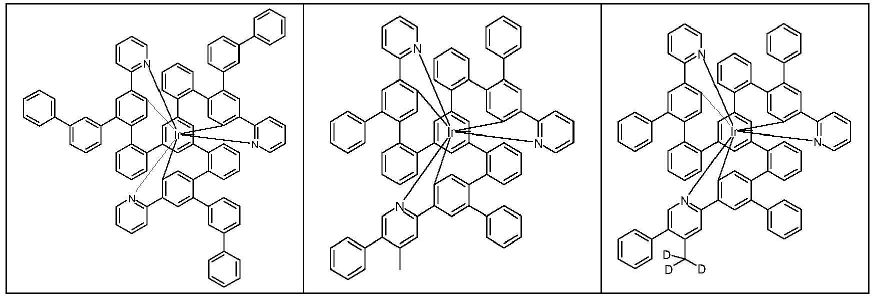

In einer bevorzugten Ausführungsform der vorliegenden Erfindung wird die Verbindung der Formel (I) in einer emittierenden Schicht als Matrixmaterial in Kombination mit einer oder mehreren emittierenden Verbindungen, vorzugsweise phosphoreszierenden emittierenden Verbindungen, eingesetzt. Die phosphoreszierende emittierende Verbindung ist dabei bevorzugt ein rot oder grün phosphoreszierender Emitter. Dies ist die am stärksten bevorzugte Verwendung der Verbindungen der Formel (I).

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) stellt dabei bevorzugt das Matrixmaterial mit lochtransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen oder den bevorzugten Matrixmaterialien für fluoreszierende emittierende Verbindungen, je nachdem welche Art von emittierender Verbindung im mixed-Matrix-System eingesetzt wird.

In einer alternativen bevorzugten Ausführungsform der Erfindung werden die Verbindungen gemäß Formel (I) als Matrixmaterial für Emittermaterialien eingesetzt, welche TADF (thermisch aktivierte verzögerte Fluoreszenz) zeigen. Derartige Emittermaterialien sind dem Fachmann bekannt. Insbesondere können die in H. Uoyama et al., Nature 2012, 492, 234 ff., in Abb. 1b) gezeigten Verbindungen als TADF-Emittermaterialien verwendet werden.

In einer alternativen bevorzugten Ausführungsform der Erfindung werden die Verbindungen gemäß Formel (I) als elektronentransportierendes Material eingesetzt. Dies gilt insbesondere, wenn die Verbindung der Formel (I) mindestens eine elektronenarme Heteroarylgruppe, wie beispielsweise Triazinyl, Pyrimidyl, Pyrazinyl, Pyridazinyl, Pyridyl, Benzimidazolyl, Chinazolinyl, Chinolinyl oder Phenanthrolinyl, enthält.

Wenn die Verbindungen als elektronentransportierendes Material eingesetzt werden, dann werden sie bevorzugt in einer Lochblockierschicht, einer Elektronentransportschicht oder in einer Elektroneninjektionsschicht eingesetzt. In einer bevorzugten Ausführungsform ist dabei die genannte Schicht n-dotiert. Die Schicht enthaltend die Verbindung der Formel (I) kann diese Verbindung aber auch als Reinmaterial enthalten.

Unter einem n-Dotanden wird vorliegend eine organische oder anorganische Verbindung verstanden, die in der Lage ist, Elektronen abzugeben (Elektronendonator), d.h. eine Verbindung, die als Reduktionsmittel wirkt.

Die zur n-Dotierung eingesetzten Verbindungen können als Precursor eingesetzt werden, wobei diese Precursor-Verbindungen durch Aktivierung n-Dotanden freisetzen.

Bevorzugt sind n-Dotanden ausgewählt aus elektronenreichen Metallkomplexen; P=N-Verbindungen; N-Heterocyclen, besonders bevorzugt, Naphthylencarbodiimiden, Pyridinen, Acridinen und Phenazinen; Fluorenen und Radikal-Verbindungen.

Besonders bevorzugte elektronenreiche Metallkomplexe werden unter anderem in der Offenlegungsschrift WO 2005/86251 A2 beschrieben, welche zu Offenbarungszwecken in die vorliegende Anmeldung einzubeziehen ist. Unter den elektronenreichen Metallkomplexen sind neutrale elektronenreiche Metallkomplexe bevorzugt.

Besonders bevorzugte P=N-Verbindungen werden unter anderem in der Offenlegungsschrift WO 2012/175535 A1 offenbart, welche zu Offenbarungszwecken in die vorliegende Anmeldung einzubeziehen ist.

Eine weitere Gruppe von n-Dotanden stellen N-Heterocyclen dar. N-Heterocyclen sind cyclische Verbindungen, deren Ringstruktur neben Wasserstoff und Kohlenstoff mindestens ein Stickstoffatom aufweist. Diese Verbindungen können gesättigt, teilweise ungesättigt oder heteroaromatisch sein.

N-Heterocyclen können bevorzugt als Precursor eingesetzt werden, wobei sich Precursor-Verbindungen dadurch auszeichnen, dass ihre Wirkung als n-Dotand erst nach einer Aktivierung einsetzt. Bevorzugte N-Heterocyclen, die sich insbesondere als Precursor eingesetzt werden können, sind unter anderem in der Offenlegungsschrift WO 2009/00237 A1 beschrieben, welche zu Offenbarungszwecken in die vorliegende Anmeldung einzubeziehen ist.

Eine weitere Gruppe N-Heterocyclen, die sich als n-Dotand eignen, stellen Naphthylencarbodiimide dar. Naphthylencarbodiimide umfassen mindestens eine Carbodiimid-Gruppe (N=C=N) und eine Naphthylen-Gruppe.

Überraschende Vorteile können durch die in der Offenlegungsschrift WO 2012/168358 A1 beschriebenen Naphthylencarbodiimide erzielt werden, welche zu Offenbarungszwecken in die vorliegende Anmeldung einzubeziehen ist.

Zu den bevorzugten, als n-Dotanden einsetzbare N-Heterocylcen zählen weiterhin Pyridin-, Acridin- und Phenazin-Derivate. Diese Verbindungen umfassen Pyridin-, Acridin- und Phenazin-Strukturelemente und sind in der Fachwelt bekannt. Bevorzugte Acridine und Phenazine sind unter anderem in der Offenlegungsschrift US 2007/0145355 A1 dargelegt, welche zu Offenbarungszwecken in die vorliegende Anmeldung einzubeziehen ist.

Überraschende Vorteile können durch die in EP 2 452 946 A1 und EP 2 463 927 A1 beschriebenen Pyridine erzielt werden, welche zu Offenbarungszwecken in die vorliegende Anmeldung einzubeziehen sind.

Gemäß einer besonderen Ausgestaltung der vorliegenden Erfindung können Fluorene als n-Dotanden eingesetzt werden. Bevorzugte Fluorene sind unter anderem in der Offenlegungsschrift WO 2012/031735 A1 beschrieben, welche zu Offenbarungszwecken in die vorliegende Anmeldung einzubeziehen ist.

Zu den bevorzugten n-Dotanden zählen Radikal-Verbindungen, die in der Fachwelt bekannt sind. Bevorzugte Radikal-Verbindungen umfassen heterocyclische Gruppen. Besonders bevorzugte Radikal-Verbindungen sind unter anderem in EP 1 837 926 A1 und WO 2007/107306 A1 offenbart, welche zu Offenbarungszwecken in die vorliegende Anmeldung einzubeziehen sind.

Von den genannten n-Dotanden sind die in WO 2005/86251 A2 offenbarten elektronenreichen Metallkomplexe besonders bevorzugt, wobei die Metallkomplexe der Formel W₂(hpp)₄ ganz besonders bevorzugt sind, worin hpp für das Anion von 1, 3, 4, 6, 7, 8-Hexahydro-2H-pyrimido [1, 2-a] pyrimidin steht. Hierbei sind neutrale elektronenreiche Metallkomplexe besonders bevorzugt.

Im Folgenden werden bevorzugte Ausführungsformen für die unterschiedlichen Funktionsmaterialien der elektronischen Vorrichtung aufgeführt.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine, die in WO 2014/111269 und in WO 2017/036574 offenbarten erweiterten Benzoindenofluorene, die in WO 2017/028940 und WO 2017/028941 offenbarten Phenoxazine, und die in WO 2016/150544 offenbarten Fluoren-Derivate, die mit Furan-Einheiten oder mit Thiophen-Einheiten verbunden sind.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, die in EP 1749809, EP 1905754 und US 2012/0187826 offenbarten Pyren-Verbindungen, die in WO 2015/158409 offenbarten Benzanthracenyl-Anthracen-Verbindungen, die in WO 2017/025165 offenbarten Indeno-Benzofurane, und die in WO 2017/036573 offenbarten Phenanthryl-Anthracene.

Bevorzugte Matrixmaterialien für phosphoreszierende emittierende Verbindungen sind neben den Verbindungen der Formel (I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Verbindungen, die bevorzugt in lochtransportierenden Schichten der erfindungsgemäßen OLEDs eingesetzt werden, sind insbesondere Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938, WO 2014/015935 und WO 2015/082056), Spiro-Dibenzopyran-Amine (z. B. gemäß WO 2013/083216), Dihydroacridin-Derivate (z. B. gemäß WO 2012/150001), Spirodibenzofurane und Spirodibenzothiophene, z.B. gemäß WO 2015/022051, WO 2016/102048 und WO 2016/131521, Phenanthren-Diarylamine, z.B. gemäß WO 2015/131976, Spiro-Tribenzotropolone, z.B. gemäß WO 2016/087017, Spirobifluorene mit meta-Phenyldiamingruppen, z.B. gemäß WO 2016/078738, Spiro-Bisacridine, zB. gemäß WO 2015/158411, Xanthen-Diarylamine, z.B. gemäß WO 2014/072017, und 9,10-Dihydroanthracen-Spiroverbindungen mit Diarylaminogruppen gemäß WO 2015/086108.

Als Materialien für die Elektronentransportschicht können neben den erfindungsgemäßen Verbindungen alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

### Stufe a) 7- Brom-[1,4]Benzothiazino[2,3,4-kl]phenothiazin

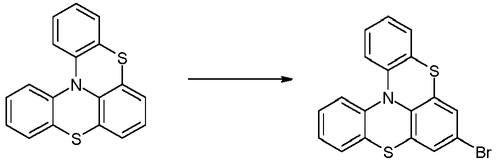

Eine Lösung von [1,4]Benzothiazino[2,3,4-kl]phenothiazin (CAS 1050521-47, 48,5 g, 154 mmol) in Chloroform (1000 mL) wird bei 0 °C unter Lichtausschluss portionsweise mit N-Bromsuccinimid (24.7 g, 139 mmol) versetzt und 2 h bei dieser Temperatur gerührt. Die Reaktion wird durch Zugabe von Natriumsulfit-Lösung beendet und weitere 30 min bei Raumtemperatur gerührt. Nach Phasentrennung wird die organische Phase mit Wasser gewaschen und die wässrige Phase mit Dichlormethan extrahiert. Die vereinigten organischen Phasen werden über Natriumsulfat getrocknet und im Vakuum eingeengt. Der Rückstand wird in Essigsäureethylester gelöst und über Kieselgel filtriert. Anschließend wird das Rohprodukt aus Heptan umkristallisiert.

Ausbeute: 42 g (110 mmol), 64 % d. Th., farbloser Feststoff.

Analog können folgende Verbindungen erhalten werden:

| **Nr.** | **Edukt** | **Produkt** | **Ausbeute** |
|---|---|---|---|
| 2a | 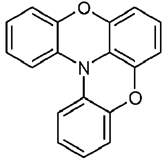 [784189-24-8 ] | 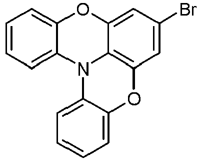 | 82% |
| 3a | 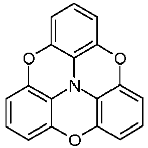 [781643-18-3 ] | 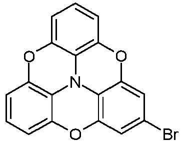 | 65% |
| 4a | 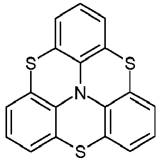 [252020-71-6 ] | 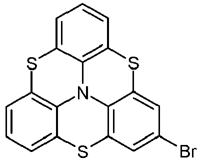 | 71% |

Analog können mit zwei Äq. NBS folgende Verbindungen erhalten werden:

| **Nr.** | **Edukt** | **Produkt** | **Ausbeute** |
|---|---|---|---|
| 5a | 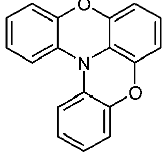 [784189-24-8] | 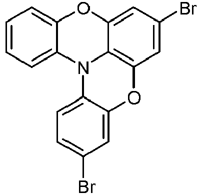 | 56% |
| 6a | 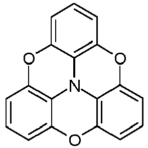 [781643-18-3 ] | 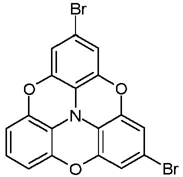 | 58% |
| 7a | 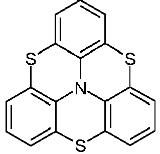 [252020-71-6 ] | 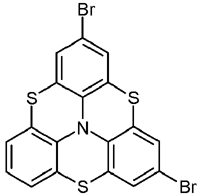 | 51% |

### Stufe b) Dithia-13b-aza-naphtho[3,2,1-de]anthracen-7-boronsäure

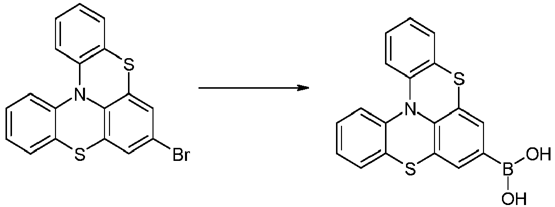

28 g (73 mmol) 7- Brom-[1,4]Benzothiazino[2,3,4-kl]phenothiazin werden in 150 mL trockenem THF gelöst und auf -78°C gekühlt. Bei dieser Temperatur wird mit 30 mL (76 mmol / 2.5 M in Hexan) n-Butyllithium innerhalb von ca. 5 min. versetzt und anschließend für 2.5h bei -78°C nachgerührt. Bei dieser Temperatur wird mit 15 g (145 mmol) Borsäure-trimethylester möglichst zügig versetzt und die Reaktion langsam auf Raumtemperatur kommen gelassen (ca. 18h). Die Reaktionslösung wird mit Wasser gewaschen und der ausgefallene Feststoff und die organische Phase werden mit Toluol azeotrop getrocknet. Das Rohprodukt wird aus Toluol/Methylenchlorid bei ca. 40°C ausgerührt und abgesaugt. Ausbeute: 22g (63 mmol), 90 % der Theorie.

Analog dazu werden die folgenden Verbindungen hergestellt:

| **Nr.** | **Edukt** | **Produkt** | **Ausbeute** |
|---|---|---|---|
| 2b | 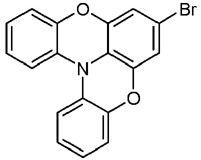 | 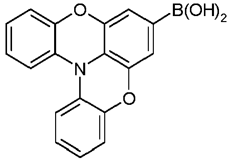 | 69% |
| 3b | 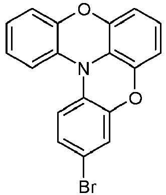 [1416903-68-8] | 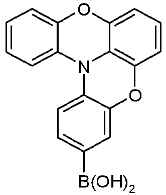 | 71% |
| 4b | 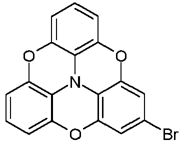 | 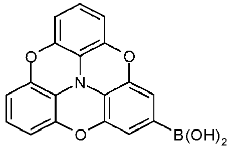 | 72% |
| 5b | 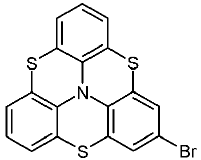 | 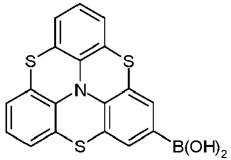 | 68% |
| 6b | 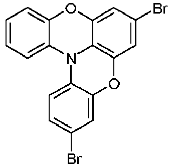 | 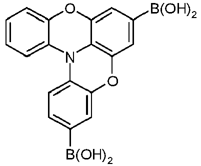 | 66% |
| 7b | 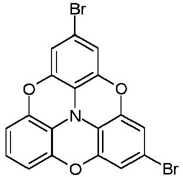 | 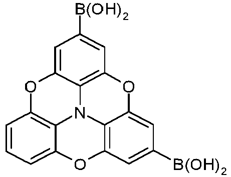 | 68% |
| 8b | 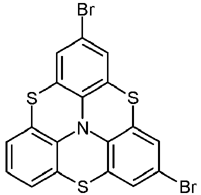 | 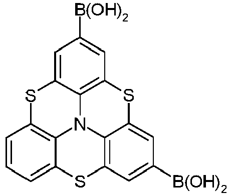 | 71% |
| 9b | 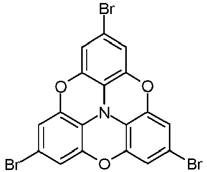 [1380076-62-9 ] | 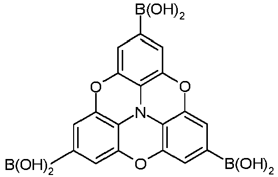 | 80% |

### Stufe c): 3,7-Bis-dibenzofuran-1-yl-5,9-dioxa-13b-aza-naphtho[3,2,1de]-anthracen

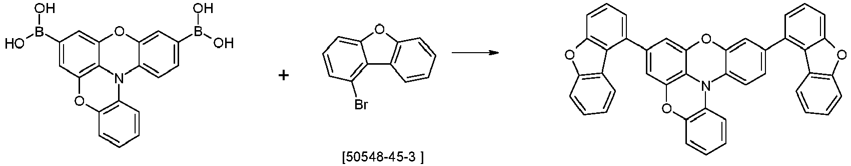

Zur einer entgasten Mischung aus 116 g (470 mmol) 1-Brom-Dibenzofuran, 169 g (470.0 mmol) 5,9-dioxa-13b-aza-naphtho[3,2,1-de]anthracen 3-7-bisboronsäure, 149.02 g (702.0 mmol) K₃PO₄, 1000 ml Dioxan und 1000 ml Wasser werden 13.52 g (11.7 mmol) Pd(PPh₃)₄ gegeben. Nach Erhitzen der Mischung für 7 h auf 80 °C werden 4.58 g (93.6 mmol) NaCN zugegeben. Nach Abkühlen auf Raumtemperatur wird die wässrige Phase abgetrennt. Die organische Phase wird zweimal mit H₂O gewaschen und anschließend über Na₂SO₄ getrocknet. Nach Entfernen des Lösungsmittels und zweimaliger Umkristallisation des dunkelroten Feststoffs aus Dioxan wurde das Produkt in Form von roten Nadeln erhalten.

Ausbeute: 184 g (304 mmol),66% d. Th.; Reinheit: 97 % n. HPLC

Analog können folgende Verbindungen hergestellt werden:

| **Nr.** | **Edukt 1** | **Edukt 2** | **Produkt** | **Ausbeute** |
|---|---|---|---|---|
| 1c | 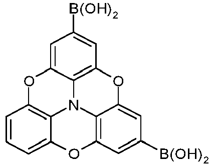 | 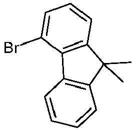 942615-32-9 | 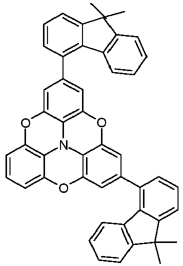 | 80% |
| 2c | 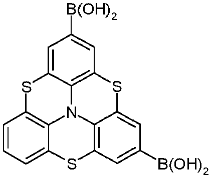 | 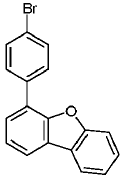 955959-84-9 | 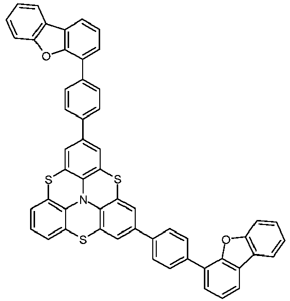 | 74% |

### Stufe d) 3-Bromo-7,11-bis-dibenzofuran-1-yl-5,9-dioxa-13b-aza-naphtho[3,2,1 -de]anthracen

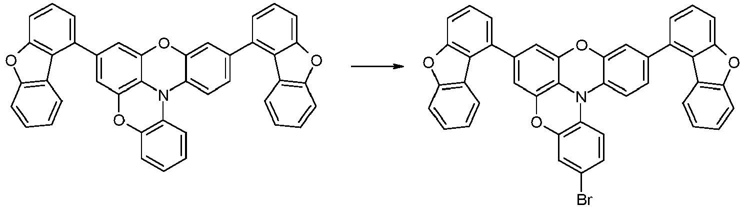

45,1 g (74,6 mmol) 3,7-Bis-dibenzofuran-1-yl-5,9-dioxa-13b-aza-naphtho[3,2,1de] anthracen werden in 80 mL DMF vorgelegt. Anschließend werden 13,3 g (74,6 mmol) NBS portionsweise zugegeben und man rührt 4 h weiter bei dieser Temperatur. Anschließend wird die Mischung mit 15mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 39 g (58 mmol), 78% der Theorie, Reinheit nach ¹H-NMR ca. 96 %.

Analog können folgende Verbindungen hergestellt werden:

| **Nr.** | **Edukt 1** | **Produkt** | **Ausbeute** |
|---|---|---|---|
| 1d | 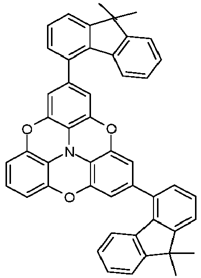 | 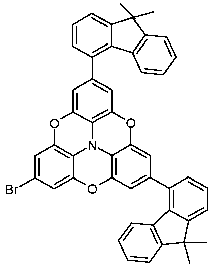 | 80% |
| 2d | 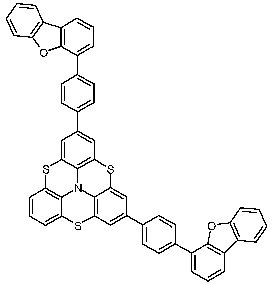 | 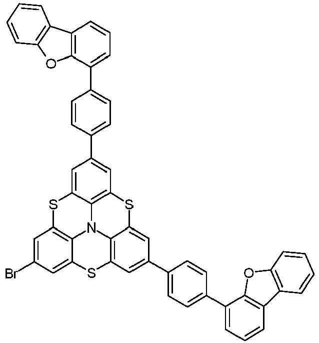 | 74% |

### Stufe e) 7-[9-(4,6-Diphenyl-[1,3,5]triazin-2-yl)-9H-carbazol-3-yl]-5,9-dithia-13b-aza-naphtho[3,2,1-de]anthracen

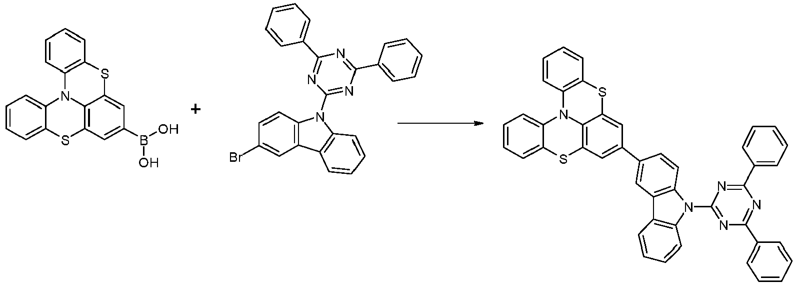

11 g (32 mmol) Dithia-13b-aza-naphtho[3,2,1-de]anthracen-7-boronsäure, 14 g (31,6 mmol) 3-Brom-9-(4,6-diphenyl-[1,3,5]triazin-2-yl)-9H-carbazol, und 31 ml (63 mmol) Na₂CO₃ (2 M-Lösung) werden in 120 mL Toluol, 120 mL Ethanol suspendiert. Zu dieser Suspension werden 0,73 g (0,63 mmol) Pd(PPh₃)₄ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert und durch zweimalige Sublimation im Vakuum (p = 5 × 10⁻⁵ mbar, T = 329 °C) gereinigt. Die Ausbeute beträgt 16,7 g (24 mmol), entsprechend 76% der Theorie.

Analog werden folgende Verbindungen erhalten:

| **Bsp.** | **Edukt 1** | **Edukt 2** | **Produkt** | **Ausbeute** |
|---|---|---|---|---|
| 1e | 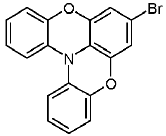 | 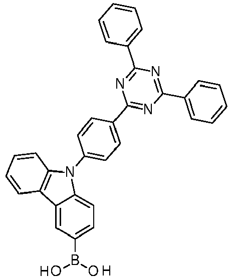 | 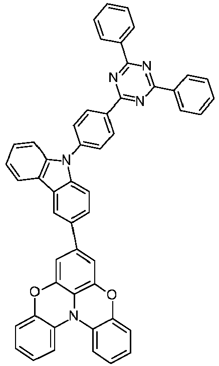 | 64% |
| 2e | 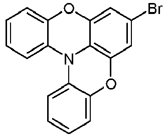 | 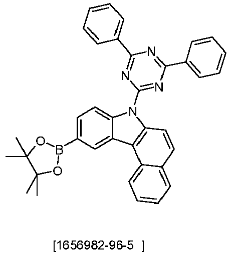 | 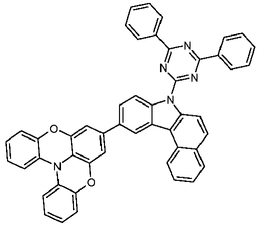 | 63 % |
| 3e | 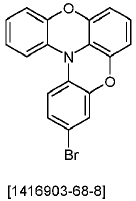 | 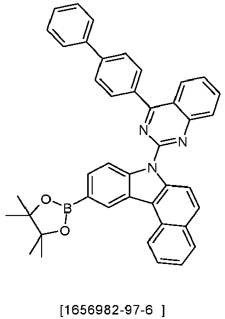 | 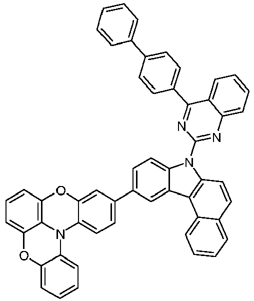 | 73% |
| #4e | 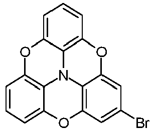 | 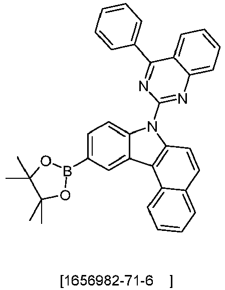 | 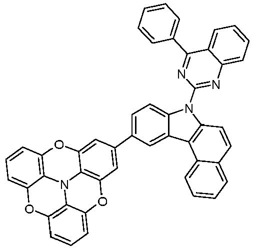 | 79% |
| 5e | 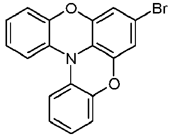 | 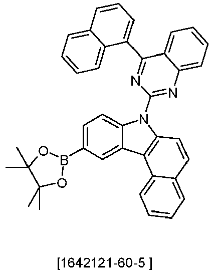 | 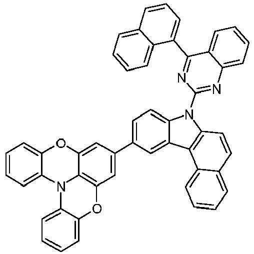 | 70% |
| #6e | 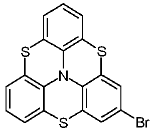 | 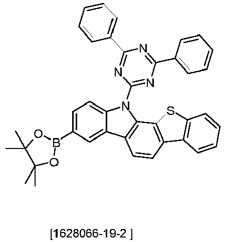 | 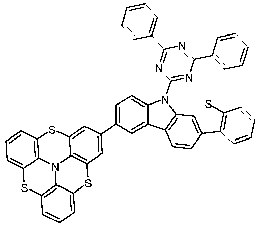 | 74% |
| 7e | 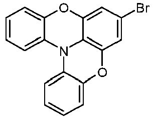 | 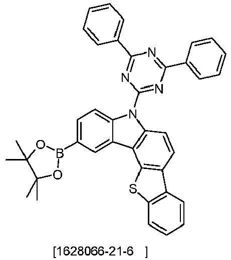 | 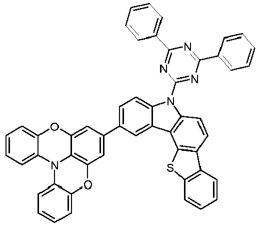 | 71% |
| 8e | 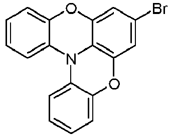 | 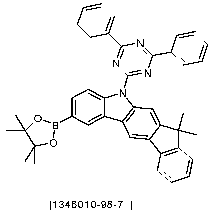 | 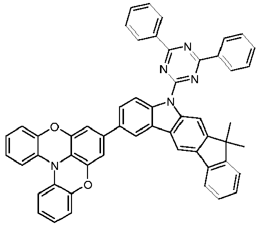 | 65% |
| #9e | 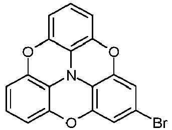 | 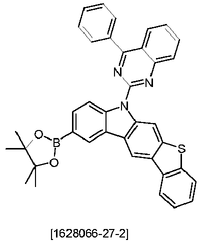 | 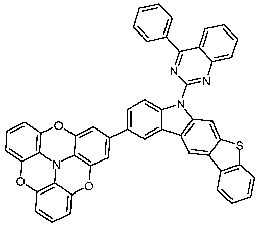 | 87% |
| 10e | 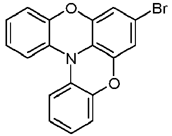 | 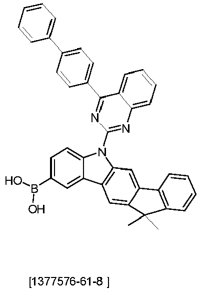 | 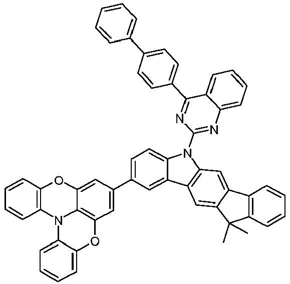 | 73% |
| #11e | 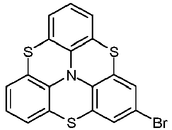 | 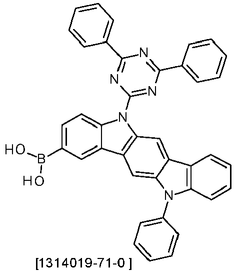 | 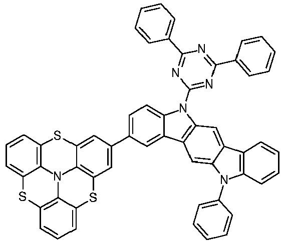 | 72% |
| 12e | 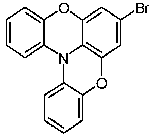 | 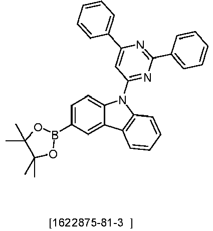 | 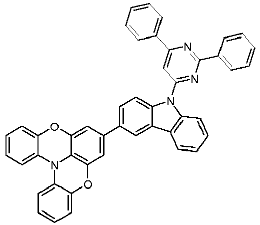 | 70% |
| 13e | 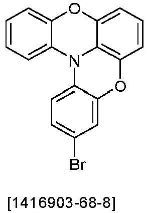 | 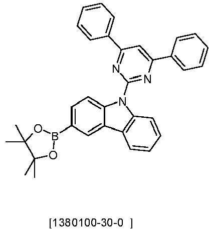 | 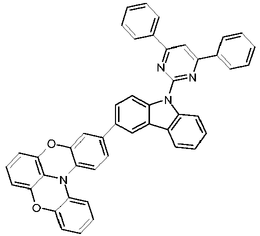 | 77% |
| #14e | 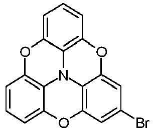 | 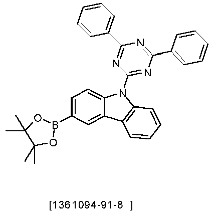 | 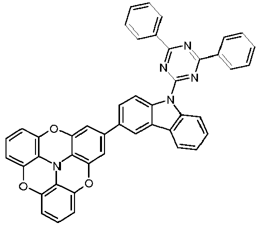 | 76% |
| 15e | 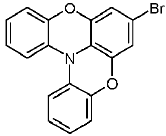 | 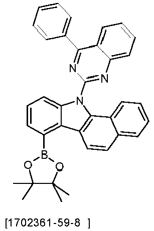 | 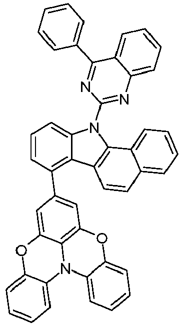 | 70% |
| #16e | 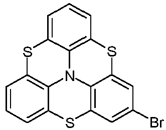 | 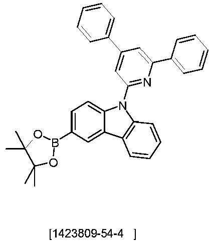 | 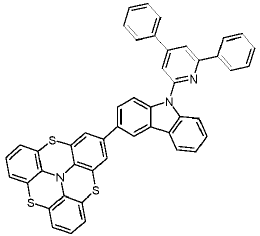 | 69% |
| 17e | 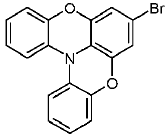 | 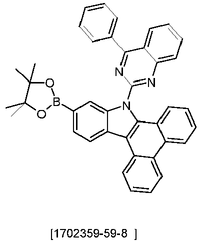 | 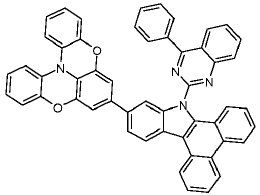 | 80% |
| 18e | 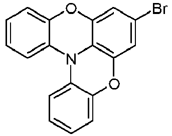 | 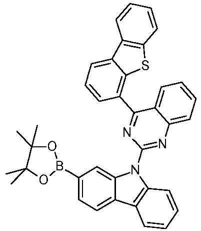 [1702359-04-3 ] | 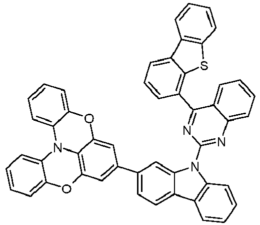 | 72% |
| 19e | 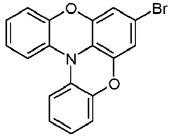 | 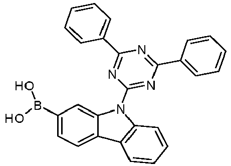 [1642121-55-8 ] | 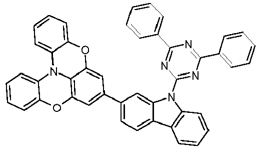 | 73% |
| 20e | 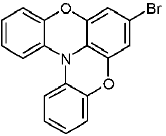 | 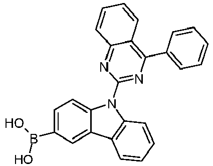 [1642121-58-1 ] | 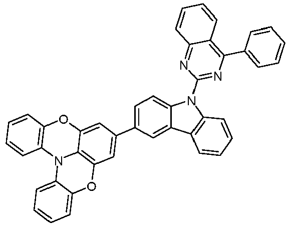 | 77% |
| #21e | 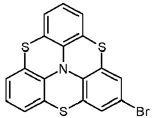 | 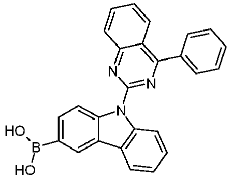 [1642121-58-1 ] | 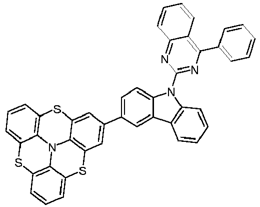 | 71% |
| 22e | 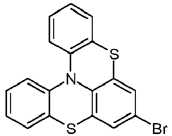 | 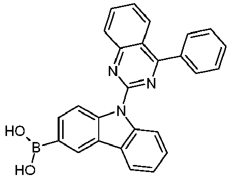 [1842121-58-1 ] | 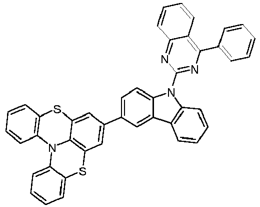 | 56% |
| 23e | 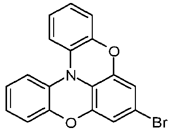 | 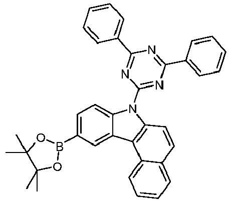 [1656982-96-5 ] | 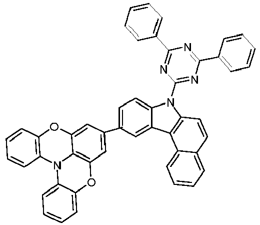 | 72% |
| 24e | 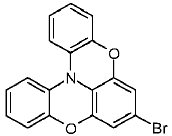 | 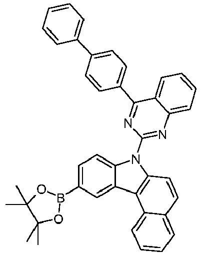 [1656982-97-6 ] | 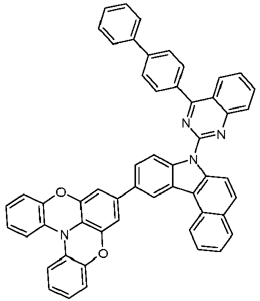 | 78% |
| 25e | 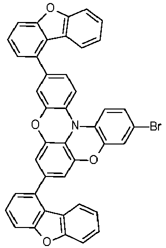 | 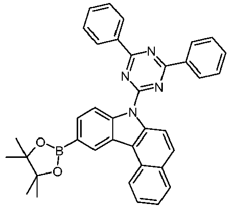 [1656982-96-5 ] | 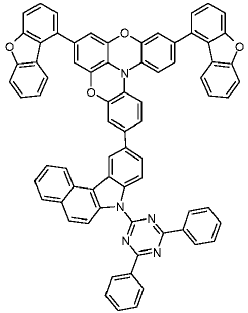 | 77% |
| #26e | 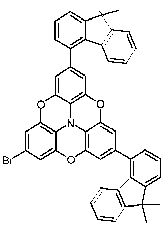 | 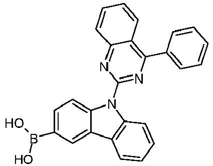 [1642121-58-1 ] | 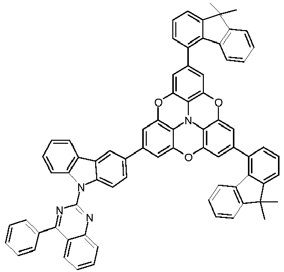 | 70% |
| #27e | 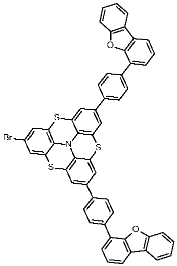 | 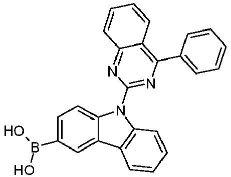 [1842121-58-1 ] | 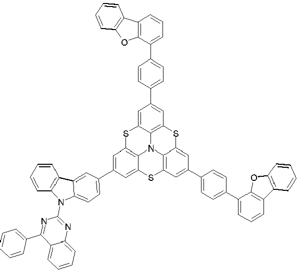 | 67% |
| 28e | 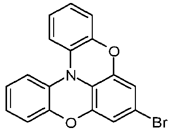 | 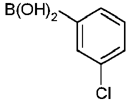 [63503-60-6 ] | 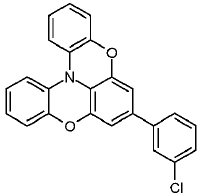 | 69% |
| 29e | 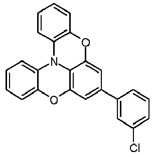 | 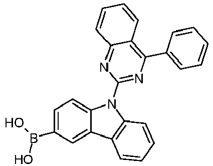 [1642121-58-1 ] | 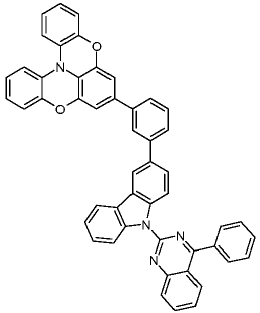 | 72% |
| 31e | 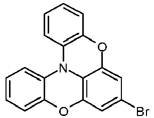 | 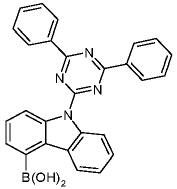 [1821233-72-0 ] | 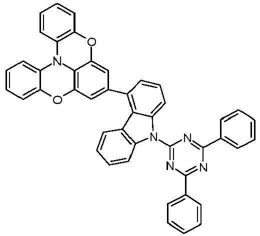 | 67% |
| 32e | 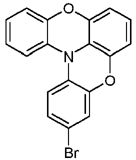 [1416903-68-8] | 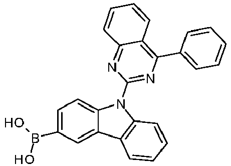 [1642121-58-1 ] | 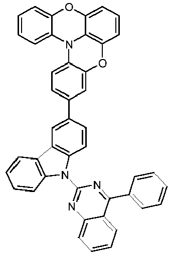 | 77% |
| #33e | 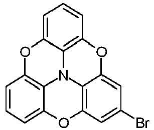 | 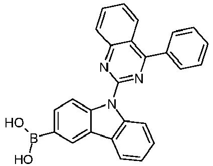 [1642121-58-1 ] | 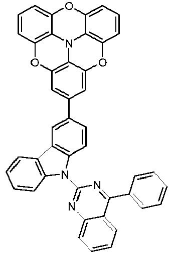 | 75% |
| 34e | 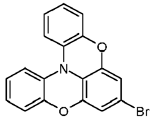 | 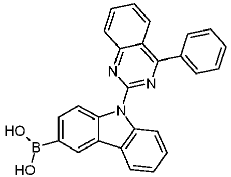 [1842121-58-1 ] | 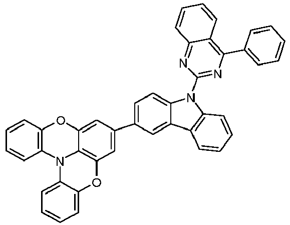 | 70% |
| 35e | 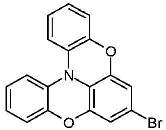 | 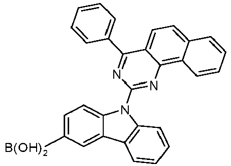 [1813537-15-3 ] | 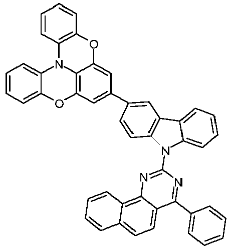 | 65% |
| 36e | 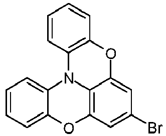 | 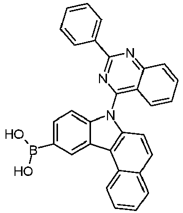 [1813537-17-5] | 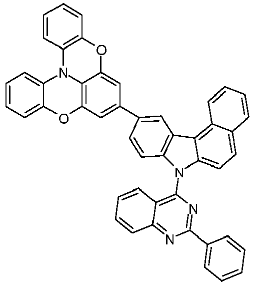 | 67% |
| 37e | 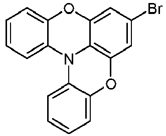 | 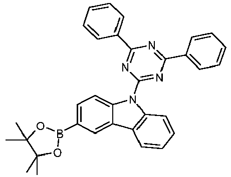 [1361094-91-8 ] | 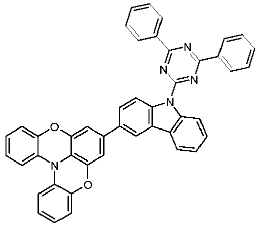 | 77% |
| 38e | 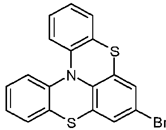 | 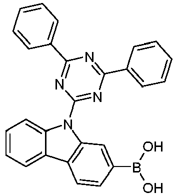 [. 1642121-55-8 ] | 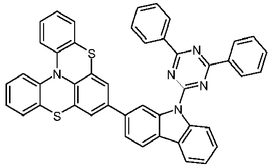 | 69% |
| 39e | 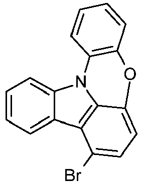 CAS [1415901-44-8 ] | 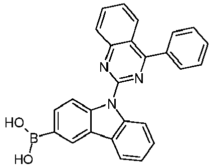 [1642121-58-1 ] | 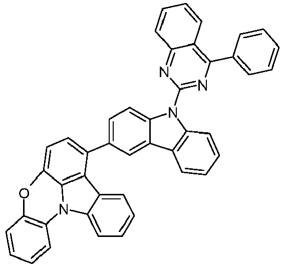 | 62% |
| 40e | 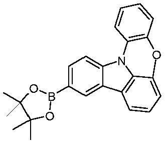 [1380485-64-2 ] | 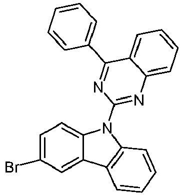 [1476799-10-6] | 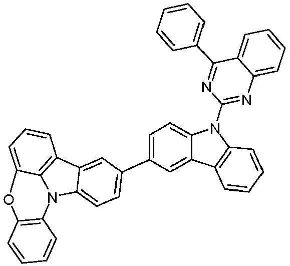 | 61% |
| 41e | 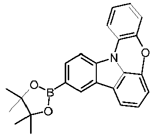 [1380485-64-2 ] | 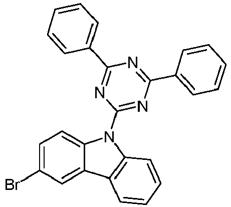 [1266389-17-6 ] | 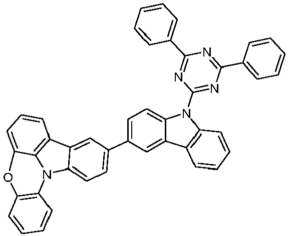 | 66% |

Die obenstehenden und im Folgenden mit # markierten Verbindungen sind nicht anspruchsgemäß.

### B) Device-Beispiele

Die folgenden Beispiele E1 bis E12 (siehe Tabelle 1) zeigen die Verwendung der erfindungsgemäßen Verbindungen in OLEDs. Beispiele V1 bis V4 (siehe Tabelle 1) sind Referenzbeispiele.

### 1) Allgemeine Beschreibung der Herstellung und Vermessung der OLEDs:

Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) und abschließend eine Kathode. Die Kathode wird durch eine 100 nm dicke Aluminiumschicht gebildet. Als HIL wird eine 5 nm dicke Schicht des Materials HATCN verwendet. Als HTL wird eine 125 nm dicke Schicht des Materials SpMA1 verwendet. Als EBL wird eine 10 nm dicke Schicht des Materials SpMA3 verwendet. Der weitere Aufbau der OLEDs ist Tabelle 1 zu entnehmen. Die zur Herstellung der OLEDs verwendeten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC5:IC3:TEG2 (55%:35%:10%) bedeutet hierbei, dass das Material IC5 in einem Volumenanteil von 55%, IC3 in einem Anteil von 35% und TEG2 in einem Anteil von 10%, jeweils bezogen auf das Volumen, in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren und die Strom-Spannungs-Leuchtdichte-Kennlinien (IUL-Kennlinien) gemessen. Die Elektrolumineszenzspektren werden bei einer Leuchtdichte von 1000 cd/m² bestimmt und daraus die CIE 1931 x und y Farbkoordinaten berechnet.

### 2) Vergleich der erfindungsgemäßen Verbindungen EG1 - EG4 mit Verbindungen gemäß Stand der Technik SdT1 bis SdT4

Die erfindungsgemäßen Materialien können in der Emissionsschicht in phosphoreszierenden roten OLEDs eingesetzt werden. Gegenüber dem Stand der Technik (Verbindungen SdT1-4 aus den Beispielen V1-V4 in Tabelle 1) ergibt sich beim Einsatz der erfindungsgemäßen Moleküle als Matrixmaterial in der Emissionsschicht eine Verringerung der Spannung bei einer Leuchtdichte von 1000 cd/m² um jeweils 10% (Direktvergleich der Beispiele E1 mit V1, E2 mit V2, E3 mit V3 und E4 mit V4). Dies stellt eine signifikante Verbesserung der OLEDs dar.

### 3) Verwendung der erfindungsgemäßen Verbindungen in der emittierenden Schicht, in der Lochblockierschicht und in der Elektronentransportschicht von OLEDs

Die erfindungsgemäßen Verbindungen EG1 bis EG9 und EG13 bis EG15 werden in den Beispielen E1 bis E12 als Matrixmaterial in der Emissionsschicht in Kombination mit phosphoreszierenden Emittern eingesetzt. Die Farbkoordinaten der Elektrolumineszenzspektren der OLEDs aus diesen Versuchen liegen bei CIEx=0.67 und CIEy= 0.33. Somit eignen sich die Materialien für den Einsatz in der Emissionsschicht von roten OLEDs.

Desweiteren lassen sich die erfindungsgemäßen Materialien in der Elektronentransportschicht (ETL) bzw. der Lochblockierschicht (HBL) erfolgreich einsetzen. Dies ist in den Versuchen E13 - E15 gezeigt. Auch hier liegen die Farbkoordinaten des Spektrums der OLEDs bei CIEx=0.67 und CIEy=0.33.

**Tabelle 1: Aufbau der OLEDs**

| **Bsp.** | **EML Dicke** | **HBL Dicke** | **ETL Dicke** |
|---|---|---|---|
| V1 | SdT1:TER5 (95%:5%) 40nm | --- | ST2:LiQ (50%:50%) 35nm |
| V2 | SdT2:TER5 (95%:5%) 40nm | --- | s.o. |
| V3 | SdT3:TER5 (95%:5%) 40nm | --- | s.o. |
| V4 | SdT4:TER5 (95%:5%) 40nm | --- | s.o. |
| E1 | EG1:TER5 (95%:5%) 40nm | --- | s.o. |
| E2 | EG2:TER5 (95%:5%) 40nm | --- | s.o. |
| E3 | EG3:TER5 (95%:5%) 40nm | --- | s.o. |
| E4 | EG4:TER5 (95%:5%) 40nm | --- | s.o. |
| E5 | EG5:SdT1:TER5 (85%:10%:5%) 40nm | --- | s.o. |
| E6 | EG6:TER5 (95%:5%) 40nm | --- | s.o. |
| E7 | EG7:TER5 (95%:5%) 40nm | --- | s.o. |
| E8 | EG8:TER5 (95%:5%) 40nm | --- | s.o. |
| E9 | EG9:TER5 (95%:5%) 40nm | --- | s.o. |
| E10 | EG13:TER5 (95%:5%) 40nm | --- | s.o. |
| E11 | EG14:TER5 (95%:5%) 40nm | --- | s.o. |
| E12 | EG15:TER5 | --- | s.o. |
| | (95%:5%) 40nm | | |
| E13 | EG1:TER5 (95%:5%) 40nm | --- | EG10:LiQ (50%:50%) 35nm |
| E14 | s.o. | EG11 5nm | ST2:LiQ (50%:50%) 30nm |
| E15 | s.o. | EG12 5nm | s.o. |

**Tabelle 2: Strukturformeln der Materialien für die OLEDs**

| | |
|---|---|
| 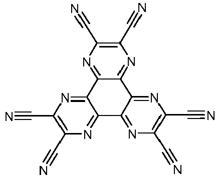 | 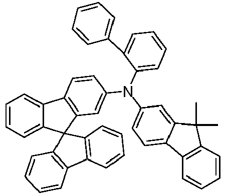 |
| HATCN | SpMA1 |
| 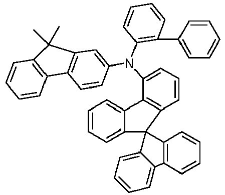 | 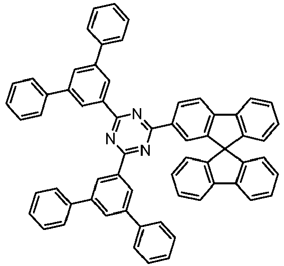 |
| SpMA3 | ST2 |
| 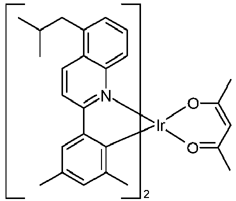 | 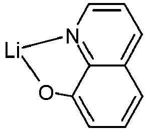 |
| TER5 | LiQ |
| 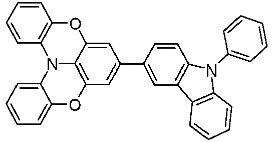 | 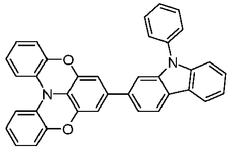 |
| SdT1 | SdT2 |
| 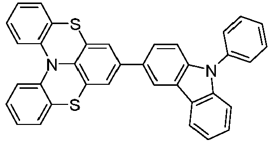 | 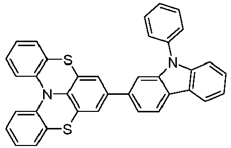 |
| SdT3 | SdT4 |
| 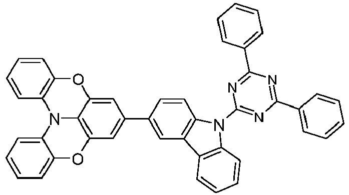 | 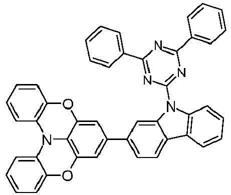 |
| EG1 | EG2 |
| 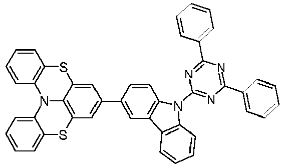 | 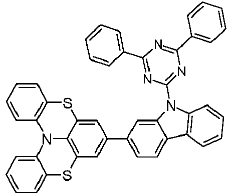 |
| EG3 | EG4 |
| 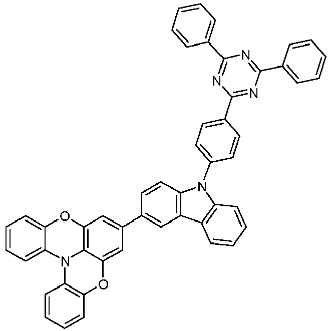 | 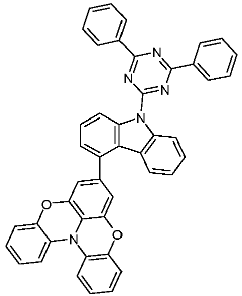 |
| EG5 | EG6 |
| 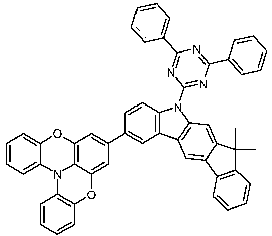 | 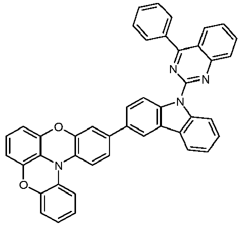 |
| EG7 | EG8 |
| 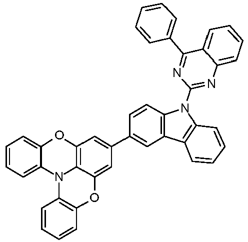 | 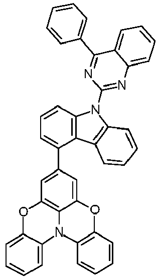 |
| EG9 | EG10 |
| 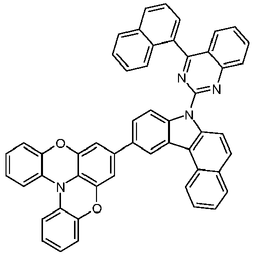 | 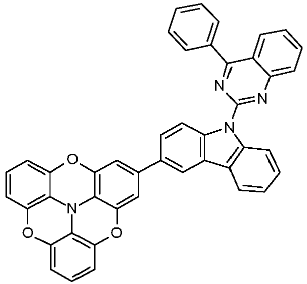 |
| EG11 | #EG12 |
| 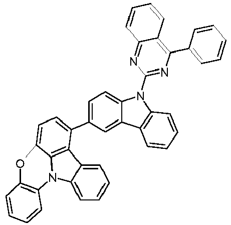 | 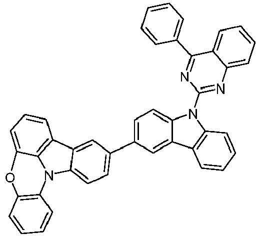 |
| EG13 | EG14 |
| 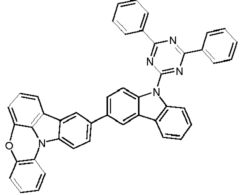 | |
| EG15 | |

## Patentansprüche

1. Verbindung gemäß Formel (I) 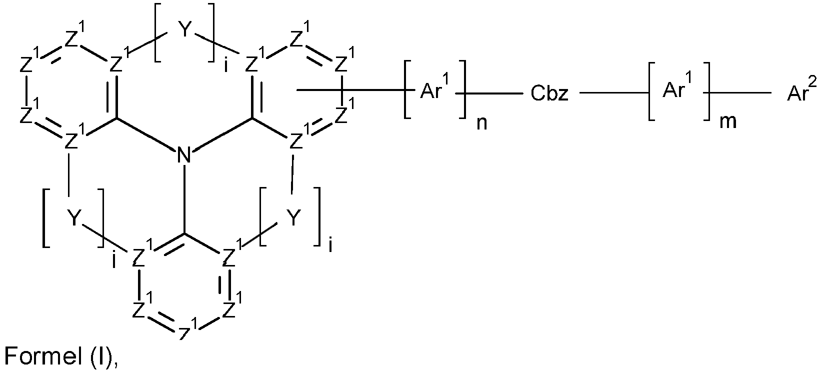 wobei für die auftretenden Variablen gilt:
Y ist bei jedem Auftreten gleich oder verschieden gewählt aus Einfachbindung, O und S, wobei mindestens eine Gruppe Y vorhanden ist, die gewählt ist aus O und S;
Z¹ ist bei jedem Auftreten gleich oder verschieden CR¹, N oder C, wobei eine Gruppe Z¹ genau dann gleich C ist, wenn eine Gruppe Y daran gebunden ist;
Ar¹ ist bei jedem Auftreten gleich oder verschieden ein aromatisches Ringsystem mit 6 bis 24 aromatischen Ringatomen, das mit einem oder mehreren Resten R² substituiert sein kann, oder ein heteroaromatisches Ringsystem mit 5 bis 24 aromatischen Ringatomen, das mit einem oder mehreren Resten R² substituiert sein kann;
Cbz ist eine divalente Gruppe, die an ihren freien Positionen jeweils mit einem Rest R³ substituiert ist, und die ein oder mehrere Strukturelemente der Formel (Cbz) enthält 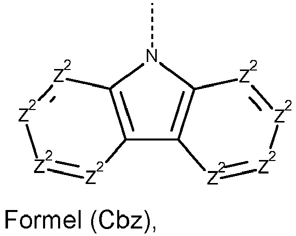
wobei eine der zwei Bindungen der divalenten Gruppe an den Rest der Verbindung die gestrichelte Bindung am Stickstoffatom der Formel (Cbz) ist,
wobei die zweite dieser zwei Bindungen an einer beliebigen freien Position der Gruppe angeordnet sein kann, und wobei
Z² bei jedem Auftreten gleich oder verschieden gewählt ist aus C und N;
Ar² ist gewählt aus Pyridin, Pyrimidin, Pyridazin, Pyrazin, Triazin, Benzimidazol, Chinolin, Chinazolin, Benzo[h]chinazolin, Phenanthrolin, Phenanthridin, Diazaphenanthren, und Acridin, die jeweils mit einem oder mehreren Resten R⁴ substituiert sein können;
R¹, R², R³, R⁴ sind bei jedem Auftreten gleich oder verschieden gewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² bzw. R³ bzw. R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch-R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R⁵ ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R⁶ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁶ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
i ist bei jedem Auftreten gleich oder verschieden 0 oder 1, wobei zwei der drei Indices i in Formel (I) gleich 1 sind, und einer der drei Indices i gleich 0 ist, und wobei die betreffende Gruppe Y nicht vorhanden ist, wenn der dazugehörige Index i = 0 ist;
n ist gleich 0, 1, 2, 3 oder 4;
m ist gleich 0, 1, 2, 3 oder 4.

2. Verbindung nach Anspruch 1, **dadurch gekennzeichnet, dass** Ar² gewählt ist aus Triazin und Chinazolin, die jeweils mit einem oder mehreren Resten R⁴ substituiert sein können.

3. Verbindung nach Anspruch 1 oder 2, **dadurch gekennzeichnet, dass** die Gruppe Cbz bei jedem Auftreten gleich oder verschieden gewählt ist aus Carbazol, Azacarbazol, Benzocarbazol, Dibenzocarbazol, Indenocarbazol, Indolocarbazol, Carbazol mit ankondensiertem Benzofuran und Carbazol mit ankondensiertem Benzothiophen, wobei die genannten Gruppen jeweils mit einem oder mehreren Resten R³ substituiert sein können.

4. Verbindung nach einem oder mehreren der Ansprüche 1 bis 3, **dadurch gekennzeichnet, dass** die Gruppe Cbz gewählt ist aus den Formeln
| | |
|---|---|
| 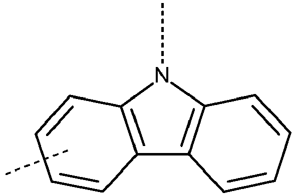 | 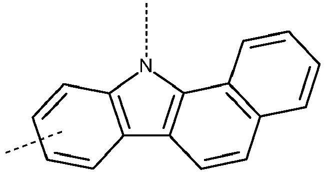 |
| Formel (Cbz-1) | Formel (Cbz-2) |
| 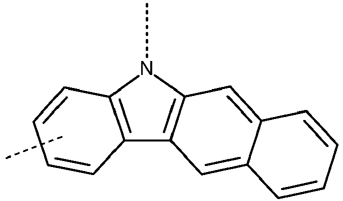 | 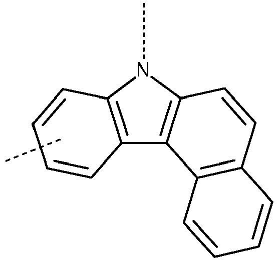 |
| Formel (Cbz-3) | Formel (Cbz-4) |
| 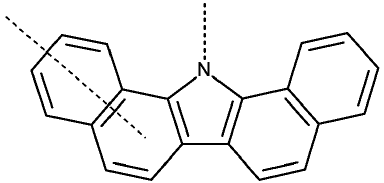 | 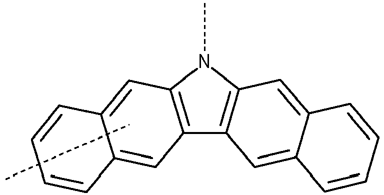 |
| Formel (Cbz-5) | Formel (Cbz-6) |
| 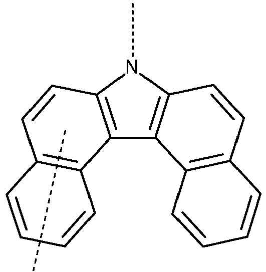 | 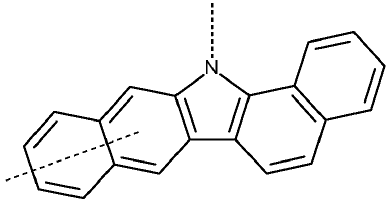 |
| Formel (Cbz-7) | Formel (Cbz-8) |
| 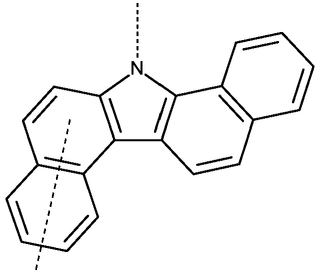 | 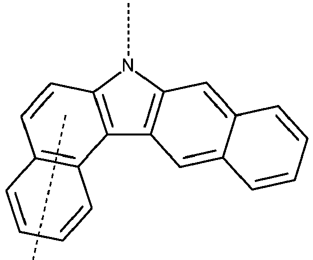 |
| Formel (Cbz-9) | Formel (Cbz-10) |
| 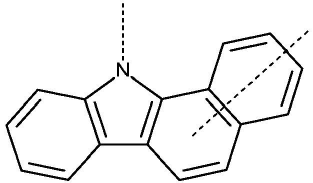 | 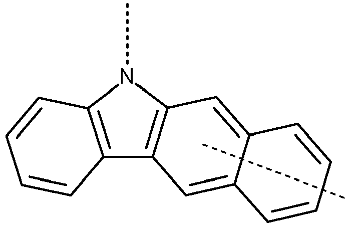 |
| Formel (Cbz-11) | Formel (Cbz-12) |
| 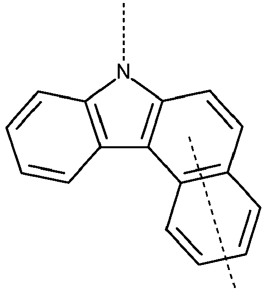 | 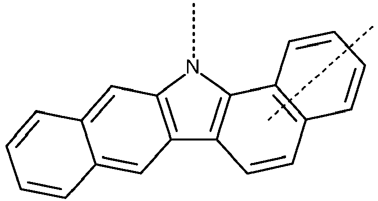 |
| Formel (Cbz-13) | Formel (Cbz-14) |
| 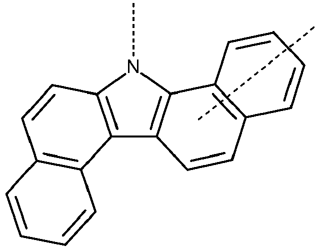 | 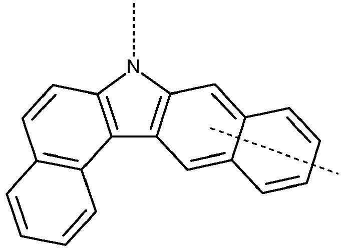 |
| Formel (Cbz-15) | Formel (Cbz-16) |
| 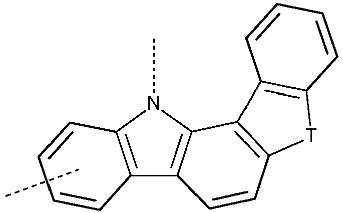 | 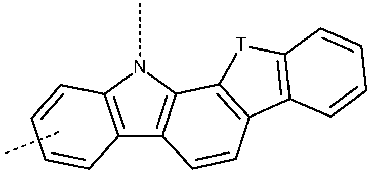 |
| Formel (Cbz-17) | Formel (Cbz-18) |
| 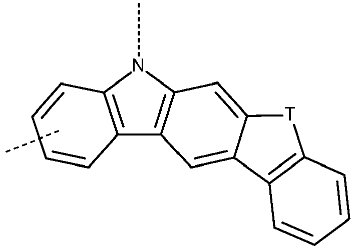 | 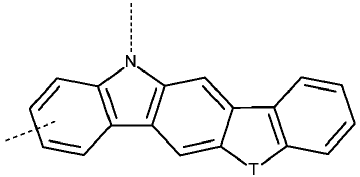 |
| Formel (Cbz-19) | Formel (Cbz-20) |
| 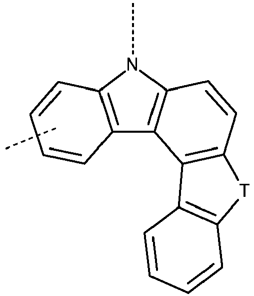 | 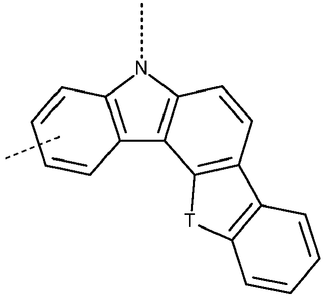 |
| Formel (Cbz-21) | Formel (Cbz-22) |
| 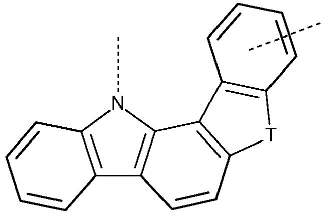 | 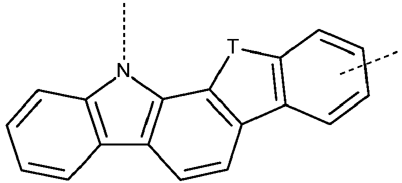 |
| Formel (Cbz-23) | Formel (Cbz-24) |
| 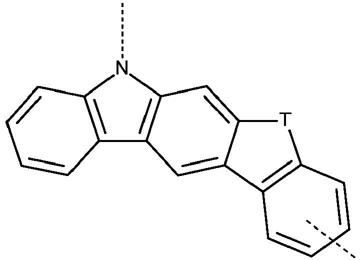 | 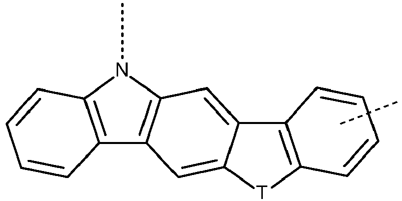 |
| Formel (Cbz-25) | Formel (Cbz-26) |
| 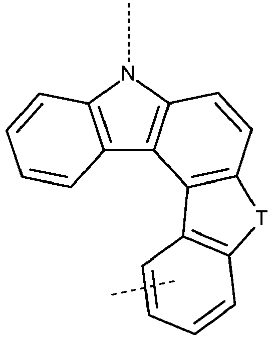 | 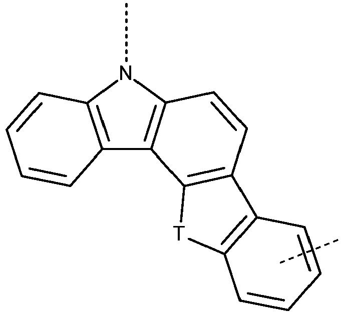 |
| Formel (Cbz-27) | Formel (Cbz-28) |
| 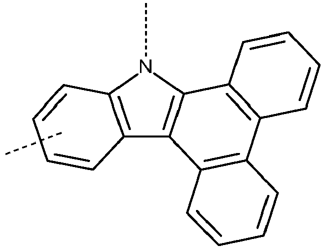 | |
| Formel (Cbz-29) | |
wobei gilt:
T ist gleich C(R³)₂, O, S oder NR³;
wobei die gestrichelten Bindungen die Bindungen an den Rest der Verbindung darstellen, und
wobei die Gruppen der oben genannten Formeln an allen unsubstituiert gezeichneten Positionen jeweils mit einem Rest R³ substituiert sein können.

5. Verbindung nach einem oder mehreren der Ansprüche 1 bis 4, **dadurch gekennzeichnet, dass** Y bei jedem Auftreten gleich oder verschieden gewählt ist aus O und S.

6. Verbindung nach einem oder mehreren der Ansprüche 1 bis 5, **dadurch gekennzeichnet, dass** Z¹ gleich CR¹ oder C ist, wobei eine Gruppe Z¹ genau dann gleich C ist, wenn eine Gruppe Y daran gebunden ist.

7. Verbindung nach einem oder mehreren der Ansprüche 1 bis 6, **dadurch gekennzeichnet, dass** Ar¹ bei jedem Auftreten gleich oder verschieden eine divalente Gruppe ist, die abgeleitet ist von den Grundkörpern Benzol, Biphenyl, Terphenyl, Naphthalin, Dibenzofuran, Dibenzothiophen, Carbazol, und Fluoren, wobei die divalente Gruppe mit einem oder mehreren Resten R² substituiert sein kann.

8. Verbindung nach einem oder mehreren der Ansprüche 1 bis 7, **dadurch gekennzeichnet, dass** R¹, R², R³ und R⁴ bei jedem Auftreten gleich oder verschieden gewählt sind aus H, D, F, CN, Si(R⁵)₃, N(R⁵)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁵ substituiert sein können; und wobei in den genannten Alkyl- oder Alkoxygruppen eine oder mehrere CH₂-Gruppen durch -C≡C-, -R⁵C=CR⁵-, Si(R⁵)₂, C=O, C=NR⁵, - NR⁵-, -O-, -S-, -C(=O)O- oder -C(=O)NR⁵- ersetzt sein können.

9. Verbindung nach einem oder mehreren der Ansprüche 1 bis 8, **dadurch gekennzeichnet, dass** R⁵ bei jedem Auftreten gleich oder verschieden gewählt ist aus H, D, F, CN, Si(R⁶)₃, N(R⁶)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁶ substituiert sein können; und wobei in den genannten Alkyl- oder Alkoxygruppen eine oder mehrere CH₂-Gruppen durch -C≡C-, -R⁶C=CR⁶-, Si(R⁶)₂, C=O, C=NR⁶, -NR⁶-, -O-, -S-, - C(=O)O- oder -C(=O)NR⁶- ersetzt sein können.

10. Verbindung nach einem oder mehreren der Ansprüche 1 bis 9, **dadurch gekennzeichnet, dass** R⁶ gleich H ist.

11. Verbindung nach einem oder mehreren der Ansprüche 1 bis 10, **dadurch gekennzeichnet, dass** m und n bei jedem Auftreten gleich 0 sind.

12. Verbindung nach einem oder mehreren der Ansprüche 1 bis 11, **dadurch gekennzeichnet, dass** Formel (I) einer der folgenden Formeln entspricht
| |
|---|
| 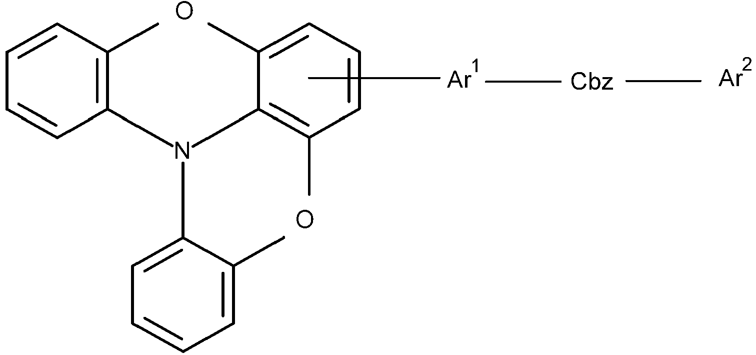 |
| Formel (I-5) |
| 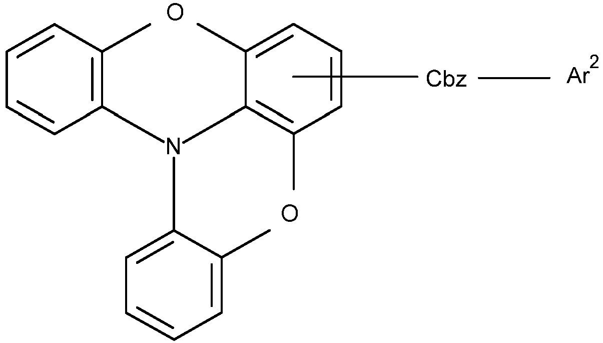 |
| Formel (I-6) |
| 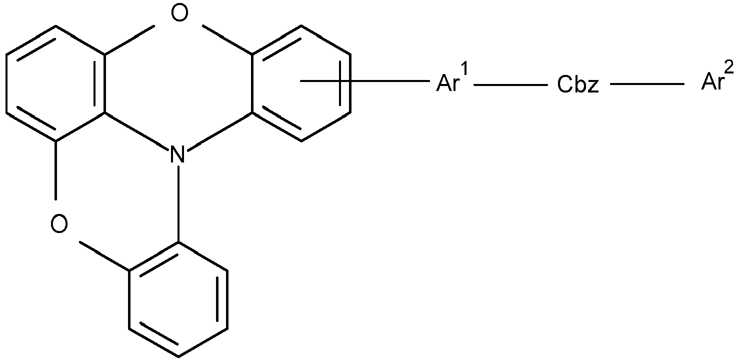 |
| Formel (I-7) |
| 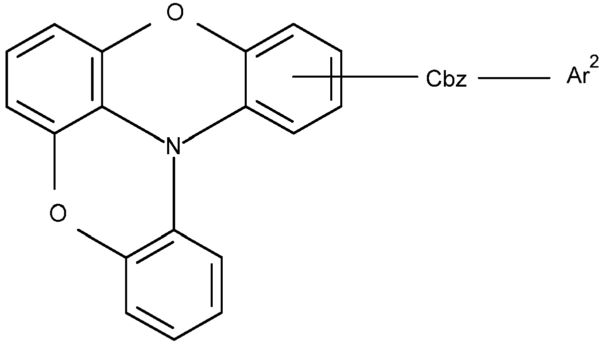 |
| Formel (I-8) |
| 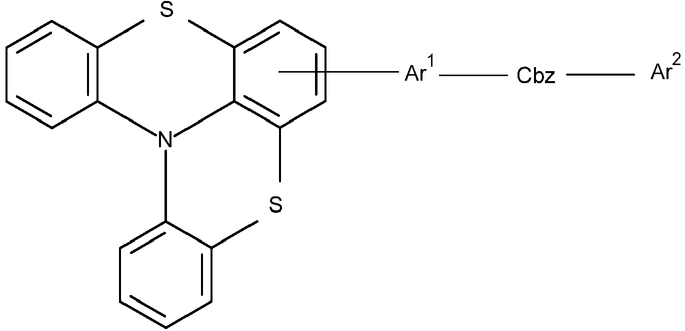 |
| Formel (I-9) |
| 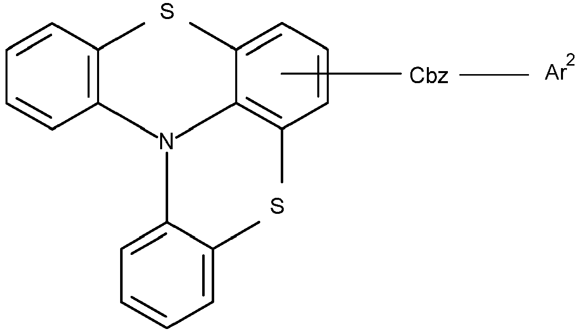 |
| Formel (I-10) |
| 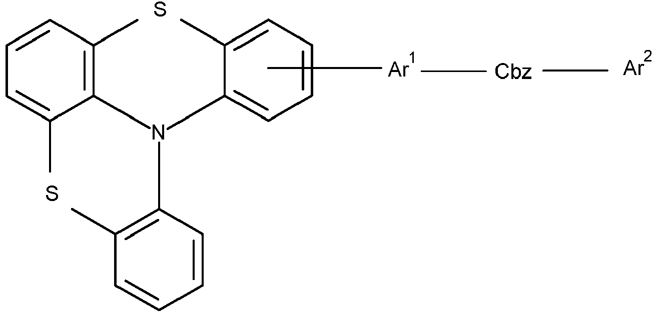 |
| Formel (I-11) |
| 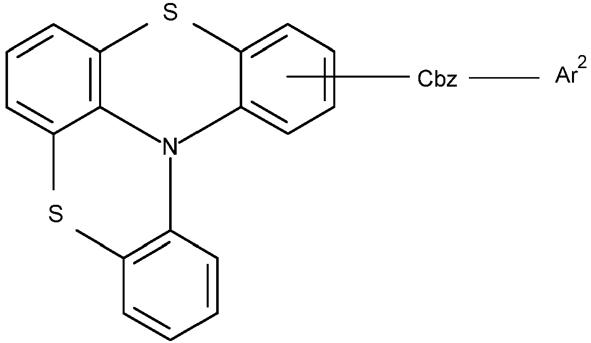 |
| Formel (I-12) |
| 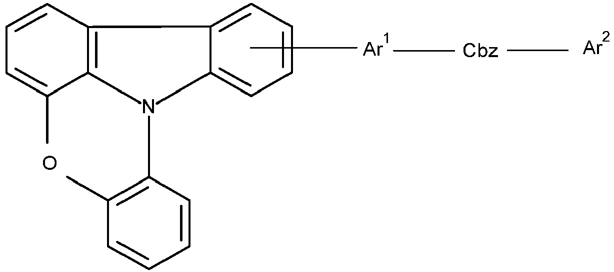 |
| Formel (I-13) |
| 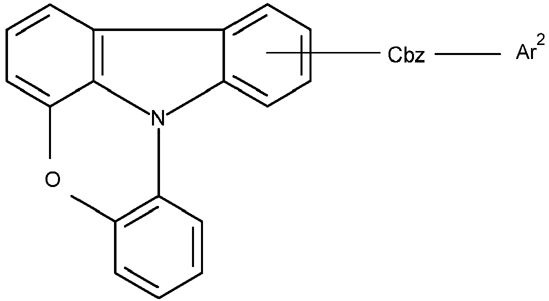 |
| Formel (I-14) |
| 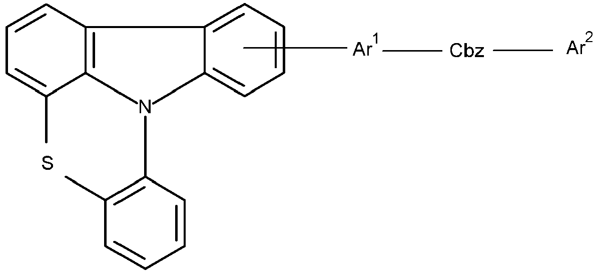 |
| Formel (I-15) |
| 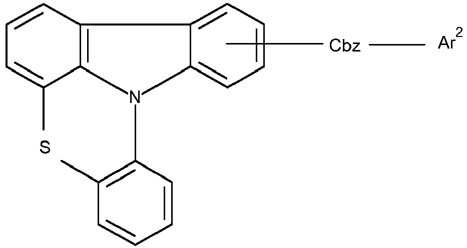 |
| Formel (I-16) |
| 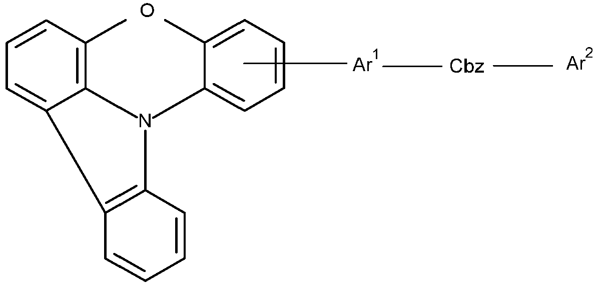 |
| Formel (I-17) |
| 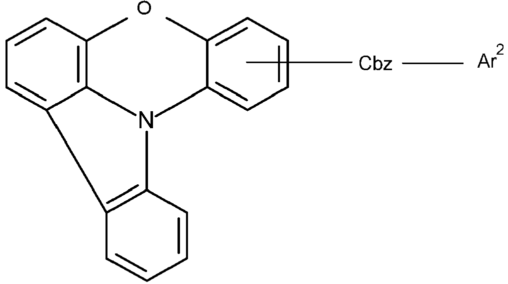 |
| Formel (I-18) |
| 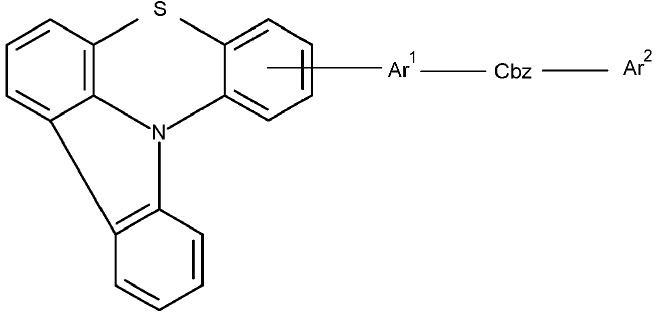 |
| Formel (I-19) |
| 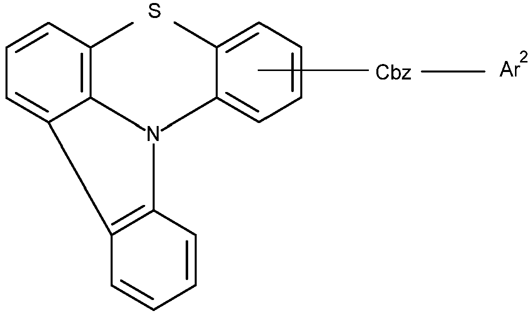 |
| Formel (I-20) |
| 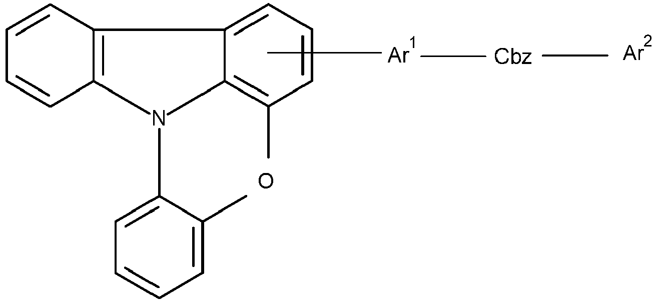 |
| Formel (I-21) |
| 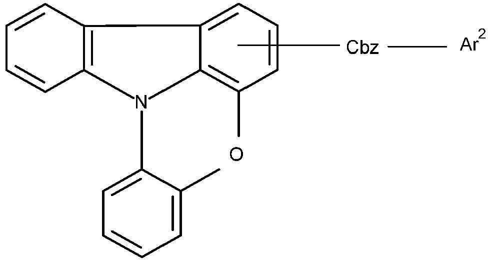 |
| Formel (I-22) |
| 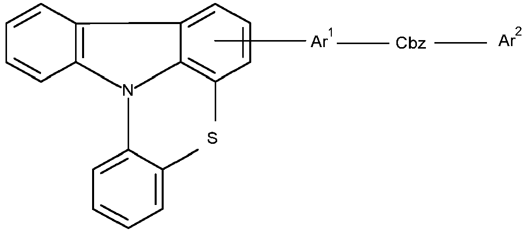 |
| Formel (I-23) |
| 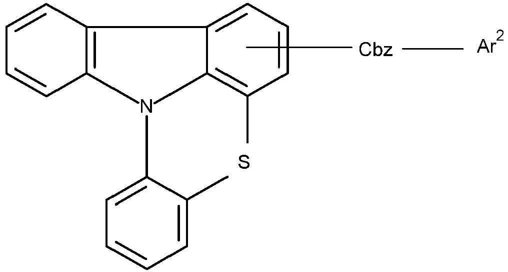 |
| Formel (I-24) |
wobei die auftretenden Variablen wie in einem oder mehreren der Ansprüche 1 bis 11 definiert sind, und die Formeln an den unsubstituiert gezeichneten Positionen an den aromatischen Sechsringen mit je einer Gruppe R¹ substituiert sein können.

13. Verfahren zur Herstellung einer Verbindung gemäß einem oder mehreren der Ansprüche 1 bis 12, **dadurch gekennzeichnet, dass** in einem ersten Schritt eine mit einer reaktiven Gruppe an einer der Phenylgruppen substituierte Triphenylaminverbindung hergestellt wird, wobei die Brücken zwischen den Phenylgruppen gewählt sind aus Einfachbindung, O und S, und wobei mindestens 2 Brücken vorliegen, und dass in einem weiteren Schritt über eine Übergangsmetall-katalysierte Kupplungsreaktion eine Carbazolgruppe in die Verbindung eingeführt wird.

14. Oligomer, Polymer oder Dendrimer, enthaltend eine oder mehrere Verbindungen gemäß Formel (I) nach einem oder mehreren der Ansprüche 1 bis 12, wobei die Bindung(en) zum Polymer, Oligomer oder Dendrimer an beliebigen, in Formel (I) mit R¹, R², R³, oder R⁴ substituierten Positionen lokalisiert sein können.

15. Formulierung, enthaltend mindestens eine Verbindung nach einem oder mehreren der Ansprüche 1 bis 12 oder ein Polymer, Oligomer oder Dendrimer nach Anspruch 14, sowie mindestens ein Lösungsmittel.

16. Elektronische Vorrichtung, enthaltend mindestens eine Verbindung nach einem oder mehreren der Ansprüche 1 bis 12, oder ein Polymer, Oligomer oder Dendrimer nach Anspruch 14.

17. Elektronische Vorrichtung nach Anspruch 16, **dadurch gekennzeichnet, dass** sie eine organische Elektrolumineszenzvorrichtung ist, enthaltend Anode, Kathode und mindestens eine emittierende Schicht, wobei mindestens eine organische Schicht der Vorrichtung, die eine emittierende Schicht, eine Elektronentransportschicht oder eine Lochblockierschicht ist, die mindestens eine Verbindung enthält.

18. Verwendung einer Verbindung nach einem oder mehreren der Ansprüche 1 bis 12 in einer elektronischen Vorrichtung.

## Claims

1. Compound of the formula (I) 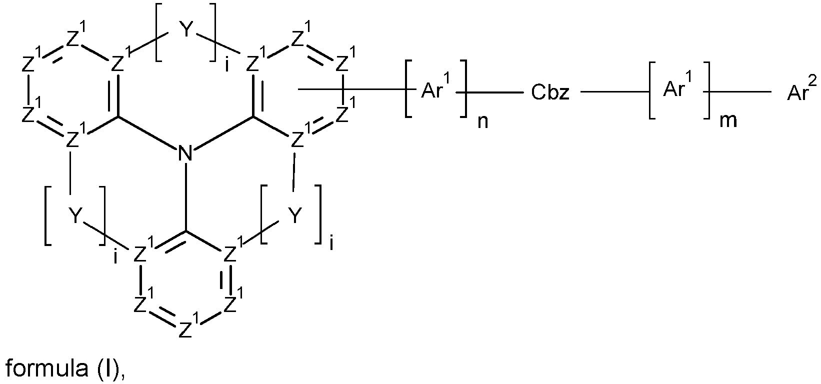 where the following applies to the variables occurring:
Y is selected on each occurrence, identically or differently, from a single bond, O and S, where at least one group Y selected from O and S is present;
Z¹ is on each occurrence, identically or differently, CR¹, N or C, where a group Z¹ is equal to C precisely if a group Y is bonded thereto;
Ar¹ is on each occurrence, identically or differently, an aromatic ring system having 6 to 24 aromatic ring atoms, which may be substituted by one or more radicals R², or a heteroaromatic ring system having 5 to 24 aromatic ring atoms, which may be substituted by one or more radicals R²;
Cbz is a divalent group which is substituted by a radical R³ at each of its free positions and which contains one or more structural elements of the formula (Cbz) 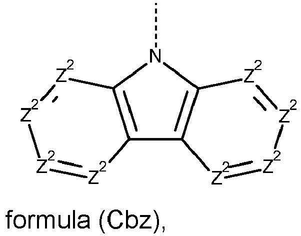
where one of the two bonds from the divalent group to the remainder of the compound is the dashed bond on the nitrogen atom of the formula (Cbz),
where the second of these two bonds may be arranged at any desired free position of the group, and where
Z² is selected on each occurrence, identically or differently, from C and N;
Ar² is selected from pyridine, pyrimidine, pyridazine, pyrazine, triazine, benzimidazole, quinoline, quinazoline, benzo[h]quinazoline, phenanthroline, phenanthridine, diazaphenanthrene and acridine, each of which may be substituted by one or more radicals R⁴;
R¹, R², R³, R⁴ are selected on each occurrence, identically or differently, from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ or R² or R³ or R⁴ may be linked to one another and may form a ring; where the said alkyl, alkoxy, alkenyl and alkynyl groups and the said aromatic ring systems and heteroaromatic ring systems may in each case be substituted by one or more radicals R⁵; and where one or more CH₂ groups in the said alkyl, alkoxy, alkenyl and alkynyl groups may be replaced by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂;
R⁵ is selected on each occurrence, identically or differently, from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁵ may be linked to one another and may form a ring; where the said alkyl, alkoxy, alkenyl and alkynyl groups and the said aromatic ring systems and heteroaromatic ring systems may in each case be substituted by one or more radicals R⁶; and where one or more CH₂ groups in the said alkyl, alkoxy, alkenyl and alkynyl groups may be replaced by -R⁶C=CR⁶, -C=C, Si(R⁶)₂, C=O, C=NR⁶, -C(=O)O-, -C(=O)NR⁶-, NR⁶, P(=O)(R⁶), -O-, -S-, SO or SO₂;
R⁶ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁶ may be linked to one another and may form a ring; and where the said alkyl, alkoxy, alkenyl and alkynyl groups, aromatic ring systems and heteroaromatic ring systems may be substituted by F or CN;
i is on each occurrence, identically or differently, 0 or 1, where two of the three indices i in formula (I) are equal to 1, and one of the three indices i is equal to 0, and where the group Y in question is not present if the associated index i = 0;
n is equal to 0, 1, 2, 3 or 4;
m is equal to 0, 1, 2, 3 or 4.

2. Compound according to Claim 1, **characterised in that** Ar² is selected from triazine and quinazoline, each of which may be substituted by one or more radicals R⁴.

3. Compound according to Claim 1 or 2, **characterised in that** the group Cbz is selected on each occurrence, identically or differently, from carbazole, azacarbazole, benzocarbazole, dibenzocarbazole, indenocarbazole, indolocarbazole, carbazole with annelated benzofuran and carbazole with annelated benzothiophene, where the said groups may in each case be substituted by one or more radicals R³.

4. Compound according to one or more of Claims 1 to 3, **characterised in that** the group Cbz is selected from the formulae
| | |
|---|---|
| 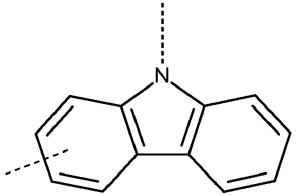 | 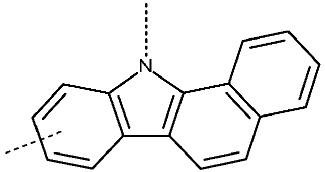 |
| formula (Cbz-1) | formula (Cbz-2) |
| 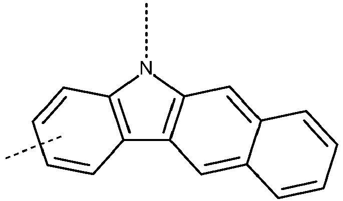 | 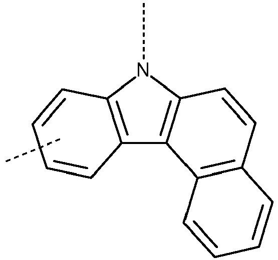 |
| formula (Cbz-3) | formula (Cbz-4) |
| 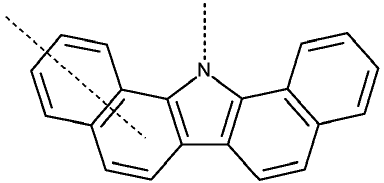 | 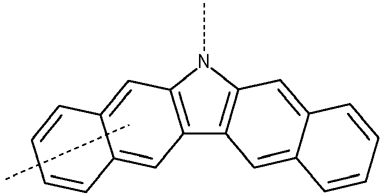 |
| formula (Cbz-5) | formula (Cbz-6) |
| 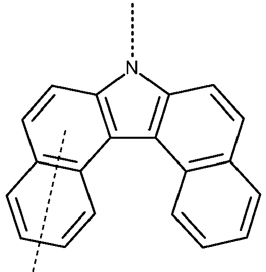 | 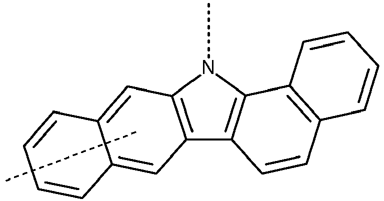 |
| formula (Cbz-7) | formula (Cbz-8) |
| 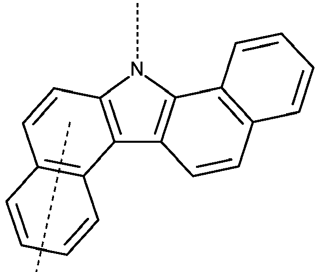 | 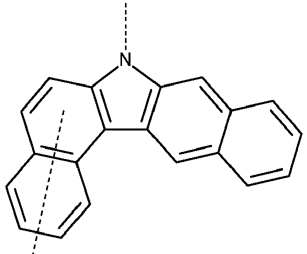 |
| formula (Cbz-9) | formula (Cbz-10) |
| 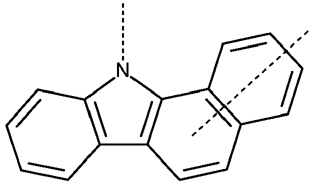 | 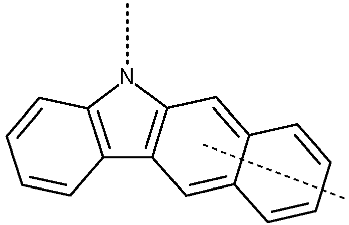 |
| formula (Cbz-11) | formula (Cbz-12) |
| 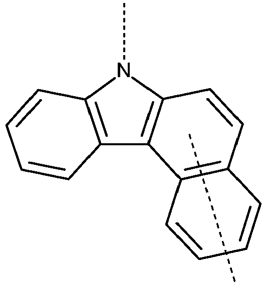 | 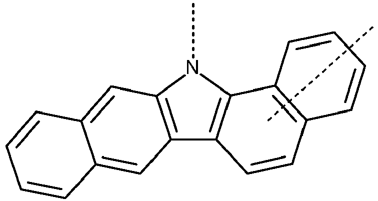 |
| formula (Cbz-13) | formula (Cbz-14) |
| 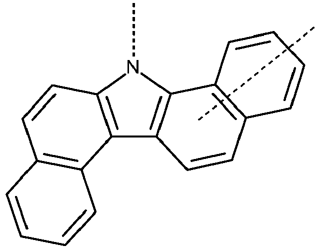 | 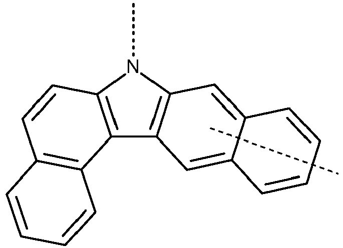 |
| formula (Cbz-15) | formula (Cbz-16) |
| 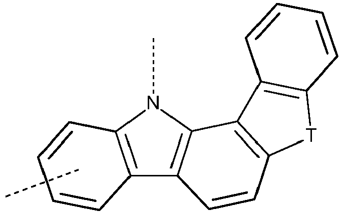 | 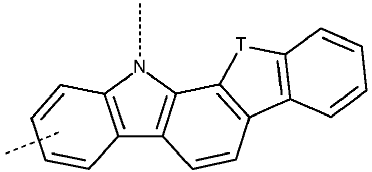 |
| formula (Cbz-17) | formula (Cbz-18) |
| 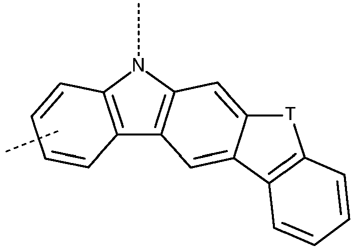 | 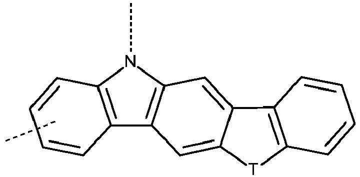 |
| formula (Cbz-19) | formula (Cbz-20) |
| 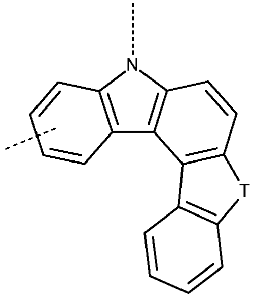 | 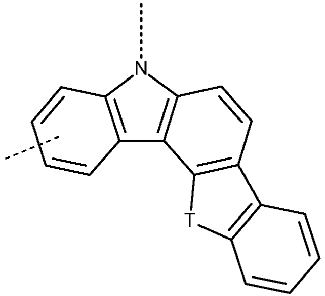 |
| formula (Cbz-21) | formula (Cbz-22) |
| 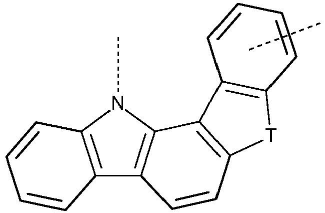 | 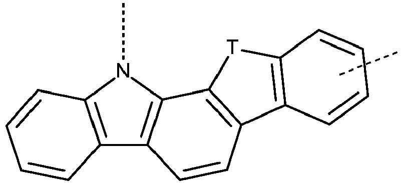 |
| formula (Cbz-23) | formula (Cbz-24) |
| 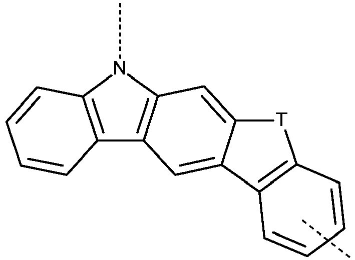 | 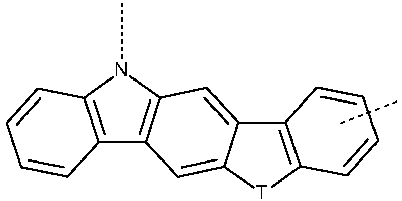 |
| formula (Cbz-25) | formula (Cbz-26) |
| 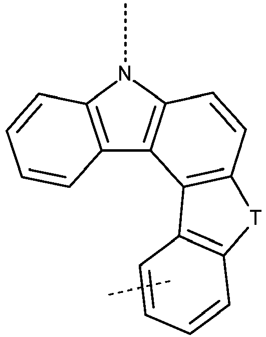 | 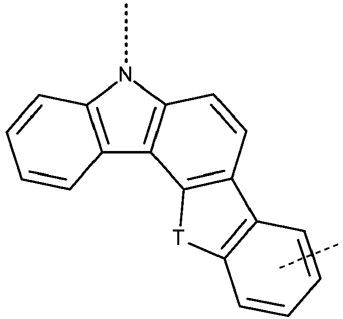 |
| formula (Cbz-27) | formula (Cbz-28) |
| 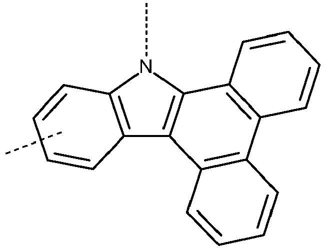 | |
| formula (Cbz-29) | |
where the following applies:
T is equal to C(R³)₂, O, S or NR³;
where the dashed bonds represent the bonds to the remainder of the compound, and
where the groups of the above-mentioned formulae may in each case be substituted by a radical R³ at all positions drawn as unsubstituted.

5. Compound according to one or more of Claims 1 to 4, **characterised in that** Y is selected on each occurrence, identically or differently, from O and S.

6. Compound according to one or more of Claims 1 to 5, **characterised in that** Z¹ is equal to CR¹ or C, where a group Z¹ is equal to C precisely if a group Y is bonded thereto.

7. Compound according to one or more of Claims 1 to 6, **characterised in that** Ar¹ is on each occurrence, identically or differently, a divalent group derived from the parent structures benzene, biphenyl, terphenyl, naphthalene, dibenzofuran, dibenzothiophene, carbazole and fluorene, where the divalent group may be substituted by one or more radicals R².

8. Compound according to one or more of Claims 1 to 7, **characterised in that** R¹, R², R³ and R⁴ are selected on each occurrence, identically or differently, from H, D, F, CN, Si(R⁵)₃, N(R⁵)₂, straight-chain alkyl or alkoxy groups having 1 to 20 C atoms, branched or cyclic alkyl or alkoxy groups having 3 to 20 C atoms, aromatic ring systems having 6 to 40 aromatic ring atoms, and heteroaromatic ring systems having 5 to 40 aromatic ring atoms; where the said alkyl and alkoxy groups, the said aromatic ring systems and the said heteroaromatic ring systems may in each case be substituted by one or more radicals R⁵; and where one or more CH₂ groups in the said alkyl or alkoxy groups may be replaced by -C=C-, -R⁵C=CR⁵-, Si(R⁵)₂, C=O, C=NR⁵, -NR⁵-, -O-, -S-, -C(=O)O- or -C(=O)NR⁵-.

9. Compound according to one or more of Claims 1 to 8, **characterised in that** R⁵ is selected on each occurrence, identically or differently, from H, D, F, CN, Si(R⁶)₃, N(R⁶)₂, straight-chain alkyl or alkoxy groups having 1 to 20 C atoms, branched or cyclic alkyl or alkoxy groups having 3 to 20 C atoms, aromatic ring systems having 6 to 40 aromatic ring atoms, and heteroaromatic ring systems having 5 to 40 aromatic ring atoms; where the said alkyl and alkoxy groups, the said aromatic ring systems and the said heteroaromatic ring systems may in each case be substituted by one or more radicals R⁶; and where one or more CH₂ groups in the said alkyl or alkoxy groups may be replaced by -C≡C-, -R⁶C=CR⁶-, Si(R⁶)₂, C=O, C=NR⁶, -NR⁶-, -O-, -S-, -C(=O)O- or -C(=O)NR⁶-.

10. Compound according to one or more of Claims 1 to 9, **characterised in that** R⁶ is equal to H.

11. Compound according to one or more of Claims 1 to 10, **characterised in that** m and n are on each occurrence equal to 0.

12. Compound according to one or more of Claims 1 to 11, **characterised in that** formula (I) corresponds to one of the following formulae
| |
|---|
| 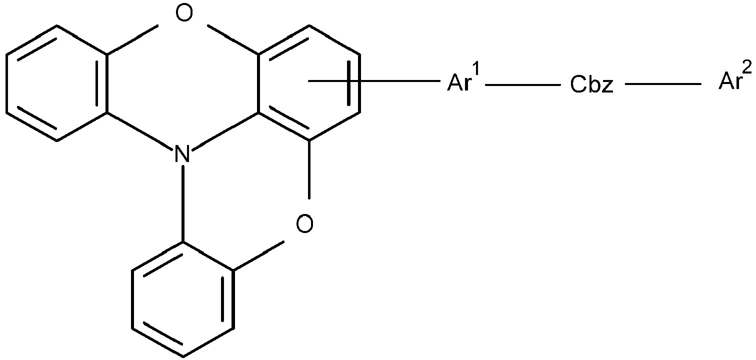 |
| formula (I-5) |
| 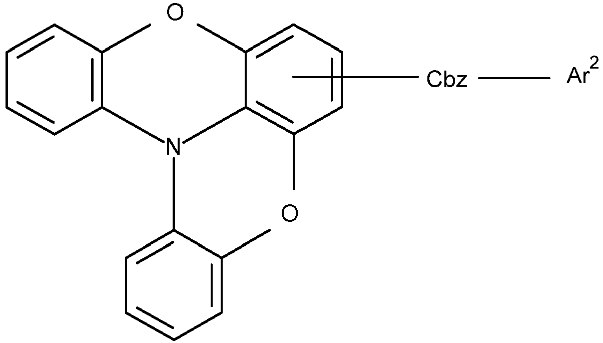 |
| formula (I-6) |
| 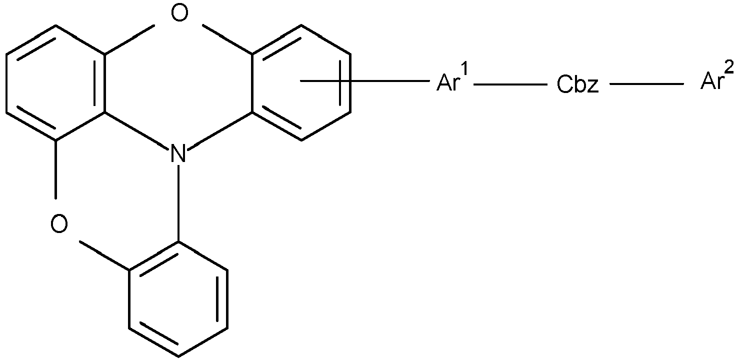 |
| formula (I-7) |
| 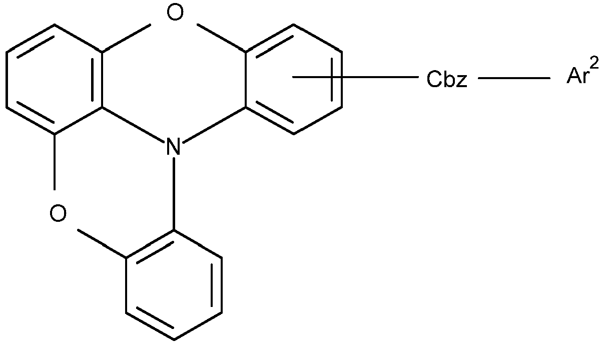 |
| formula (I-8) |
| 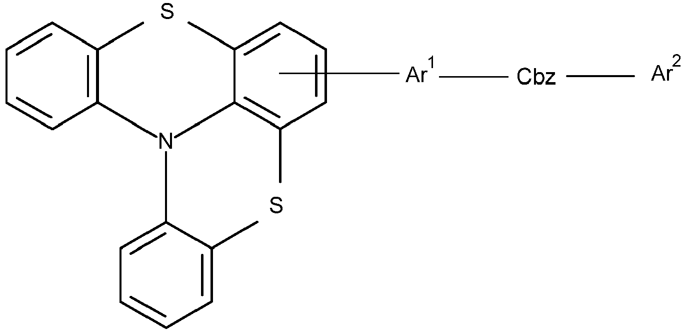 |
| formula (I-9) |
| 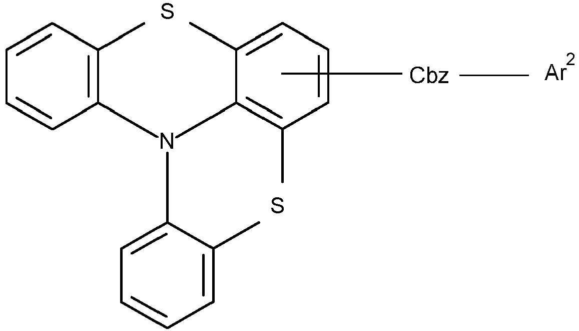 |
| formula (I-10) |
| 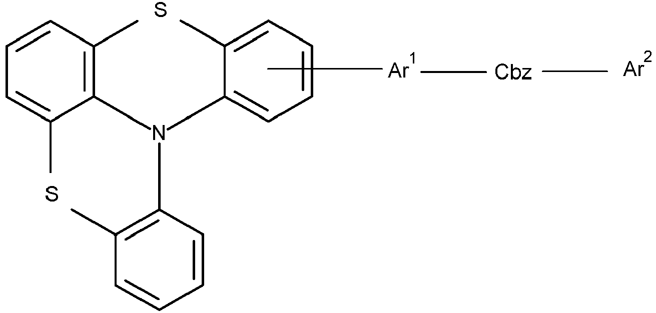 |
| formula (I-11) |
| 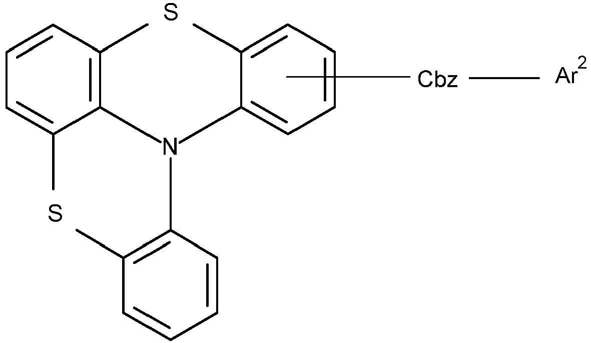 |
| formula (I-12) |
| 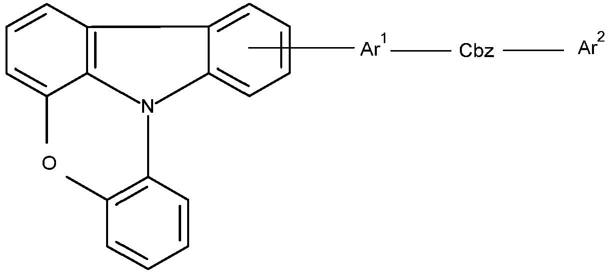 |
| formula (I-13) |
| 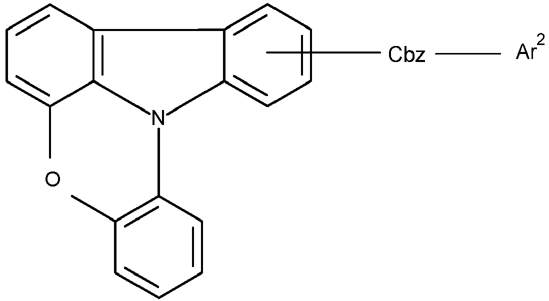 |
| formula (I-14) |
| 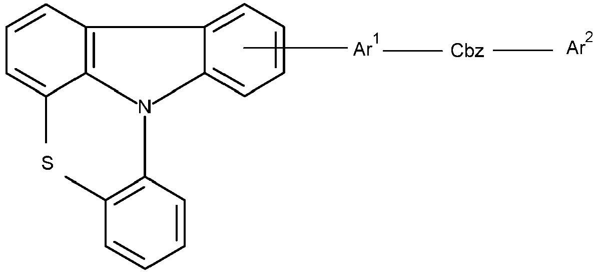 |
| formula (I-15) |
| 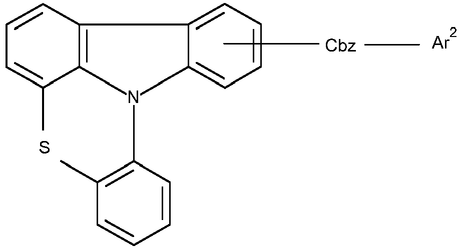 |
| formula (I-16) |
| 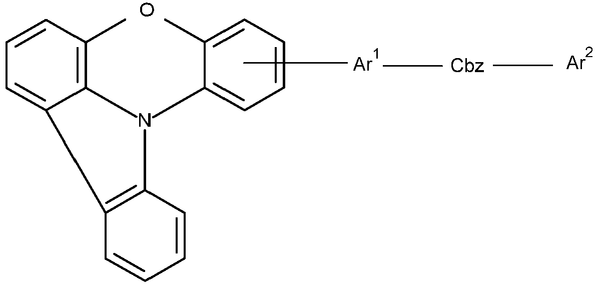 |
| formula (I-17) |
| 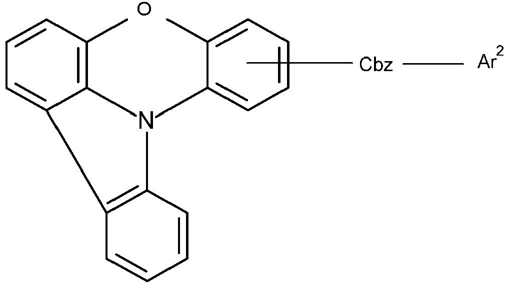 |
| formula (I-18) |
| 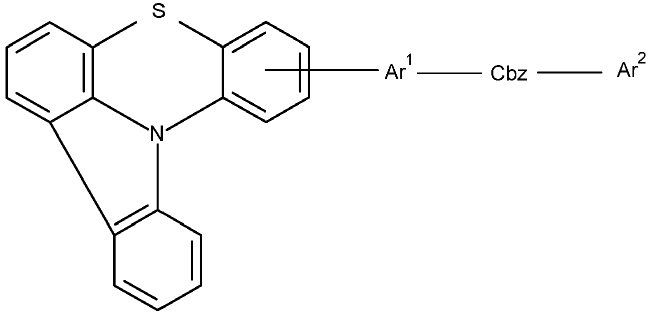 |
| formula (I-19) |
| 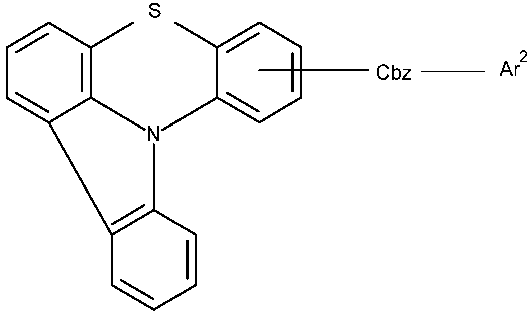 |
| formula (I-20) |
| 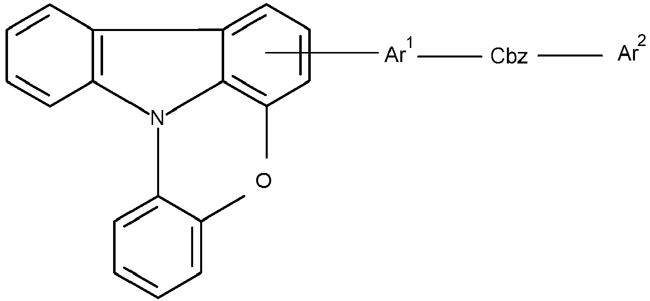 |
| formula (I-21) |
| 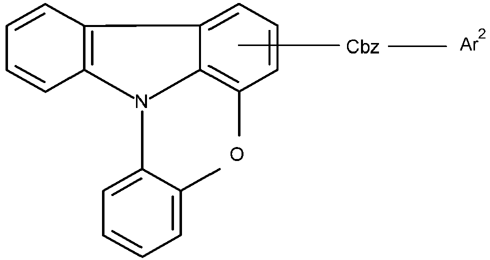 |
| formula (I-22) |
| 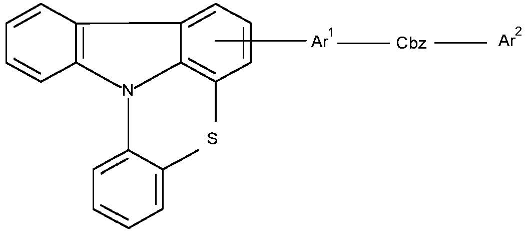 |
| formula (I-23) |
| 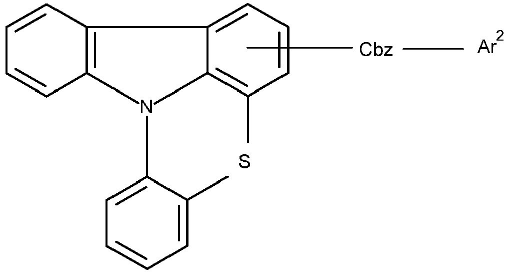 |
| formula (I-24) |
where the variables occurring are defined as in one or more of Claims 1 to 11, and the formulae may be substituted by a group R¹ at each of the positions drawn as unsubstituted on the aromatic six-membered rings.

13. Process for the preparation of a compound according to one or more of Claims 1 to 12, **characterised in that**, in a first step, a triphenylamine compound which is substituted by a reactive group on one of the phenyl groups is prepared, where the bridges between the phenyl groups are selected from a single bond, O and S, and where at least 2 bridges are present, and **in that**, in a further step, a carbazole group is introduced into the compound via a transition metal-catalysed coupling reaction.

14. Oligomer, polymer or dendrimer containing one or more compounds of the formula (I) according to one or more of Claims 1 to 12, where die bond(s) to the polymer, oligomer or dendrimer may be localised at any desired positions in formula (I) that are substituted by R¹, R², R³ or R⁴.

15. Formulation comprising at least one compound according to one or more of Claims 1 to 12 or a polymer, oligomer or dendrimer according to Claim 14, and at least one solvent.

16. Electronic device containing at least one compound according to one or more of Claims 1 to 12, or a polymer, oligomer or dendrimer according to Claim 14.

17. Electronic device according to Claim 16, **characterised in that** it is an organic electroluminescent device comprising anode, cathode and at least one emitting layer, where at least one organic layer of the device that is an emitting layer, an electron-transport layer or a hole-blocking layer comprises the at least one compound.

18. Use of a compound according to one or more of Claims 1 to 12 in an electronic device.

## Revendications

1. Composé de formule (I) 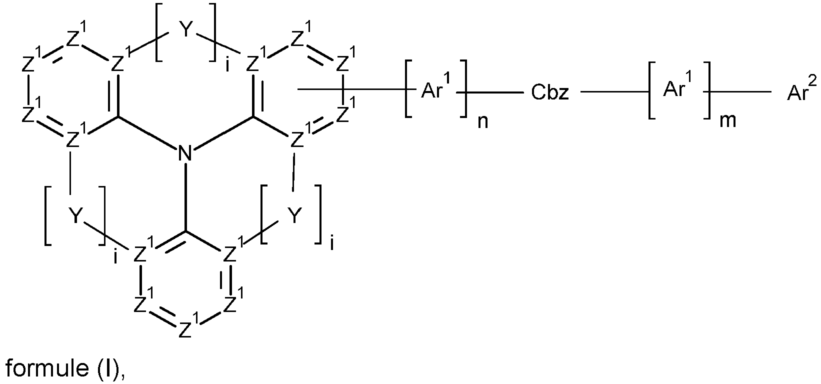 dan laquelle ce qui suit s'applique aux variables présentes :
Y est choisi à chaque occurrence, de manière identique ou différente, parmi une liaison simple, O et S, où au moins un groupement Y choisi parmi O et S est présent ;
Z¹ est à chaque occurrence, de manière identique ou différente, CR¹, N ou C, où un groupement Z¹ est égal à C précisément si un groupement Y est lié à celui-ci ;
Ar¹ est à chaque occurrence, de manière identique ou différente, un noyau aromatique ayant de 6 à 24 atomes de cycle aromatique, pouvant être substitué par un ou plusieurs radicaux R², ou un noyau hétéroaromatique ayant de 5 à 24 atomes de cycle aromatique, pouvant être substitué par un ou plusieurs radicaux R² ;
Cbz est un groupement divalent qui est substitué par un radical R³ au niveau de chacune de ses positions libres et qui contient un ou plusieurs éléments structurels de formule (Cbz) 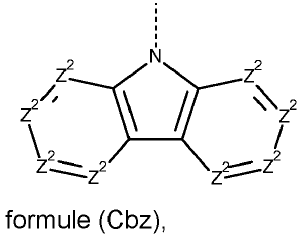
dans laquelle l'une des deux liaisons du groupement divalent au reste du composé est la liaison en pointillés sur l'atome d'azote de la formule (Cbz),
où la deuxième parmi ces deux liaisons peut être disposée au niveau d'une quelconque position libre désirée du groupement, et où
Z² est choisi à chaque occurrence, de manière identique ou différente, parmi C et N ;
Ar² est choisi parmi pyridine, pyrimidine, pyridazine, pyrazine, triazine, benzimidazole, quinoléine, quinazoline, benzo[h]quinazoline, phénanthroline, phénanthridine, diazaphénanthrène et acridine, chacun d'entre eux pouvant être substitué par un ou plusieurs radicaux R4 ;
R¹, R², R³, R⁴ sont choisis à chaque occurrence, de manière identique ou différente, parmi H, D, F, C(=O)R⁵, CN, Si(R⁵)₃, N(R⁵)₂, P(=O)(R⁵)₂, OR⁵, S(=O)R⁵, S(=O)₂R⁵, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹ ou R² ou R³ ou R⁴ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁵ ; et où un ou plusieurs groupements CH₂ dans lesdits groupements alkyle, alcoxy, alcényle et alcynyle peuvent être remplacés par -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO ou SO₂ ;
R⁵ est choisi à chaque occurrence, de manière identique ou différente, parmi H, D, F, C(=O)R⁶, CN, Si(R⁶)₃, N(R⁶)₂, P(=O)(R⁶)₂, OR⁶, S(=O)R⁶, S(=O)₂R⁶, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁵ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁶ ; et où un ou plusieurs groupements CH₂ dans lesdits groupements alkyle, alcoxy, alcényle et alcynyle peuvent être remplacés par -R⁶C=CR⁶, -C=C, Si(R⁶)₂, C=O, C=NR⁶, -C(=O)O-, -C(=O)NR⁶-, NR⁶, P(=O)(R⁶), -O-, -S-, SO ou SO₂ ;
R⁶ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁶ peuvent être liés les uns aux autres et peuvent former un cycle ; et où lesdits groupements alkyle, alcoxy, alcényle et alcynyle, noyaux aromatiques et noyaux hétéroaromatiques peuvent être substitués par F ou CN ;
i vaut à chaque occurrence, de manière identique ou différente, 0 ou 1, où deux parmi les trois indices i dans la formule (I) sont égaux à 1, et l'un parmi les trois indices i est égal à 0, et où le groupement Y en question n'est pas présent si l'indice i associé = 0 ;
n est égal à 0, 1, 2, 3 ou 4 ;
m est égal à 0, 1, 2, 3 ou 4.

2. Composé selon la revendication 1, **caractérisé en ce que** Ar² est choisi parmi triazine et quinazoline, chacun d'entre eux pouvant être substitué par un ou plusieurs radicaux R⁴.

3. Composé selon la revendication 1 ou 2, **caractérisé en ce que** le groupement Cbz est choisi à chaque occurrence, de manière identique ou différente, parmi carbazole, azacarbazole, benzocarbazole, dibenzocarbazole, indénocarbazole, indolocarbazole, carbazole à benzofurane annelé et carbazole à benzothiophène annelé, où lesdits groupements peuvent dans chaque cas être substitués par un ou plusieurs radicaux R³.

4. Composé selon l'une ou plusieurs parmi les revendications 1 à 3, **caractérisé en ce que** le groupement Cbz est choisi parmi les formules
| | |
|---|---|
| 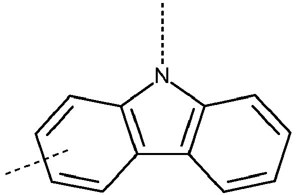 | 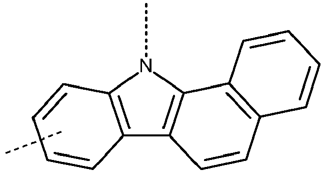 |
| formule (Cbz-1) | formule (Cbz-2) |
| 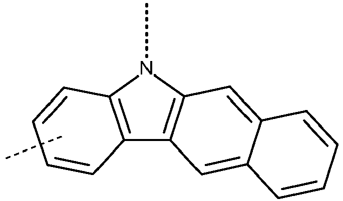 | 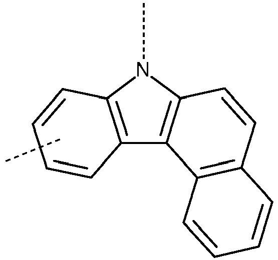 |
| formule (Cbz-3) | formule (Cbz-4) |
| 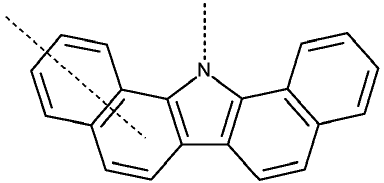 | 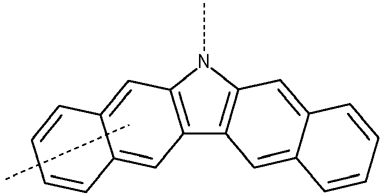 |
| formule (Cbz-5) | formule (Cbz-6) |
| 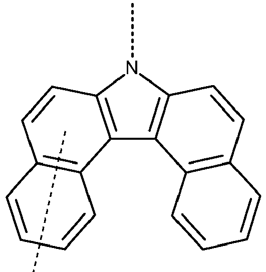 | 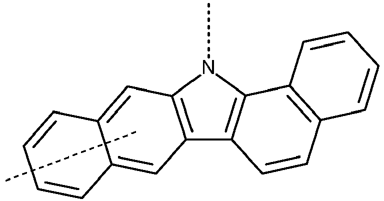 |
| formule (Cbz-7) | formule (Cbz-8) |
| 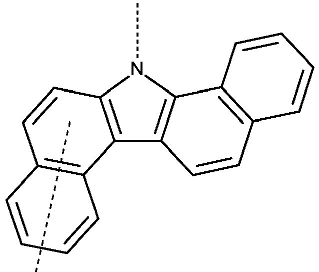 | 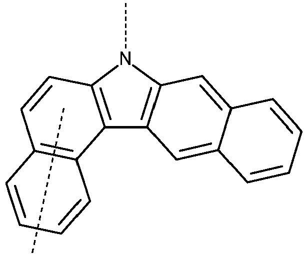 |
| formule (Cbz-9) | formule (Cbz-10) |
| 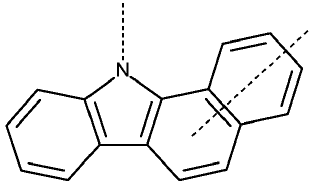 | 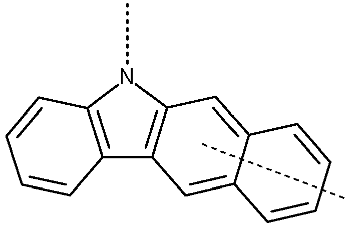 |
| formule (Cbz-11) | formule (Cbz-12) |
| 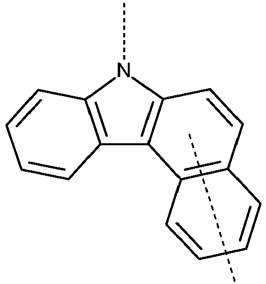 | 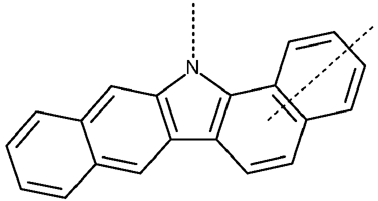 |
| formule (Cbz-13) | formule (Cbz-14) |
| 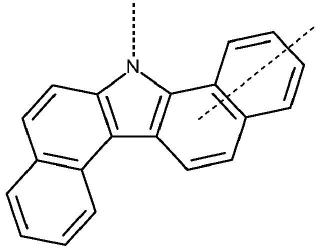 | 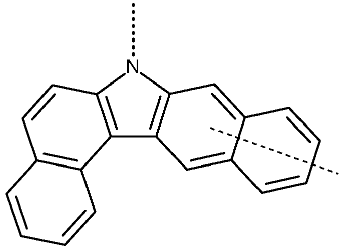 |
| formule (Cbz-15) | formule (Cbz-16) |
| 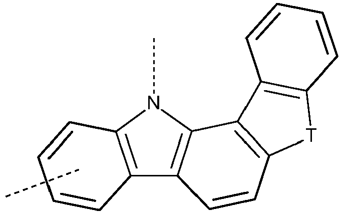 | 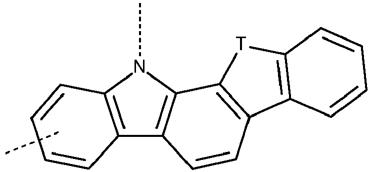 |
| formule (Cbz-17) | formule (Cbz-18) |
| 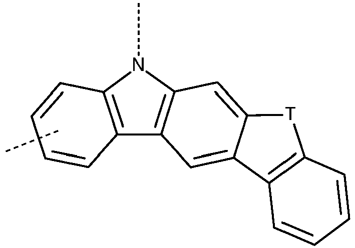 | 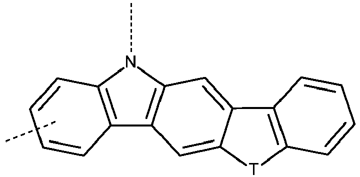 |
| formule (Cbz-19) | formule (Cbz-20) |
| 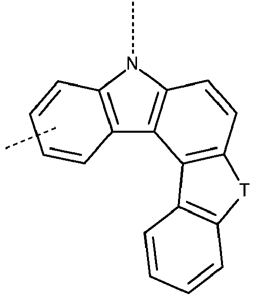 | 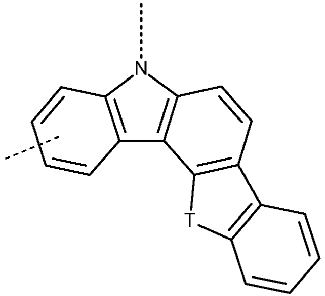 |
| formule (Cbz-21) | formule (Cbz-22) |
| 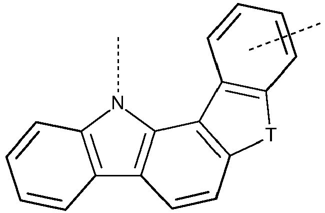 | 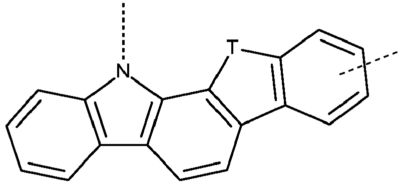 |
| formule (Cbz-23) | formule (Cbz-24) |
| 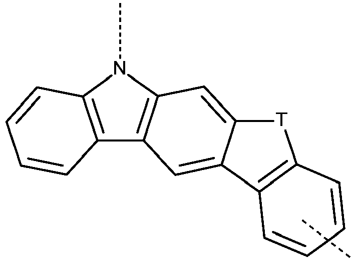 | 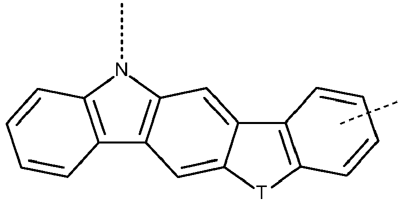 |
| formule (Cbz-25) | formule (Cbz-26) |
| 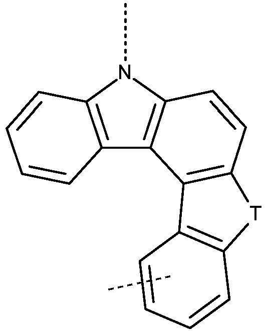 | 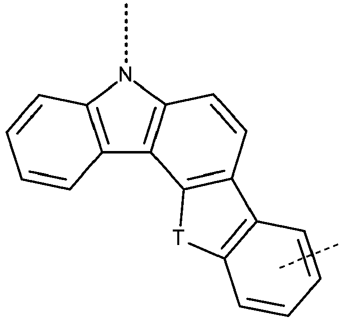 |
| formule (Cbz-27) | formule (Cbz-28) |
| 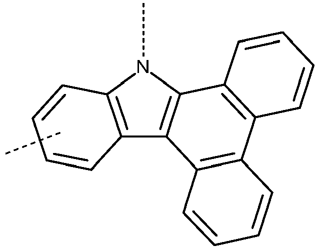 | |
| formule (Cbz-29) | |
où ce qui suit s'applique :
T est égal à C(R³)₂, O, S ou NR³ ;
où les liaisons en pointillés représentent les liaisons au reste du composé, et
où les groupements des formules ci-dessus peuvent dans chaque cas être substitués par un radical R³ au niveau de toutes les positions illustrées comme étant non substituées.

5. Composé selon l'une ou plusieurs parmi les revendications 1 à 4, **caractérisé en ce que** Y est choisi à chaque occurrence, de manière identique ou différente, parmi O et S.

6. Composé selon l'une ou plusieurs parmi les revendications 1 à 5, **caractérisé en ce que** Z¹ est égal à CR¹ ou C, où un groupement Z¹ est égal à C précisément si un groupement Y est lié à celui-ci.

7. Composé selon l'une ou plusieurs parmi les revendications 1 à 6, **caractérisé en ce que** Ar¹ est à chaque occurrence, de manière identique ou différente, un groupement divalent dérivé des structures parentes de benzène, biphényle, terphényle, naphtalène, dibenzofurane, dibenzothiophène, carbazole et fluorène, où le groupement divalent peut être substitué par un ou plusieurs radicaux R².

8. Composé selon l'une ou plusieurs parmi les revendications 1 à 7, **caractérisé en ce que** R¹, R², R³ et R⁴ sont choisis à chaque occurrence, de manière identique ou différente, parmi H, D, F, CN, Si(R⁵)₃, N(R⁵)₂, des groupements alkyle ou alcoxy à chaîne linéaire ayant de 1 à 20 atomes de C, des groupements alkyle ou alcoxy ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle et alcoxy, lesdits noyaux aromatiques et lesdits noyaux hétéroaromatiques peuvent dans chaque cas être substitués par un ou plusieurs radicaux R⁵ ; et où un ou plusieurs groupements CH₂ dans lesdits groupements alkyle ou alcoxy peuvent être remplacés par -C≡C-, -R⁵C=CR⁵-, Si(R⁵)₂, C=O, C=NR⁵, -NR⁵-, -O-, -S-, -C(=O)O- ou -C(=O)NR⁵-.

9. Composé selon l'une ou plusieurs parmi les revendications 1 à 8, **caractérisé en ce que** R⁵ est choisi à chaque occurrence, de manière identique ou différente, parmi H, D, F, CN, Si(R⁶)₃, N(R⁶)₂, des groupements alkyle ou alcoxy à chaîne linéaire ayant de 1 à 20 atomes de C, des groupements alkyle ou alcoxy ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle et alcoxy, lesdits noyaux aromatiques et lesdits noyaux hétéroaromatiques peuvent dans chaque cas être substitués par un ou plusieurs radicaux R⁶ ; et où un ou plusieurs groupements CH₂ dans lesdits groupements alkyle ou alcoxy peuvent être remplacés par -C=C-, -R⁶C=CR⁶-, Si(R⁶)₂, C=O, C=NR⁶, -NR⁶-, -O-, -S-, -C(=O)O- ou -C(=O)NR⁶-.

10. Composé selon l'une ou plusieurs parmi les revendications 1 à 9, **caractérisé en ce que** R⁶ est égal à H.

11. Composé selon l'une ou plusieurs parmi les revendications 1 à 10, **caractérisé en ce que** m et n sont à chaque occurrence égaux à 0.

12. Composé selon l'une ou plusieurs parmi les revendications 1 à 11, **caractérisé en ce que** la formule (I) correspond à l'une des formules suivantes
| |
|---|
| 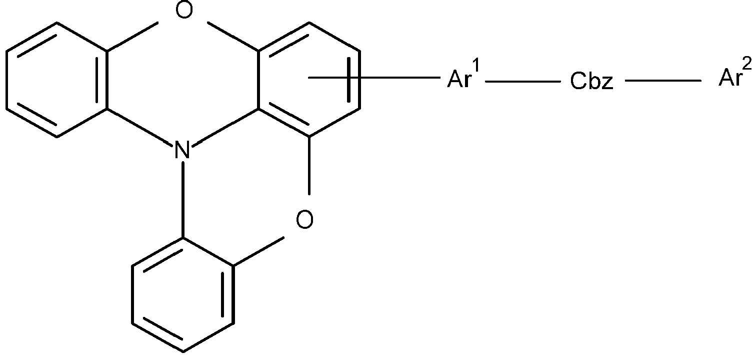 |
| formule (I-5) |
| 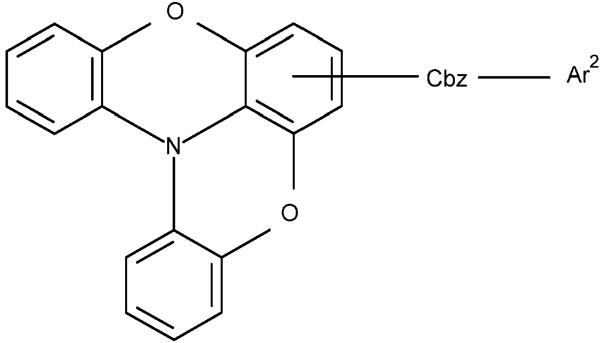 |
| formule (I-6) |
| 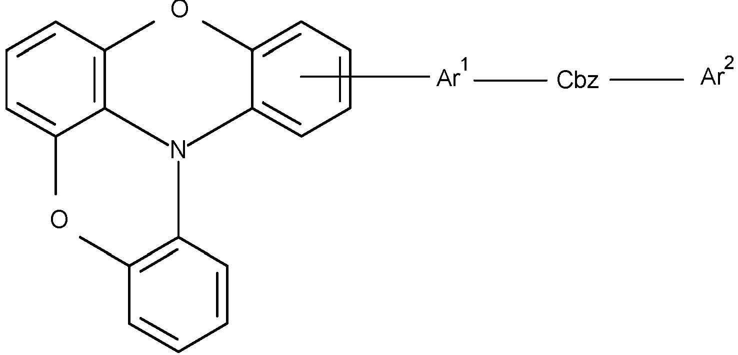 |
| formule (I-7) |
| 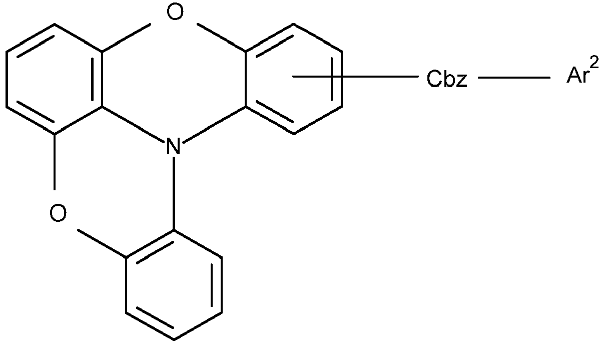 |
| formule (I-8) |
| 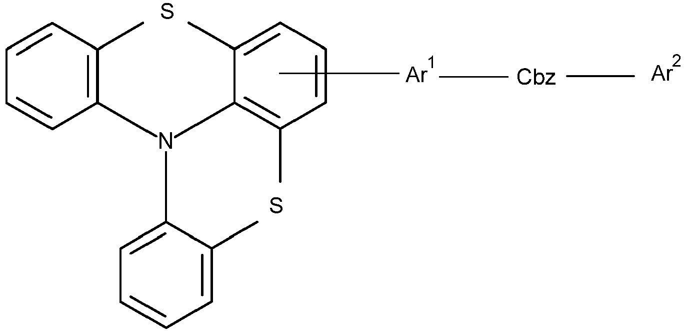 |
| formule (I-9) |
| 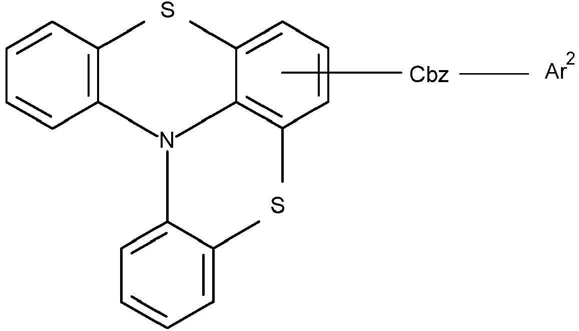 |
| formule (I-10) |
| 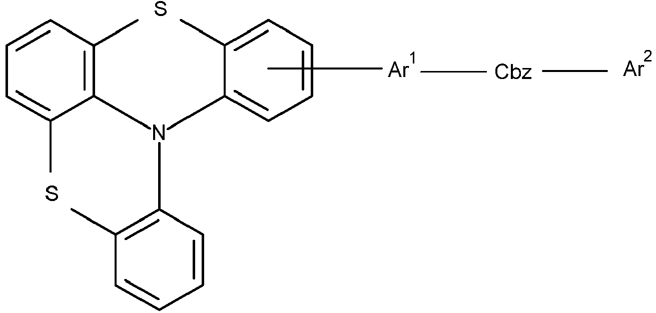 |
| formule (I-11) |
| 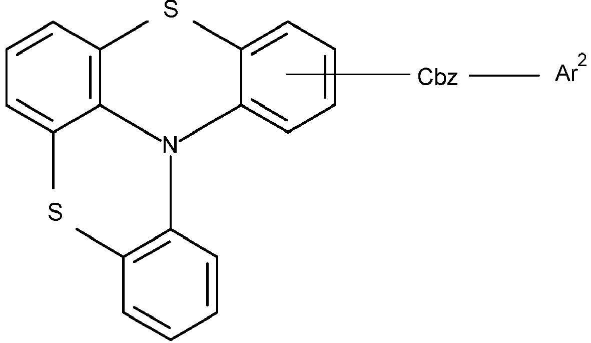 |
| formule (I-12) |
| 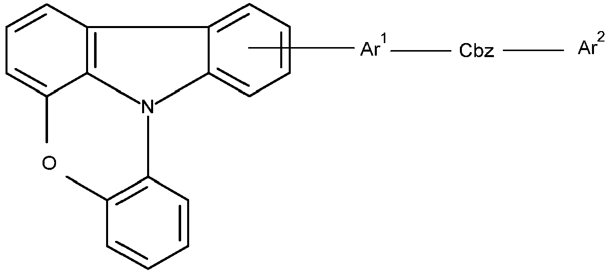 |
| formule (I-13) |
| 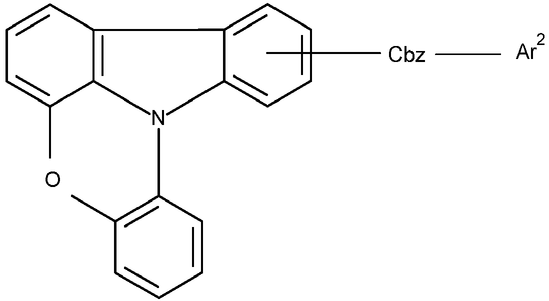 |
| formule (I-14) |
| 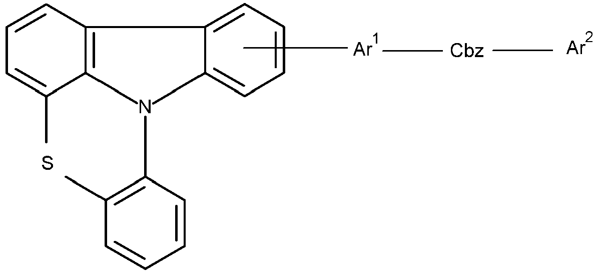 |
| formule (I-15) |
| 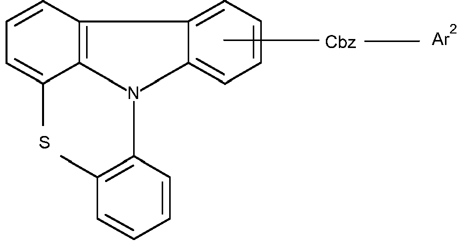 |
| formule (I-16) |
| 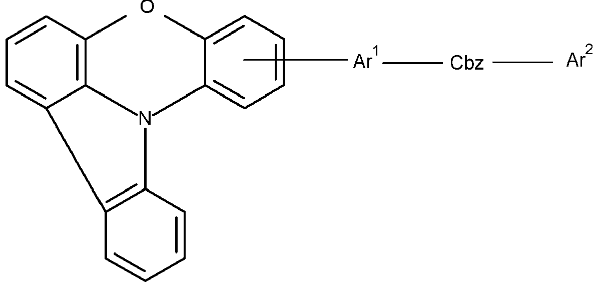 |
| formule (I-17) |
| 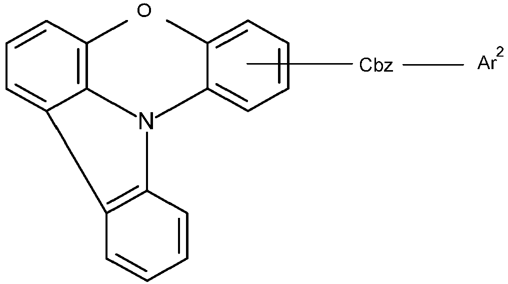 |
| formule (I-18) |
| 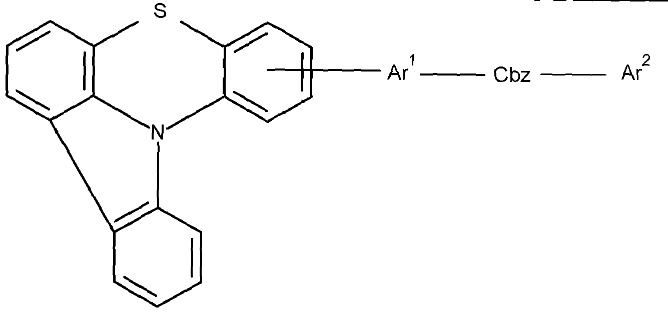 |
| formule (I-19) |
| 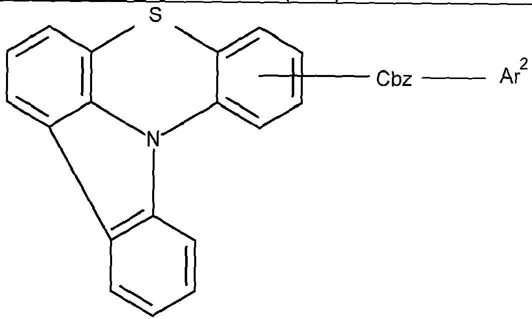 |
| formule (I-20) |
| 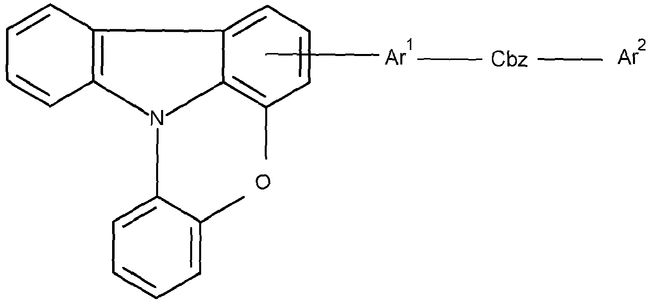 |
| formule (I-21) |
| 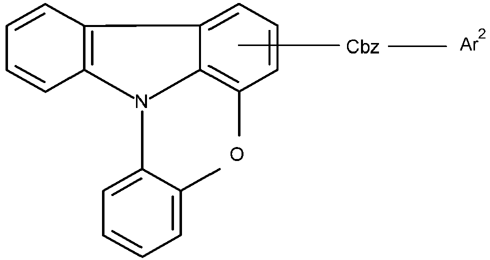 |
| formule (I-22) |
| 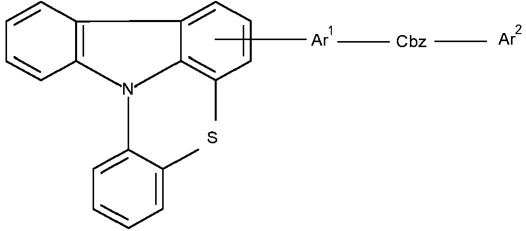 |
| formule (I-23) |
| 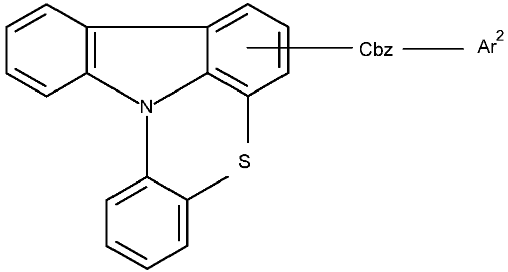 |
| formule (I-24) |
où les variables présentes sont définies tel que selon l'une ou plusieurs parmi les revendications 1 à 11, et les formules peuvent être substituées par un groupement R¹ au niveau de chacune des positions illustrées comme étant non substituées sur les cycles aromatiques à six chaînons.

13. Procédé de préparation d'un composé selon l'une ou plusieurs parmi les revendications 1 à 12, **caractérisé en ce que**, dans une première étape, un composé de triphénylamine qui est substitué par un groupement réactif sur l'un des groupements phényle est préparé, où les ponts entre les groupements phényle sont choisis parmi une liaison simple, O et S, et où au moins 2 ponts sont présents, et **en ce que**, dans une étape supplémentaire, un groupement carbazole est introduit dans le composé via une réaction de couplage catalysée par un métal de transition.

14. Oligomère, polymère ou dendrimère contenant un ou plusieurs composés de formule (I) selon l'une ou plusieurs parmi les revendications 1 à 12, la ou les liaisons au polymère, à l'oligomère ou au dendrimère pouvant être situées au niveau de positions quelconques dans la formule (I) qui sont substituées par R¹, R², R³ ou R⁴.

15. Formulation comprenant au moins un composé selon l'une ou plusieurs parmi les revendications 1 à 12 ou un polymère, oligomère ou dendrimère selon la revendication 14, et au moins un solvant.

16. Dispositif électronique contenant au moins un composé selon l'une ou plusieurs parmi les revendications 1 à 12, ou un polymère, oligomère ou dendrimère selon la revendication 14.

17. Dispositif électronique selon la revendication 16, **caractérisé en ce qu'**il s'agit d'un dispositif électroluminescent organique comprenant une anode, une cathode et au moins une couche émettrice, où au moins une couche organique du dispositif, qui est une couche émettrice, une couche de transport d'électrons ou une couche de blocage de trous, comprend le au moins un composé.

18. Utilisation d'un composé selon l'une ou plusieurs parmi les revendications 1 à 12, dans un dispositif électronique.
